# EUROPEAN PATENT APPLICATION

(11) **EP 3 854 886 A2**
(43) Date of publication of application: **28.07.2021**
(21) Application number: 18832907.2
(22) Date of filing: 03.09.2018
(51) Int. Cl.: C12Q 1/6886, C12Q 1/686, C12Q 1/6844, C12Q 1/70, C07K 14/01

(54) **METHODS AND KITS FOR DETERMINING A RISK OF CANCER**

(30) Priority: 30.11.2017 NL 1042665; 09.07.2018 MX 2018008471
(71) Applicant: Hakken Enterprise SA de CV, Xochitepec, Morelos 62790 (MX)
(72) Inventor: AGUIRRE GIL, Martha Gisela, 04770 Ciudad De México (MX); MEDA MONZÓN, Elizabeth, 62215 Cuernavaca, Morelos (MX); DELGADO PASTELIN, Lucero, 62766 Morelos (MX)
(74) Representative: Arnold & Siedsma
(86) International application number: PCT/MX2018/000084
(87) International publication number: WO 2019/013613

(57) **Abstract**

Disclosed are methods and kits for determining the susceptibility of cancer in a subject by performing a simultaneous identification and detection of HPV strain genotypes and genetic markers for cancer susceptibility in a sample from a subject.

LISTADO DE SECUENCIAS

<110> HAKKEN ENTERPRISE SA DE CV

<120> METODOS Y KITS PARA DETERMINAR EL RIESGO DE CANCER.

<140> MX/a/2018/008471
<141> 2018-09-07

<150> US 62/530,245
<151> 09/07/2017

<150> NL 1042665
<151> 10/01/2018

<160> 82

<170> PatentIn version 3.5

<210> 1
<211> 250
<212> DNA
<213> Homo sapiens

<220>
<221> CACUA
<223> misc_feature


<400> 1
agcaagtgca gactactctt acccacttcc cccaagcaca gttggggtgg gggacagctg        60
aagaggtgga aacatgtgcc tgagaatcct aatgaaatcg gggtaaagga gcctggaaca       120
catcctgtga ccccgcctgt mctgtaggaa gccagtctct ggaaagtaaa atggaagggc       180
tgcttgggaa ctttgaggat atttagccca ccccctcatt tttacttggg gaaactaagg       240
cccagagacc                                                              250

<210> 2
<211> 250
<212> DNA
<213> Homo sapiens

<220>
<221> CACUB
<223> misc_feature


<400> 2
catctacagt ggggccgacc gctatcgcct gcacacagct gctggtggca ccgtgcacct        60
ggagatcggc ctgctgctcc gcaacttcga ccgctacggc gtggagtgct gagggactct       120
gcctccaacr tcaccaccat ccacaccccg gacacccagt gatgggggag gatggcacag       180
tggtcaagag cacagactct agagactgtc agagctgacc ccagctaagg catggcaccg       240
cttctgtcct                                                              250

<210> 3
<211> 250
<212> DNA
<213> Homo sapiens

<220>
<221> CACUC
<223> misc_feature

<400> 3
ggtccctctg ggcccagttt ccctatctgt aaattgggga cagtaaatgt atggggtcgc 60
agggtgttga gtgacaggag gctgcttagc cacatgggag gtgctcagta aaggagagca 120
attcttacag gtgtctgcct cctgaccctt ccatccytca ggtgtcctgt tgccccctcc 180
tcccactgac accctccgga ggcccccatg ttgacagacc ctcttctcct accttgtttc 240
ccagcctgac 250

<210> 4
<211> 250
<212> DNA
<213> Homo sapiens

<220>
<221> CACUF
<223> misc_feature

<400> 4
cttcctgctt tctggtggaa gggaggggca gacagtgggt gtgtcctgct ccagtgtcta 60
ggcaggagag tttgtgaagc ygaccggaca cctgtggctc ttatttccta ggtggcccga 120
ggcagccggg atgacagctc tccccaggaa tcctgctgcc tgctgagaaa catggtcagc 180
aagtcccgtg agtgtcatcc gagggctccc ccaccctgga ggacaggcct cagaagccgt 240
cttcagcagg 250

<210> 5
<211> 250
<212> DNA
<213> Homo sapiens

<220>
<221> CACUG
<223> misc_feature

<400> 5
ctggggaatt ctggctggac tcagtccaga gtcccacctc atcacctgtt cacttccagt 60
tgtcctgaag ctggctacca ggtctccttg gccacctgaa ggcctaatcc ttctggcccc 120
gccagacccy aggcctccag tcacctaagg cccagtgagt gtcctctctt gcttctaggt 180
ccgaactcga aataacaaca gtgtctccag ccgggagagg cacaggcctt ccccacgttc 240
cacccgaggc 250

<210> 6
<211> 250
<212> DNA
<213> Homo sapiens

<220>
<221> CACUH
<223> misc_feature

<400> 6
ttcctgaaac ctcagaatag acctaccttg ccaagggctt ccttatgggt aaggacctta 60
tggacctgct gggacccaaa ctaggcctca cctgatacga cctgtccttc tcaaaacacc 120
taaacttggg agaacattgt yccccagtgc tggggtagga gagtctgcct gttattctgc 180
ctctatgcag agaaggagcc ccagatcagc ttttccatga caggacagtt tccaagatgc 240
cacctgtact 250

<210> 7
<211> 250
<212> DNA
<213> Homo sapiens

<220>
<221> CACUJ
<223> misc_feature

<400> 7
ccacagaagc ttacaactaa aagaaactct aaggccaatt taatccaagg tttcattcta 60
tgtgctggag atggtgtaca gtagggtgag gaaaccaaat tctcagttgg cactggtgta 120
cccttgtaca ggtgatgtaa yatctctgtg cctcagtttg ctcactataa aatagagacg 180
gtaggggtca tggtgagcac tacctgacta gcatataaga agctttcagc aagtgcagac 240
tactcttacc 250

<210> 8
<211> 250
<212> DNA
<213> Homo sapiens

<220>
<221> CAPA
<223> misc_feature

<400> 8
gctcatccac caagaagcct gcaccatgtt ttgaggttga gtgacatgtt cgaaacctgt 60
ccataaagta attttgtgaa agaaggagca agagaacatt cctctgcagc acttcactac 120
caaatgagcm ttagctactt ttcagaattg aaggagaaaa tgcattatgt ggactgaacc 180
gacttttcta aagctctgaa caaaagcttt tctttccttt tgcaacaaga caaagcaaag 240
ccacattttg 250

<210> 9
<211> 250
<212> DNA
<213> Homo sapiens

<220>
<221> CAPB
<223> misc_feature

<400> 9
aaactcagtg gggcggctgg ggtactcttc cccggccgtg ggagtctccg cggggtacgc 60
ggccagggtg gctgcctggg cacagggttt cagcgagctc cgggacactc ggcaggagta 120
gtacccgcct ycaaagtaac cataaggcac gggagctggg gacgtcccct ggggcacccc 180
agggcatggg tggcattgct ttggcggctc cgccgagcct ggcagatcca agggggcata 240
gttgacagca 250

<210> 10
<211> 250
<212> DNA
<213> Homo sapiens

<220>
<221> CAPC
<223> misc_feature

<400> 10
cyagcagagc ccagtgctgg caggcagcgc cactttggtc gcccttgggg cactggcctt 60
gtacgtcgcg aagcctccgg ctacgggaag cacacggaga gcctgaagcc ggcggctacc 120
cgcctgccar cccrcgccgc ctggttcctg caggagctgc cttccttcgc ggtgcccgcg 180
gggatcctcg cccggcagcc cctctccctc ttcgggccac ctgggacggt acttctgggc 240
ctcttctgcs 250

<210> 11
<211> 250
<212> DNA
<213> Homo sapiens

<220>
<221> CAPD

<223> misc_feature

<400> 11
aatttaaaat gatacaaagt tcaaagttta acaaatacat ttgaagccat ttgcaacaaa 60
tacatctgaa gctaattgct ggctctagaa agtgtggggt ctttgttgtg gagcagtgtt 120
aatgatttag cattacttat mtctggcaaa tggtattttt gagataacat gttatggaag 180
aaagtgaact gaacttggaa gtttgaagat ctcgattgaa gtatcatttc tgcctcaact 240
acttgcatta 250

<210> 12
<211> 250
<212> DNA
<213> Homo sapiens

<220>
<221> CAPE
<223> misc_feature

<400> 12
ccatatttgg gctatattaa gtaataagcc cacatgcttt ctgttgagaa aatacaaaaa 60
gatgtttccc tctgtcataa agaaaaagag gtaacccagg gaacattttg tccctctagt 120
tatcttcccr caggcccatc aagaatcagg cagtaggtga aaaagaaaca cagagaacct 180
aggaacacaa taggaagacc accatgggcc cttagggagt cagcgaaggc ttatgatgca 240
aaaagaaggt 250

<210> 13
<211> 250
<212> DNA
<213> Homo sapiens

<220>
<221> CAPF
<223> misc_feature

<400> 13
ggggacaagg taaacacata acttgggtgg agatgtgatt gattaataac tcataaatca 60
tctgaaacca tactttcctt ttgatagtca accctctgta aacactcaat gtgttctcac 120
cttgttatca ttcccaatga ygtcgaatgc gtggttgccc tctccagtat aaaagtttga 180
tgcagctttg cctggatgta cctgtctata aggagtcctg cttatcacaa tggtaggtaa 240
ctggttttat 250

<210> 14
<211> 250
<212> DNA
<213> Homo sapiens

<220>
<221> CAPG
<223> misc_feature

<400> 14
tctttgcttc cactttaatc tcttgtcttt ttattttttg tcacacttct ccatcccctt 60
tttcctccct cctctacgcc tccctaaact tttttctcct ctttctatcc tacttcacct 120
cttctctttt cctcatatac tcatactcct ctgcattccy gccttcatat ttcccccctc 180
cctcctttcc ctcaactttc tctccccagt cccatcttct tgttcttcct attgttgtca 240
ctttttctc 250

<210> 15
<211> 250
<212> DNA
<213> Homo sapiens

<220>

<221> CAPH
<223> misc_feature

<400> 15
caacatgcca ttgaatctgt cttttcagcc aggttttgta atccctttac tctcagaaca 60
gagtttcagg gatggtaggt agtctgtagc tgaagaggtg taggttgaac aaacttgtak 120
agtgaggaaa gagtgagtca tcattaaatg cattttcagt ggcagatgac atactccaag 180
gtttgccttt acttagacag ggcctagagt ttctgtgact tgcaatggct ctcaatcaga 240
cttgtgctgg 250

<210> 16
<211> 250
<212> DNA
<213> Homo sapiens

<220>
<221> CAPI
<223> misc_feature

<400> 16
agacactata ctagatgatg ggacaactaa agggtaatga acagttctgt ctctatgtaa 60
aaataataat gatgatgatg atgagatggg acttcaattg aggaagtgcc attggggagg 120
tatgtaaaam gtgctatgga aaaaaagcaa caggaacccc ttgatagaaa aaaaaatgct 180
ggtgggggta gggatttctg cctgtgttct tcagaatggg gtatgggaaa atctgggagg 240
aaaagaaatt 250

<210> 17
<211> 250
<212> DNA
<213> Homo sapiens

<220>
<221> CAPJ
<223> misc_feature

<400> 17
tctcagctcc ctatccataa aacagaggga cgaataaact ctcctcctac cactaagagg 60
tgtagccaga gttaataccc tcatcgtcct ttgagctcag cagatgaaag kcactgagaa 120
aagtacaaag aatttttatg tgctattgac tttattttat tttatgtggg ggagggagcc 180
ggccccagct ggaaagctgc tttctctgaa tcaaagggca ggaacccagc aagtttctca 240
ggattggggc 250

<210> 18
<211> 250
<212> DNA
<213> Homo sapiens

<220>
<221> COLA
<223> misc_feature

<400> 18
ctccaatatg tttttcaaga tgtagttcat tatcatcttt gtcatcagct gaagatgaaa 60
taggatgtaa tcagacgaca caggaagcag attctgctaa taccctgcaa atagcagaaa 120
waaaagaaaa gattggaact aggtcagctg aagatcctgt gagcgaagtt ccagcagtgt 180
cacagcaccc tagaaccaaa tccagcagac tgcagggttc tagtttatct tcagaatcag 240
ccaggcacaa 250

<210> 19
<211> 250
<212> DNA
<213> Homo sapiens

<220>
<221> COLB
<223> misc_feature

<400> 19
gagccagcac cgctctttga ccttgccatg cttgccttag atagtccaga gagtggctgg 60
acagaggaag atggtcccaa agaaggactt gctgaataca ttgttgagtt tctgaagaag 120
nggctgagat gcttgcagac tatttctctt tggaaattga tgaggtgtga cagccattct 180
tatacttctg ttgtattctt caaataaaat ttccagccgg gtgcggtggc tcatggctgt 240
aatcccagca 250

<210> 20
<211> 250
<212> DNA
<213> Homo sapiens

<220>
<221> COLC
<223> misc_feature

<400> 20
acattgcaac ttaccacaat aaagtaaata tcacaagata aatgacaatg ttggaatgga 60
aactacttag ggactcagaa cggtcagacg caaacagttt caagactatt mgctgttaaa 120
ggttatgcct tatgtcaccc aaaagggttt tcccctagat ttatagcaca aactcatgga 180
agatttattg ccgtcttaat tttttcccca attttaactt tamgaacagt cagcctggaa 240
tkcctcctaa 250

<210> 21
<211> 250
<212> DNA
<213> Homo sapiens

<220>
<221> COLD
<223> misc_feature

<400> 21
ccgatctccg cagccctccg ttgaggttct tcgccttggg cggtgacgcg gagcgcgcca 60
agggcctggg cccaggccca gcctcgctcc aggccgcatc cccgcctggg gaaggagagg 120
ccycgggggc agcggcggca cggccgcctt cgcgtgaggc cctggccgag gccttgttgg 180
catcactcag cgccggagac ttggggaaga agctgtacag ggtgctctgt cgcgacatac 240
cgacagccgg 250

<210> 22
<211> 250
<212> DNA
<213> Homo sapiens

<220>
<221> COLE
<223> misc_feature

<400> 22

aggtggaggc cagcctctcc tgactgtcat ccctattggc aggttacccc aaaggccacc 60
ccgaagcagg gagctttgag gctgacctga agcacttgaa ggagaaggtg tctgcgggag 120
ycgatttcat catcacgcag cttttctttg aggctgacac attcttccgc tttgtgaagg 180
catgcaccga catgggcatc acttgcccca tcgtccccgg gatctttccc atccaggtga 240
ggggcccagg 250

<210> 23
<211> 250
<212> DNA

<213> Homo sapiens

<220>
<221> COLF
<223> misc_feature

<400> 23

gaacagcctg gggcccttgg ggcccaaatt ctgctggtgc agaggcccaa ctcaggtacc 60
tggatactgg gcgtggaggg cagtggcatg agtaacaaga gagaatggag ggaatcggca 120
gctgaggcct gacccctgcc tggctgccct ccctctcagr tctgctggca ggactgtggg 180
agttcccgtc cgtgacctgg gagccctcag agcagcttca gcgcaaggcc ctgctgcagg 240
aactacagcg 250

<210> 24
<211> 250
<212> DNA
<213> Homo sapiens

<220>
<221> COLG
<223> misc_feature

<400> 24

ctggaggtga gagccaccct agggtagggg aaataggaac aatagaggga ctgacgggtg 60
atctctttga cctctgatcc tacccacagg aggtgaatca actctgggct ggcctgggct 120
rctattctcg tggccggcgg ctgcaggagg gagctcggaa ggtaagggga tggcaggagg 180
gtaggaaccc aggagtcttg ggtgtcttat aatcttgagt cttgcactcc aatcaggtgg 240
tagaggagct 250

<210> 25
<211> 250
<212> DNA
<213> Homo sapiens

<220>
<221> GASA
<223> misc_feature

<400> 25
aacaaaagaa ctcagccaag tgtaaaagcc ctttctgatc ccaggtctta gtgagccacc 60
ggcggggctg ggattcgaac ccagtggaat cagaaccgtg caggtcccat aacccaccta 120
gaccctagca actccaggct agagggtcam cgcgtctatg cgaggccggg tgggcgggcc 180
gtcagctccg ccctggggag gggtccgcgc tgctgattgg ctgtggccgg caggtgaacc 240
ctcagccaat 250

<210> 26
<211> 250
<212> DNA
<213> Homo sapiens

<220>
<221> GASB
<223> misc_feature

<400> 26
attagtcccc tcccctaaga agcttccacc aatactcttt tcccctttcc tttaacttga 60
ttgtgaaatc aggtattcaa cagagaaatt tctcagcctc ctacttctgc ttttgaaagc 120
yataaaaaca gcgaggagaa aactggcaga taccaaacct cttcgaggca caaggcacaa 180
caggctgctc tgggattctc ttcagccaat cttcattgct caagtatgac tttaatcttc 240
cttacaacta 250

<210> 27
<211> 250
<212> DNA
<213> Homo sapiens

<220>
<221> GASC
<223> misc_feature

<400> 27
atcatgatag catctgtaat taactgaaaa aaaataatta tgccattaaa agaaaatcat 60
ccatgatctt gttctaacac ctgccactct agtactatat ctgtcacatg gtactatgat 120
aaagttatct agaaataaaa aagcatacaw ttgataattc accaaattgt ggagcttcag 180
tattttaaat gtatattaaa attaaattat tttaaagatc aaagaaaact ttcgtcatac 240
tccgtatttg 250

<210> 28
<211> 250
<212> DNA
<213> Homo sapiens

<220>
<221> GASD
<223> misc_feature

<400> 28
ttctgggaga atttagaaat gaaggaaact tggaaaagtt tgacaaatct gctctagagg 60
gcctgtgcaa tttgaccatt gaagaattcc gattagcata cttagactac tacctcgatg 120
rtattattga cttatttaat tgtttgacaa atgtttcttc attttccctg gtgagtgtga 180
ctattgaaag ggtaaaagac ttttcttata atttcggatg gcaacattta gaattagtta 240
actgtaaatt 250

<210> 29
<211> 250
<212> DNA
<213> Homo sapiens

<220>
<221> GASE
<223> misc_feature

<400> 29
cttccaacaa aggtgggaat gctttttcag aagttgatct accaagcctt gagtttctag 60
atctcagtag aaatggcttg agtttcaaag gttgctgttc tcaaagtgat tttgggacaa 120
ycagcctaaa gtatttagat ctgagcttca atggtgttat taccatgagt tcaaacttct 180
tgggcttaga acaactagaa catctggatt tccagcattc caatttgaaa caaatgagtg 240
agttttcagt 250

<210> 30
<211> 250
<212> DNA
<213> Homo sapiens

<220>
<221> GASG
<223> misc_feature

<400> 30
ctgaagcccc tcccagttct agttctatct ttttcctgca tcctgtctgg aagttagaag 60
gaaacagacc acagacctgg tccccaaaag aaatggaggc aataggtttt gaggggcatg 120
rggacggggt tcagcctcca gggtcctaca cacaaatcag tcagtggccc agaagacccc 180
cctcggaatc ggagcaggga ggatggggag tgtgaggggt atccttgatg cttgtgtgtc 240
cccaactttc 250

<210> 31
<211> 250
<212> DNA
<213> Homo sapiens

<220>
<221> LEUA
<223> misc_feature

<400> 31
cagctctacg tctcctccga gagccgcttc aacaccctgg ccgagttggt tcatcatcat 60
tcaacggtgg ccgacgggct catcaccacg ctccattatc cagccccaaa gcgcaacaag 120
cccactgtct atggtgtgtc ccccaactac gacaagtggg agatggaacg cacggacatc 180
accatgaagc acargctggg cgggggccag tacggggagg tgtacgaggg cgtgtggaag 240
aaatacagcc 250

<210> 32
<211> 250
<212> DNA
<213> Homo sapiens

<220>
<221> LEUB
<223> misc_feature

<400> 32
ttggaggtgg agttggcttt cttcccactg cgttcatttg ttactcagag tggtagcagt 60
gtttggttat agtttagttk tgtgatgttt tttttcttga gtgatgaagc aatagtgagg 120
gcaaaagctg atagtaacat tttgatacat atttttgtat ttgtctatgc ctctatttta 180
agtggtttgc ttttgaaaac agctggttca aacccttggt tttgggtttg aaatattctt 240
tgttttgttt 250

<210> 33
<211> 250
<212> DNA
<213> Homo sapiens

<220>
<221> LEUC
<223> misc_feature

<400> 33
agagacaggt accacgtact gaggcagcaa agggactcag tgagtgtcag gagaagcctc 60
ttacccctct ggccctcaac tgctttatct gtaaaatgtg gctcacccta ycttgctggt 120
ctgagggcag agcccaccag atgatctctt gaggtctctc ccagcacatc ttgagatctg 180
cgattttaca gagccaattt tggttctacc tctaaacaat cgtgtgactc caggtaagtc 240
ccttcccctc 250

<210> 34
<211> 250
<212> DNA
<213> Homo sapiens

<220>
<221> LEUD
<223> misc_feature

<400> 34
ttgagatcct gatagcctgt tacaggaatg aagtaaaggt catttttttt tgtattgatt 60
ttcacagctt tgaggaacat gcataagaaa tgtagctgaa gtagaggggr cgtgagagaa 120
gggccaggcc ggcaggccaa ccctcctcca atggaaattc ccgtgttgct tcaaactgag 180
acagatggga cttaacaggc aatggggtcc acttccccct cttcagcatc ccccgtaccc 240

cactttctg                                                          249

<210> 35
<211> 250
<212> DNA
<213> Homo sapiens

<220>
<221> LEUE
<223> misc_feature

<400> 35
attctttgta cttttttttt tccttagtct ttctttgaag cagcaagtat gatgagcaag      60
ctttctcaca agcatttggt tttaaattat ggagtatgtd tctgtggaga cgagagtaag     120
taaaactaca ggctttctaa tgcctttctc agagcatctg tttttgttta tatagaaaat     180
tcagtttcag gatcacagct aggtgtcagt gtaaactata atttaacagg agttaagtat     240
ttttgaaact                                                            250

<210> 36
<211> 250
<212> DNA
<213> Homo sapiens

<220>
<221> LEUF
<223> misc_feature

<400> 36
aacttctgtt gtttatagta caactgcatg gaattggttg acttacctct ctgtgctttc      60
tgtatcctca gagtggcatt ctgcatttct gtggcttcca agtcttagaa yctcaactga     120
catatagcat tgggcacact ccagcagacg cccgaattca aatcctggaa ggatggaaga     180
aacgcctgga gaatatttgg gatgagacac cactgtattt tgctccaagc agcctctttg     240
acctaaactt                                                            250

<210> 37
<211> 250
<212> DNA
<213> Homo sapiens

<220>
<221> MAMA
<223> misc_feature

<400> 37
tttttactca aactattggg tggatttgtt tgtatattct aggtgaaaac tgacttttgt      60
cagactgtac ttccatactt gattcatgat attttactcc aaratacaaa tgaatcatgg     120
agaaatctgc tttctacaca tgttcaggga tttttcacca gctgtcttcg acacttctcg     180
caaacgagcc gatccacaac ccctgcaaac ttggattcag gtattctatt aaatttttaa     240
cattaatact                                                            250

<210> 38
<211> 250
<212> DNA
<213> Homo sapiens

<220>
<221> MAMB
<223> misc_feature

<400> 38
aaagatatat atatatgttt ttctaatgtg ttaaagttca ttggaacaga aagaaatgga      60
tttatctgct cttcgcgttg aagaagtaca aaatgtcatt aatgctatgc agaaaatctt     120
nagtgtccca tctggtaagt cagcacaaga gtgtattaat ttgggattcc tatgattatc     180

tcctatgcaa atgaacagaa ttgaccttac atactaggga agaaaagaca tgtctagtaa 240
gattaggcta 250

<210> 39
<211> 250
<212> DNA
<213> Homo sapiens

<220>
<221> MAMC
<223> misc_feature

<400> 39
ctactagttt agcttgtgtt gaaattgtaa ataccttggc attagataat caaaagaaac 60
tgagcaagcc tcagtcaatt aatactgtat ctgcacattt acagagtagt gtagttgttt 120
ctgattgtaa aaatagtcat ataacccctn cagatgttat tttccaagca ggattttaat 180
tcaaaccata atttaacacc tagccaaaag gcagaaatta cagaactttc tactatatta 240
gaagaatcag 250

<210> 40
<211> 250
<212> DNA
<213> Homo sapiens

<220>
<221> MAMD
<223> misc_feature

<400> 40
catctgggct ccatttagac ctgaaagggt tagttgagac cattcacagg ccaaagacgg 60
tacaacttcc ttggagattt tgtcacttcc actctcaaag ggcttctgat ktgctacatt 120
tgaatctaat ggatcagtat catttggttc cacttcagat acaaatgagt atttttcttt 180
cacttggttt ttagattttt cacattcatc agcgtttgct tcatggaaaa tttttttcct 240
agtcttgcta 250

<210> 41
<211> 250
<212> DNA
<213> Homo sapiens

<220>
<221> MAME
<223> misc_feature

<400> 41
tgaaatctgt catcagtagg gaatattggt agctgagtta tttttcgagt ggtaatccga 60
gaataaaacg gcagatccca gcactcatcg ccacttaatg aacctgtttg yggagagtcc 120
acctggtgcc tgcctggctt taggaacccg cagcagtccg agtggtgtct ggggtaagct 180
gagctgctct gggaacacat ctcgtgcgtg gggtgaatga acagcacact tacccagtgg 240
ggtaggctgg 250

<210> 42
<211> 250
<212> DNA
<213> Homo sapiens

<220>
<221> MAMF
<223> misc_feature

<400> 42
ccaagcctcc cctccaccac tgcgcccttc tccctgagga cctcagcttt ccctcgaggc 60
cctcctacct tttgccggga gacccccagc ccctgcaggg gcggggcctc cccaccacac 120

cagccctgtt cgcgctctcg gcagtgccgg ggggcgccgc ctcccccatg ccgccctccg 180
ggctgcggct gctgcbgctg ctgctaccgc tgctgtggct actggtgctg acgcctggcc 240
ggccggccgc 250

<210> 43
<211> 250
<212> DNA
<213> Homo sapiens

<220>
<221> MAMG
<223> misc_feature

<400> 43
tgcagtgtgc ctaccttgcc tcagtcctgg cctgggcagg agtctggcgg agcctctgag 60
gtgacgagac cacctggctg ggtgaatgtc cagagggtgg gcagctgggc tcccggtctc 120
nagagttctc cccagctccc tgatcacatc ccttttataa agcccggggt gacgtctcca 180
agcacaggcc cactctgccg cccctccctc ctcctcagac cctcctgaag agcatggcac 240
cttgggaggc 250

<210> 44
<211> 250
<212> DNA
<213> Homo sapiens

<220>
<221> PROA
<223> misc_feature

<400> 44
gctcatccac caagaagcct gcaccatgtt ttgaggttga gtgacatgtt cgaaacctgt 60
ccataaagta attttgtgaa agaaggagca agagaacatt cctctgcagc acttcactac 120
caaatgagcm ttagctactt ttcagaattg aaggagaaaa tgcattatgt ggactgaacc 180
gacttttcta aagctctgaa caaaagcttt tctttccttt tgcaacaaga caaagcaaag 240
ccacattttg 250

<210> 45
<211> 250
<212> DNA
<213> Homo sapiens

<220>
<221> PROB
<223> misc_feature

<400> 45
aaactcagtg gggcggctgg ggtactcttc cccggccgtg ggagtctccg cggggtacgc 60
ggccagggtg gctgcctggg cacagggttt cagcgagctc cgggacactc ggcaggagta 120
gtacccgcct ycaaagtaac cataaggcac gggagctggg gacgtcccct ggggcacccc 180
agggcatggg tggcattgct ttggcggctc cgccgagcct ggcagatcca agggggcata 240
gttgacagca 250

<210> 46
<211> 250
<212> DNA
<213> Homo sapiens

<220>
<221> PROC
<223> misc_feature

<400> 46
cyagcagagc ccagtgctgg caggcagcgc cactttggtc gcccttgggg cactggcctt 60

gtacgtcgcg aagcctccgg ctacgggaag cacacggaga gcctgaagcc ggcggctacc 120
cgcctgccar cccrcgccgc ctggttcctg caggagctgc cttccttcgc ggtgcccgcg 180
gggatcctcg cccggcagcc cctctccctc ttcgggccac ctgggacggt acttctgggc 240
ctcttctgcs 250

<210> 47
<211> 250
<212> DNA
<213> Homo sapiens

<220>
<221> PROD
<223> misc_feature

<400> 47
ttggcgttcc tcggtgcgcg ctctacgctg cgctcctgga cgccgggagc agggcagtgc 60
gctgcactgg gcgcccgcaa gggaaaaacg ctacctgtgg aagtaatgta sgcagaagag 120
gcccagaagt accgtcccag gtggcccgaa gagggagagg ggctgccggg cgaggatccc 180
cgcgggcacc gcgaaggaag gcagctcctg caggaaccag gcggcgcggg ctggcaggcg 240
ggtagccgcc 250

<210> 48
<211> 250
<212> DNA
<213> Homo sapiens

<220>
<221> PROE
<223> misc_feature

<400> 48
cttttgagaa gtttccagtc tgcctttcct gcctccttct cctttctgaa gaaattctca 60
ttgaatacag agaggcagca cagactggar atgctgcata aagcttaaat tgggcagggc 120
ccaagcgttg ttgggtcttt ggagacaatg gctcctgaga atttttttag gctttccagg 180
aactacagag agttgcttca tgtcaggaac acaaattctt aaagagctag tcaccaagta 240
tgggggcca 250

<210> 49
<211> 250
<212> DNA
<213> Homo sapiens

<220>
<221> PROF
<223> misc_feature

<400> 49
   tctttgcttc cactttaatc tcttgtcttt ttattttttg tcacacttct ccatcccctt 60
   tttcctccct cctctacgcc tccctaaact tttttctcct ctttctatcc tacttcacct 120
   cttctctttt cctcatatac tcatactcct ctgcattccy gccttcatat ttcccccctc 180
   cctcctttcc ctcaactttc tctccccagt cccatcttct tgttcttcct attgttgtca 240
   cttttttctc 250

<210> 50
<211> 250
<212> DNA
<213> Homo sapiens

<220>
<221> PANA
<223> misc_feature

<400> 50

ctgacatgag accaactaag acagcataac ccgtggggca ggacagactc ctggggtggc 60
agctgtgttc atcatcttga ctgggtcaag atgtatccag ctgtaccttt yatgtgcggt 120
ttattgtata catccgttga aacatattaa gacaaaaaag ggaaaacaaa gaccacaaag 180
gagggacagg gaagaacccg gaaggcgctg gcagcacagg gaggaggcag gtgtttgcag 240
tcagacctga 250

<210> 51
<211> 250
<212> DNA
<213> Homo sapiens

<220>
<221> PANB
<223> misc_feature

<400> 51
ttagggcttc gctttgacgg cgacagtgac agcgacagcc aaagcagggt ccgaaaccaa 60
ggcgggctgc caggtggggc tggaccacgt gagcaaaatc tgggvgaggc ggagctttgg 120
agggcgacgg ggcctgctgg agtgggtggg actgaaatga aggtgagggt gagaaggaag 180
ctggaaatgg agttgagctg ggagcggagg tcaggtgggg actgggctgg agactggggg 240
gactcgcctt 250

<210> 52
<211> 250
<212> DNA
<213> Homo sapiens

<220>
<221> PANC
<223> misc_feature

<400> 52
cattcaaagg aactctttgt gggctcaagg ttggagttga tagattcagc aagaattttt 60
ccctcttcaa cttcgtcagc ccaatcctca gtaggtaaag ttgtcttctt gtctgatgca 120
cgtgctgccc ttgctgcacy tctatcacac ctgtgccttc tctatcatga cacttaatct 180
accgtcctgt agatgggagt cctggcagga cacagataac ctcttggagc agtactctcc 240
attcctctat 250

<210> 53
<211> 250
<212> DNA
<213> Homo sapiens

<220>
<221> PAND
<223> misc_feature

<400> 53
gcaggaggtc gcacagcagg ttgctgtagt gggtcacctg gctgcgcacg tcagcagcct 60
tggcctgccg tgacagtgtg tccccgatga acaccagctt gtgggcgctr aggatgacga 120
acttgctgtg cgccacaaag atcttgggcg gctggttggt ggccacggcg gtaaagaagg 180
cgtccacggc gttggtcagt gtggtcaggt tggcctcaca ctgctccagg tagaagagca 240
gcagctgccg 250

<210> 54
<211> 250
<212> DNA
<213> Homo sapiens

<220>
<221> PULA
<223> misc_feature

<400> 54
tgggttttac tgtagggagg aagaagagga ggtagcagtg aagaggtgta gccgctgcac 60
ttaagcagtc tgtttgaggg acaagactct attttttgag acagggtccc caggtcatcc 120
aggctggagt gcactggtac cattttgttt cactgtaacc tccacctccy gggctcacac 180
gattctccca cctcagcctc tgagtagttg gggccgcagg cacacgccac cacagctttt 240
tttttttttt 250

<210> 55
<211> 250
<212> DNA
<213> Homo sapiens

<220>
<221> PULB
<223> misc_feature

<400> 55
gtgtgccgcc tgctgggcat ctgcctcacc tccaccgtgc arctcatcay gcagctcatg 60
cccttcggct gcctcctgga ctatgtccgg gaacacaaag acaatattgg ctcccagtac 120
ctgctcaact ggtgtgtgca gatcgcaaag gtaatcaggg aagggagata cggggagggg 180
agataaggag ccaggatcct cacatgcggt ctgcgctcct gggatagcaa gagtttgcca 240
tggggatatg 250

<210> 56
<211> 250
<212> DNA
<213> Homo sapiens

<220>
<221> PULC
<223> misc_feature

<400> 56
agcctgaccg tgcagggtct rctctctccc tccaccctga ctgtgctctg tccccccagg 60
gctggacatc cacttcatcc acgtgaagcc cccccagctg cccgcaggcc rtaccccgaa 120
gcccttgctg atggtgcacg gctggcccgg ctctttctac gagttttata agatcatccc 180
actcctgact gaccccaaga accatggcct gagcgatgag cacgtttttg aagtcatctg 240
cccttccatc 250

<210> 57
<211> 250
<212> DNA
<213> Homo sapiens

<220>
<221> PULD
<223> misc_feature

<400> 57
gtcctacgat aaggcgaaaa tcgatctggt cctgatcggc tcttccatga acctcaagga 60
ctattgggag agcggcgagt gggccatcat caaagcccca ggctayaaac acgacatcaa 120
gtacaactgc tgcgaggaga tctaccccga catcacatac tcgctgtaca tccggcgcct 180
gcccttgttc tacaccatca acctcatcat cccctgcctg ctcatctcct tcctcactgt 240
gctcgtcttc 250

<210> 58
<211> 250
<212> DNA
<213> Homo sapiens

<220>
<221> PULE
<223> misc_feature

<400> 58
tatataagat attatttttt ttacagagta acagactact agagacaatg aattaaggga 60
aaatgacaaa gaacagctca aagcaatttc tacacgagat cctctctctg aaatcactra 120
gcaggagaaa gattttctat ggagtcacag gtaagtgcta aaatggagat tctctgtttc 180
tttttcttta ttacagaaaa aataactgaa tttggctgat ctcagcatgt ttttaccata 240
cctattgg 248

<210> 59
<211> 250
<212> DNA
<213> Homo sapiens

<220>
<221> PULF
<223> misc_feature

<400> 59
gaaagaccct agccttagat aaaactgagc aagaggcttt ggagtatttc atgaaacaaa 60
tgaatgatgc acdtcatggt ggctggacaa caaaaatgga ttggatcttc cacacaatta 120
aacagcatgc attgaactga aaagataact gagaaaatga aagctcactc tggattccac 180
actgcactgt taataactct cagcaggcaa agaccgattg cataggaatt gcacaatcca 240
tgaacagcat 250

<210> 60
<211> 250
<212> DNA
<213> Homo sapiens

<220>
<221> PULG
<223> misc_feature

<400> 60
ccacttcagc tgtctccctc tggttacagg aagctatggg tcaacccatc tgagttccta 60
cctgaacggt ttctcacccc tgatggtgct atcgacaagg tgttaagtga gaaggtgatt 120
atctttggca tgggcaagcg gaagtgtatc ggtgagaccn ttgcccgctg ggaggtcttt 180
ctcttcctgg ctatcctgct gcaacgggtg gaattcagcg tgccactggg cgtgaaggtg 240
gacatgaccc 250

<210> 61
<211> 250
<212> DNA
<213> Homo sapiens

<220>
<221> GENA
<223> misc_feature

<400> 61
ggagggcggg atttcggacg gctctcgcgg cggtgggggt gggggtggtt cggaggtctc 60
cgcgggagtt cagggtaaag gtcacggggg ccgggggctg cggggccgct kcggcgcggg 120
aggtccggat gatcgcaggt gcctgtcggg tcactagtgt gaacgctgcg cgtagtctgg 180
gcgggattgg gccggttcag tgggcaggtt gactcagctt ttcctcttga gctggtcaag 240
ttcagacacg 250

<210> 62
<211> 250
<212> DNA
<213> Homo sapiens

<220>
<221> GENB

<223> misc_feature

<400> 62
attacagaat tatagaaatt agatctctta cctaaactct tcataatgct tgctctgata 60
ggaaaatgag atctactgtt ttcctttact tactacacct cagatatatt tcttcatgaa 120
gacctcacag taaaaatagg tgattttggt ctagctacag wgaaatctcg atggagtggg 180
tcccatcagt ttgaacagtt gtctggatcc attttgtgga tggtaagaat tgaggctatt 240
tttccactga 250

<210> 63
<211> 250
<212> DNA
<213> Homo sapiens

<220>
<221> GEND
<223> misc_feature

<400> 63
gcagtttcta ctaaatgcat gttgcttttg taccgtcata aagtcaaaca attgtaactt 60
gaaccatctt ttaactcagg tactgtgtat atacttactt ctccccctcc tctgttgctg 120
cagatccgtg ggcgtgagcn cttcgagatg ttccgagagc tgaatgaggc cttggaactc 180
aaggatgccc aggctgggaa ggagccaggg gggagcaggg ctcactccag gtgagtgacc 240
tcagcccctt 250

<210> 64
<211> 250
<212> DNA
<213> Homo sapiens

<220>
<221> GENE
<223> misc_feature

<400> 64
gaatgccaaa caccttcatg tcccccgtgg gccccctttg tccctcccac cccaaactag 60
ccctcaatcc ctgaccctgg cttccgcccc cagccctctg acgtccatcr tctctgcggt 120
ggttggcatt ctgctggtcg tggtcttggg ggtggtcttt gggatcctca tcaagcgacg 180
gcagcagaag atccggaagt acacgatgcg gagactgctg caggaaacgg aggtgaggcg 240
gggtgaagtc 250

<210> 65
<211> 250
<212> DNA
<213> Homo sapiens

<220>
<221> GENG
<223> misc_feature

<400> 65
tgcgtaggaa gtgcagcagg acaaccctga cagaccctgc aagccaggga gcccccgagg 60
gtggggctgg gggaggcgca gggcagcact gccctctggg gtgggagttc rgagggtgaa 120
cagggcccag aagcagggtg gagctgaggg acctggcaaa ccaggccctg cggggaggtc 180
ggcctctcca ctcagtcctc tcaatgcctg ctggggtctc ggggccaata gccccccacc 240
tgacaccccc 250

<210> 66
<211> 250
<212> DNA
<213> Homo sapiens

<220>

<221> GENH
<223> misc_feature

<400> 66
ttgaaataat ttttcatata aaggtgacag tatttgatag tgtattaacc ttatgtgtga 60
catgttctaa tatagtcaca ttttcattat ttttattata aggcctgctg aaaatgactg 120
aatataaact tgtggtagtt ggagctgntg ncgtaggcaa gagtgccttg acgatacagc 180
taattcagaa tcattttgtg gacgaatatg atccaacaat agaggtaaat cttgttttaa 240
tatgcatatt 250

<210> 67
<211> 250
<212> DNA
<213> Homo sapiens

<220>
<221> GENI
<223> misc_feature

<400> 67

taatatgcat attaaaacaa gatttacctc tattgttgga tcatattcgt ccacaaaatg 60
attctgaatt agctgtatcg tcaaggcact cttgcctacg ncaccagctc caactaccac 120
aagtttatat tcagtcattt tcagcaggcc ttataataaa aataatgaaa atgtgactat 180
attagaacat gtcacacata aggttaatac actatcaaat actccaccag taccttttaa 240
tacaaactca 250

<210> 68
<211> 250
<212> DNA
<213> Papillomaviridae

<220>
<221> VPHA
<223> misc_feature

<400> 68
gctggtrctg ttggtgaaaa tgtaccagac gatttataca ttaaaggctc tgggtctact 60
gcaaatttag ccagttcaaa ttattttcct acacctagtg gttctatggt tacctctgat 120
gcccaaatat tyaataaacc ttattggtta caacgagcac agggccacaa taatggcatt 180
tgttggggta accaactatt tgttactgtt gttgatacta cacgcagtac aaatatgtca 240
ttatgtgctg 250

<210> 69
<211> 250
<212> DNA
<213> Papillomaviridae

<220>
<221> VPHB
<223> misc_feature

<400> 69
gtgggcatcc attttataat aaattagatg acactgaaag ttcccatgcc gcyacgtcta 60
atgtttctga ggacgttagg gacaatgtgt ctgtagatta taagcagaca cagttatgta 120
ttttgggctg tgcccctgct attggggaac actgggctaa aggcactgct tgtaaatcgc 180
gtcctttatc acagggcgat tgcccccctt tagaacttaa aaacacagtt ttggaagatg 240
gtgatatggt 250

<210> 70
<211> 250
<212> DNA
<213> Papillomaviridae

<220>
<221> VPHC
<223> misc_feature

<400> 70
acactgaaaa ctctaataga tatgccggtg gtcctggcac tgataatagg gaatgtatat 60
cratggatta taaacaaaca carctgtgtt tacttggttg caaaccacct attggagagc 120
attggggtaa rggtagtcct tgtagtaaya atgctartac ccctggtgat tgtcctccat 180
tagaattaaa raatwcagtt atacaagatg gggatatggt tgatacaggc tttggagcta 240
tggattttac 250

<210> 71
<211> 250
<212> DNA
<213> Papillomaviridae

<220>
<221> VPHD
<223> misc_feature

<400> 71
atgggcatgt gtaggccttg aaataggtag agggcagcca ttaggcgttg gcataagtgg 60
tcatcctyta ttaaacaaat ttgatgacac tgaaaccrgt aacargtatc ctggacaacc 120
gggtgctgat aatagggaat gtttatccat ggattataaa caaacacagt tatgtttact 180
tggatgtaag cctccaacag gggaacattg gggtaaaggt gttgcttgta ctaatgcagc 240
acctgccaat 250

<210> 72
<211> 250
<212> DNA
<213> Papillomaviridae

<220>
<221> VPHE
<223> misc_feature

<400> 72
taaggcatgg tgaagaatat gatttacagt ttatttttca gttatgtaaa ataacactaa 60
cagcagatgt tatgacatat attcatagta tgaacccgtc cattttagag gattggaatt 120
ttggccttac accaccgcct tctggtacct tagaggacac atatcgctat gtaacatcac 180
aggctgtaac ttgtcaaaaa cccagtgcac caaaacctaa agatgatcca ttaaaaaatt 240
atactttttg 250

<210> 73
<211> 250
<212> DNA
<213> Papillomaviridae

<220>
<221> VPHF
<223> misc_feature

<400> 73
gtgtatttgc ctccaccttc tgtggcgaag gttgtcaata ctgatgatta tgttacacgc 60
acaggcatat attattatgc tggcagctct agattattaa cagtaggaca tccatatttt 120
aaagtgggta tgaatggtgg tcgcaagcag gacattccaa aggtgtctgc atatcaatat 180
agggtatttc gcgtgacatt gcccgatcct aataaattca gtattccaga tgcatcctta 240
tataatccag 250

<210> 74
<211> 250
<212> DNA

<213> Papillomaviridae

<220>
<221> VPHG
<223> misc_feature

<400> 74
cgtggcactg ttagrtttag tagattgggt caaagggcaa ccatgtttac acgtagtggt 60
aaacaaatag ggggtagggt acatttttac catgatataa gccccattgc tgctacagag 120
gaaattgaat tgcagccttt amttagtgct acagatgata gtgacctgtt tgatgtatat 180
gcagacttyc cacctcctgc gtccactaca cctagcacta taaacaaatc atttacatat 240
ccaaagtatt 250

<210> 75
<211> 250
<212> DNA
<213> Papillomaviridae

<220>
<221> VPHH
<223> misc_feature

<400> 75
gagtgggttr ggtatatttt tgggtggcct aggtattggt ackgggtctg gatctggggg 60
gcgtactgga tatatccctt taggtggtgg gggtcgccca ggcgtggtgg atattgctcc 120
tgcaaggcca cctattataa ttgaacctat agcacctact gaaccttcta tagtaaattt 180
ggttgavgac tctagtatta ttcagtctgg gtctcctata cctaccttta ctggtaccga 240
tggctttgaa 250

<210> 76
<211> 250
<212> DNA
<213> Papillomaviridae

<220>
<221> VPHI
<223> misc_feature

<400> 76
gtacaaagca gtgctttttt tcctactcct agtggttcta tggtaacctc agaatcccaa 60
ttatttaata aaccgtactg gttacaacgt gcgcagggcc acaataatgg catatgttgg 120
ggcaatcagt tgtttgtcac agttgtggat accactcgta gcactaacat gactttatgt 180
gctgaggtta aaaaggaaag cacatataaa aatgaaaatt ttaaggaata ccttcgtcat 240
ggcgaggaat 250

<210> 77
<211> 250
<212> DNA
<213> Papillomaviridae

<220>
<221> VPHJ
<223> misc_feature

<400> 77
aaataataat gttatagaag atagtaggga caatatatca gttgatggca agcaaacaca 60
gttgtgtatt gttggatgta ctcccgctat gggtgaacat tggactaaag gtgctgtgtg 120
taagtccaca caagttacca caggggactg cccgcctctt gcattagtta atacacctat 180
agaggatggg gacatgatag acacaggatt tggcgctatg gactttaagg tgttgcagga 240
atctaaggct 250

<210> 78
<211> 250

<212> DNA
<213> Papillomaviridae

<220>
<221> VPHK
<223> misc_feature

<400> 78
tgcgtcgcag acgtaaacgt tttccatatt tttttgcaga tgtccgtgtg gcggcctagt 60
gaggccactg tgtacctgcc tcctgtgcct gtgtctaagg ttgtaagcac tgatgaatat 120
gtgtcacgca caagcattta ttattatgct ggcagttcca gacttttggc tgttggcaat 180
ccatattttt ccatcaaaag tcccaataac aataaaaaag tattagttcc caaggtatca 240
ggcttacagt 250

<210> 79
<211> 250
<212> DNA
<213> Papillomaviridae

<220>
<221> VPHL
<223> misc_feature

<400> 79
ttcctaaggt gtctgcatat caatacagag tatttagggt taakttacct gatccyaata 60
aattyggcct tccagataac acagtatatg atcctaactc tcaacgcytg gtcwgggcct 120
gtgtaggtgt tgaaatcggt cggggccaac ctttaggggt aggactyagt ggtcatccat 180
tmtataataa attrgatgac actgaaaact ctcatgtagc atctgctgtt gatamcaaag 240
atacacgtga 250

<210> 80
<211> 250
<212> DNA
<213> Papillomaviridae

<220>
<221> VPHM
<223> misc_feature

<400> 80
atatatacag ggagcyacat ttgcactatg gcctgtatat ttttttaaac gtaggcgccg 60
taaacgtatt ccctattktt ttgcagatgg cgatgtggcg gcctagtgac aataaggtgt 120
acctacctcc aacacctgtt tcaaaggttg tggcaacgga tacatatgta aaacgtacca 180
gtatatttta tcatgcaggt agctctaggt tgcttgctgt tggccatcct tattactctg 240
tttcyaaatc 250

<210> 81
<211> 250
<212> DNA
<213> Papillomaviridae

<220>
<221> VPHN
<223> misc_feature

<400> 81
atggcattgt ggcgmkctag cgacaacatg gtgtatttgc ctcccccctc agtggcgaag 60
gttgtcaata cagatgatta ygtracacgc actggcatkt attaytatgc tggtacatct 120
aggttattaa ctgtaggcca tccatatttt aargtyccta tgtctgggrg ccgyaagcag 180
grcattccta aggtgtctgc atatcaatay agrgtgtttm grrttwccyt rccygatcct 240
aataaattta 250

<210> 82

<211> 250
<212> DNA
<213> Papillomaviridae

<220>
<221> VPHO
<223> misc_feature

<400> 82
ccttttataa tcctgataag gagcgcctag tatgggcctg ttctggtgtg gaggttggac 60
gtggacaacc cttaggtata ggtactagtg gcaatccatt tatgaataaa ttrgatgata 120
ctgaaaatgc tcctaaatac attgctggac aaaatacaga tggtagagaa tgtatgtcag 180
tggattataa acaaacacag ttgtgtattt taggttgtag gcctccctta ggggaacatt 240
ggggtccagg 250

## Description

### CROSS-REFERENCE TO RELATED APPLICATIONS

This application claims benefit of priority under 35 U.S.C. §119(e) of the Netherlands Provisional Application No. 1042665, filed January 10, 2018, and also to U.S. Serial No. 62/530,245, filed July 9, 2017. The disclosure of the prior application is considered part of and is incorporated by reference in the disclosure of this application in its entirety.

### BACKGROUND OF THE INVENTION

### FIELD

The present invention is related to determining the susceptibility of cancer and, more particularly, to the simultaneous detection and identification of HPV strain genotypes and genetic markers for cancer susceptibility.

### INTRODUCTION

Cancer is one of the main causes of morbidity and mortality worldwide accounting for 14 million new cases and 8.2 million deaths in 2012. Of these, prostate and cervical are among the most common causes of cancer-related deaths.

The etiology of a particular type of cancer is probably associated to a set of genetic variants, many of which interact with environmental factors. Epidemiological studies supported by considerable evidence had demonstrated the role of single nucleotide polymorphisms (SNP's) as a hereditary component in different types of cancer.

Infection with human papilloma viruses (HPVs) is a primary risk factor in the development of numerous cancers. As such HPVs are responsible for the development of nearly all cervical cancers. Furthermore, HPVs have also been implicated in the development of prostate cancers.

In addition to HPV, other risk factors may be involved in the development of cervical and prostate cancers. For example, mutations may play role in 5 to 10 % or all cancer developments. Thus, mutations in cervical and prostate tissues may also constitute risk factors for cancer.

Several methods well known in the art can be used to isolate nucleic acid and to detect mutations in DNA sequences. For example, Ravinder (WO2004079011) provides methods for isolating free nucleic acids through formalin addition plus amplification of the locus of interest, hybridization, washing, staining and scanning GeneCHIP® array for detection of genetic disorders associated with chromosomal abnormalities by non-invasive sampling. Under a complex procedure that involves Q-beta phage amplification, strand displacement amplification, and splice overlap extension polymerase chain reaction, amplicon fragmentation by exonuclease digestion and BeadArray Technology. Additionally, a multiplex PCR, sequencing techniques for SNPs detection, and claims the detection of sequences from different species including human are described, although there is no genotyping of HPV and/or any other microorganism. Furthermore, there is no description of a method for cancer propensity detection but a prenatal diagnostic method comprising analyzing a composition comprising fetal DNA and maternal DNA.

Another microarray based patent describes a method for simultaneous pathogenic and host genetic sequences detection (WO2009095840). Nonetheless, genotyping of specific HPV viral types, nor specific SNPs for cervical cancer, prostate cancer or other specific types of cancer are not described. Furthermore, the use of microarrays is a time and cost consuming technology.

Giffard et.al. (WO2007109854), discloses methods for SNPs and cell or cells genotyping. These methods are applicable on either eukaryotic cells (including cancer cells) or prokaryotic cells (including several types of bacteria). The only technique claimed on this patent is real-time PCR, where multiplex analysis is limited to the number of fluorophores detected by commercial thermocyclers. Besides, compatibility between primers or probes designed for real-time PCR with different platforms (such as sequencing or others) is not claimed. Furthermore, neither viral genotyping (i.e. HPV) nor non-invasive methods for sample preparation are disclosed. Xenomics, (WO2010051261) claims the use of the EI gene fragment of papillomavirus genome as a specific marker for differential diagnosis by detection of most common risk HPV genotypes against low risk. The patent indicates several molecular technics including PCR and real-time PCR but no sequencing nor magnetic beads. It also describes the use of nucleic acids isolated from urine sample but no host genome sequences nor SNPs identification.

There are several ways to obtain a cancer diagnostic, including patient history, physical tests, evaluation risk polymorphisms and finally, genetic predisposition tests. Physical tests refer to imaging and scans, the most common of these being x-rays, ultrasounds and MRI's. They can also include PET scans and endoscopies. As far as the identification of polymorphisms, there are tests that detect specific analytes associated with cancer such as proteins or metabolic products. Finally, genetic tests can identify a change at the molecular level directly in the DNA or RNA of an individual. These changes are usually associated with an increased risk of the formation of neoplasia.

These associations with cancer are of great importance, because of all the tests previously mentioned, only the genetic tests are of a preventive nature. That is, they can detect a potential threat before it appears. As a result, an individual can take precautionary measures such as a change in habits, diet, and constant monitoring. All of this can be done before the pathology emerges and can be quite effective at preventing the development of the disease.

Currently, there are different techniques to detect genetic risk polymorphisms. For example, genome sequencing, quantitative Polymerase chain reaction (qPCR), DNA microarrays and restriction fragment length polymorphism technique (RFLPs). These techniques are widely used, not just for cancer research and well known sophisticated techniques. However, they do have drawback regarding their precision, repeatability, time of analysis, and cost of realization. Also, they usually require an invasive blood sample taken by a qualified phlebotomist.

These precedents show the need to develop new techniques for the prevention of cervical and prostate cancer that are sensitive, quick and cost effective. The present invention looks to answer this need by simultaneous detecting several strains of the HPV and risk polymorphisms associated with cancer. Not only that, it aims to do so with a non-invasive sample taken from the urine of either men or women. This non-invasive technique would allow a patient to take their own sample safely and painlessly cutting down not only risk and stress to the patient, but overall cost in the diagnostic procedure.

This is a major shift from the invasive techniques of the blood draw or the Pap smear form women, in which patients must travel to an authorized clinic and request a specialized analyst. It has been determined that urine can in fact be used as an acceptable fluid sample in clinical practices and has even been found to successfully house the presence of genomic and viral DNA from HPV when an infection is present. Not only that, but a non-invasive urine test is much safer for a patient than a pap smear that can occasionally create micro lesions around the infected area of a female patient creating an inflammatory response

Accordingly, herein is described new methods and kits for simultaneously determining whether HPV strain genotypes and genetic markers for cancer susceptibility are present in a sample from a subject.

Thus, in various embodiments, the present invention is directed to methods for simultaneously determining HPV strain genotypes and genetic markers for cancer susceptibility in a sample from a subject.

Koninkl (WO2009095840) describes a method for simultaneous pathogenic and host specific factors detection in a liquid sample comprising the steps of extracting DNA outside the human or animal body from a tissue sample; solubilizing DNA to obtain a liquid sample comprising host- DNA and/or pathogen-DNA; loading the liquid sample into the chamber of the microarray device in order to contact the probe nucleotides of said microarray device with the liquid sample and detecting interactions between the host-DNA and/or the pathogen- DNA comprised in the liquid sample and the probe nucleotides. In this method the DNA is amplified after extracting step using multiplex-PCR with primers specific for host-DNA and pathogens-DNA regions. And the microarray device comprising probe oligonucleotides being CAPable of binding host specific DNA factors implicated in the susceptibility to and/or prognosis of said specific disease, and probe oligonucleotides being CAPable of binding DNA pathogens caused a specific disease and any cancer caused by pathogens such as stomach cancer and cervical cancer. In this method up to 20-50 probes for detecting pathogenic DNA can be used and up to 30-40 probes for detecting markers in host DNA can be used.

Nonetheless, the invention do not include genotyping of specific HPV viral types, nor specific SNPs for cervical cancer, prostate cancer or other specific types of cancer. The present application differs with this work in that specific HPV strain genotypes are chosen and by the use of an oligonucleotide ligation assay instead of the time-consuming and expensive microarray. WO2009/095840 discloses that SNPs are detected, which lead to susceptibility to infection by the pathogen in the DNA of the host, whereby sensitivity to the development of cervical cancer or stomach cancer is also mentioned.

On the other hand, Rafnar et.al. (WO2010018601), describes a method to determine cancer risk or protection from the identification of one or more polymorphic marker rs401681, rs2736100 and rs2736098. The method comprising the presence of at least one allele of at least one polymorphic marker, either from a nucleic acid sample from human or in a genotype dataset from the individual. This robust work claims methods, software and hardware for determining susceptibility to cancer in a human individual or based on data indicative of at least one polymorphic marker. It also claims a method of assessing an individual for probability of response to a cancer therapeutic agent. The molecular methods for identification of the polymorphisms are based on amplification by PCR wherein genotyping is performed using a process selected from allele-specific probe hybridization, allele-specific primer extension, allele-specific amplification, nucleic acid sequencing, 5'-exonuclease digestion, molecular beacon assay, oligonucleotide ligation assay, size analysis, single-stranded conformation analysis and micro array technology. Nevertheless, nor multiplex PCR conditions nor magnetic beads are described and the multiplex CAPacity on the chemical methods are not specified. It involves a robust cancer types scope including determining risk or protection against prostate, cervical, breast, lung, skin, colorectal, and thyroid cancers according to the presence/absence of at least one allele of at least one of the three polymorphisms indicated previously.

WO2010018601 further, discloses a method for determining the sensitivity of a human to develop cervical cancer in an infection with HPV16, HPV18, HPV31, HPV33 HPV45 or any other HPV strain genotypes detecting the presence of SNPs in markers by amplifying extracted DNA from a sample obtained from the patient using PCR and then a hybridization assay, such as oligonucleotide ligation assay.

### SUMMARY OF THE INVENTION

The present invention is based on the seminal discovery of the determination of susceptibility to cancer by simultaneous identification and detection of HPV infection and the genotyping of cancer susceptibility markers.

In one embodiment, the present invention provides methods for assessing cancer susceptibility in a subject by simultaneously identifying one or more cancer susceptibility marker genes and one or more human papillomavirus (HPV) strain genotypes, thereby assessing cancer susceptibility in the subject. In one aspect, identifying the one or more cancer susceptibility marker genes is by amplifying a cell free DNA sample from the subject using primers to the one or more cancer susceptibility marker genes; and detecting the amplified DNA. In another aspect, amplifying the cell free DNA sample also uses primers to the one or more HPV strain genotypes. In an additional aspect, the cancer is cervical, prostate, colon, pancreatic, gastric, lung, leukemia and breast. In certain aspects, the one or more cancer susceptibility marker genes is CACUA. CACUB, CACUC, CACUD, CACUE, CACUF, CACUG, CACUH, CACUI, CACUJ, CAPA, CAPB, CAPC, CAPD, CAPE, CAPF, CAPG, CAPH, CAPI, CAPJ, COLA, COLB, COLC, COLD, COLE, COLF, COLG, GASA, GASB, GASC, GASD, GASE, GASG, LEUA, LEUB, LEUC, LEUD, LEUE, LEUF, MAMA, MAMB, MAMC, MAMD, MAME, MAMF, MAMG, PROA, PROB, PROC, PROD, PROE, PROF, PANA, PANB, PANC, PAND, PULA, PULB, PULC, PULD, PULE, PULF, PULG, GENA, GENB, GEND, GENE, GENG, GENH and/or GENI; a single nucleotide polymorphism (SNP) located within the gene encoding for IL10, TGF-β1, CYP1A1, ST3GAL4, IL4, AGTR1, ERBB2, HOXB13, Chr8, MSMB, RNASEL, CYP19, ABO, ABL1, ARID5B, Chr13, CCKBR, JAK2, NQO1, EGFR, EPHX1, CHRNA3, IRF4, PIK3CA, CXCL8, BRCA2, BRCA1, ATM, TCF2, TGFB1, KiSS1, APC, PGR, MTHFR, MUTYH, CDH1, IL1B, IL6, TLR4, TNF-α, MDM2, MLH1, BRAF, TP53, BCAR1, STK11, KRAS, FGFR2, SRD5A2, EIF3H and/or MSH6; and/or SEQ ID NOs:1-67. In some aspects the one or more cancer susceptibility marker genes is specific for cervical cancer (CACUA. CACUB, CACUC, CACUD, CACUE, CACUF, CACUG, CACUH, CACUI, and/or CACUJ); specific for prostate cancer (CAPA, CAPB, CAPC, CAPD, CAPE, CAPF, CAPG, CAPH, CAPI, and/or CAPJ); specific for colon cancer (COLA, COLB, COLC, COLD, COLE, COLF, and/or COLG); specific for gastric cancer (GASA, GASB, GASC, GASD, GASE, and/ or GASG); specific for leukemia (LEUA, LEUB, LEUC, LEUD, LEUE, and/or LEUF); specific for breast cancer (MAMA, MAMB, MAMC, MAMD, MAME, MAMF, and /or MAMG), PROA, PROB, PROC, PROD, PROE, PROF; specific for pancreatic cancer (PANA, PANB, PANC and/or PAND), specific for lung cancer (PULA, PULB, PULC, PULD, PULE, PULF, and/or PULG); and/or GENA, GENB, GEND, GENE, GENG, GENH and/or GENI. In a further aspect, the one or more HPV strain genotype is HPV-16, HPV-18, HPV-31, HPV-33, HPV-35, HPV-39, HPV-45, HPV-51, HPV-52, HPV-56, HPV-58, HPV-59, HPV-66, HPV-68, HPV-73 and/or SEQ ID NOs:68-82. In one aspect, the one or more HPV strain genotypes is a high-risk strain. In a specific aspect, the HPV strain genotype is HPV-16, HPV-18, HPV-31, HPV-45 and/or HPV-58. In a further aspect, the HVP strain genotype is determined by detecting HPVA, HPVB, HPVC, HPVD, HPVE, HPVF, HPVG, HPVH, HPVI, HPVJ, HPVK, HPVL, HPVM, HVPN, and/or HPVO. In an additional aspect, the detecting of the amplified DNA is performed by oligo ligation assay (OLA), PCR, qPCR, DNA sequencing, fluorescence, gel electrophoresis, magnetic beads, allele specific primer extension (ASPE) and/or direct hybridization. In another aspect, the OLA uses probes to the one or more cancer susceptibility marker genes. In a further aspect, the OLA also uses probes to the one or more HPV strain genotypes. In one aspect, the sample is a saliva, swab, blood or urine sample. Thus, in various embodiments, the present invention is directed to methods for simultaneously determining HPV strain genotypes and genetic markers for cancer susceptibility in a sample from a subject. The methods include a) providing a sample from the subject, b) obtaining cell-free DNA from the sample, c) amplifying the cell-free DNA with end-point PCR using primers to one or more HPV strain genotypes and primers to one or more cancer susceptibility marker genes; d) performing an oligonucleotide ligation assay (OLA) on the amplified cell-free DNA using probes to the one or more HPV strain genotypes and probes to the one or more cancer susceptibility markers and e) determining whether the one or more HPV strain genotypes and the one or more cancer susceptibility markers are present in the amplified cell-free DNA. In various embodiments, the sample is a urine sample. The one or more HPV strain genotypes may include a plurality of high risk HPV strain genotypes. The one or more cancer susceptibility markers may include a plurality of cervical cancer susceptibility markers or a plurality of prostate cancer susceptibility markers.

In an additional embodiment, the present invention provides methods performed by amplifying a cell free DNA sample from a subject using primers to one or more cancer susceptibility marker genes and to one or more human papillomavirus (HPV) strain genotypes; and detecting the amplified DNA. In one aspect, the one or more cancer susceptibility marker genes are markers for cervical, prostate, colon, pancreatic, lung, breast, gastric cancer or leukemia. In an additional aspect, the one or more cancer susceptibility marker genes is CACUA. CACUB, CACUC, CACUD, CACUE, CACUF, CACUG, CACUH, CACUI, CACUJ, CAPA, CAPB, CAPC, CAPD, CAPE, CAPF, CAPG, CAPH, CAPI, CAPJ, COLA, COLB, COLC, COLD, COLE, COLF, COLG, GASA, GASB, GASC, GASD, GASE, GASG, LEUA, LEUB, LEUC, LEUD, LEUE, LEUF, MAMA, MAMB, MAMC, MAMD, MAME, MAMF, MAMG, PROA, PROB, PROC, PROD, PROE, PROF, PANA, PANB, PANC, PAND, PULA, PULB, PULC, PULD, PULE, PULF, PULG, GENA, GENB, GEND, GENE, GENG, GENH and/or GENI; a single nucleotide polymorphism (SNP) located within the gene encoding for IL10, TGF-β1, CYP1A1, ST3GAL4, IL4, AGTR1, ERBB2, HOXB13, Chr8, MSMB, RNASEL, CYP19, ABO, ABL1, ARID5B, Chr13, CCKBR, JAK2, NQO1, EGFR, EPHX1, CHRNA3, IRF4, PIK3CA, CXCL8, BRCA2, BRCA1, ATM, TCF2, TGFB1, KiSS1, APC, PGR, MTHFR, MUTYH, CDH1, IL1B, IL6, TLR4, TNF-α, MDM2, MLH1, BRAF, TP53, BCAR1, STK11, KRAS, FGFR2, SRD5A2, EIF3H and/or MSH6; and/or SEQ ID NOs:1-67. In a further aspect, the one or more HPV strain genotypes is HPV-16, HPV-18, HPV-31, HPV-33, HPV-35, HPV-39, HPV-45, HPV-51, HPV-52, HPV-56, HPV-58, HPV-59, HPV-66, HPV-68 and/or HPV-73. In certain aspects, the detecting of the amplified DNA is performed by oligo ligation assay, PCR, DNA sequencing, fluorescence, gel electrophoresis, magnetic beads, allele specific primer extension (ASPE) and/or direct hybridization. In specific aspects, the PCR is qPCR, multiplex PCR or nested PCR In one aspect, the sample is a saliva, swab, blood or urine sample.

In an additional aspect, the present invention provides a kit containing at least primers to one or more cancer susceptibility marker genes; primers to one or more human papillomavirus (HPV) strain genotypes; and instructions for use. In one aspect, the one or more cancer susceptibility marker genes is CACUA. CACUB, CACUC, CACUD, CACUE, CACUF, CACUG, CACUH, CACUI, CACUJ, CAPA, CAPB, CAPC, CAPD, CAPE, CAPF, CAPG, CAPH, CAPI, CAPJ, COLA, COLB, COLC, COLD, COLE, COLF, COLG, GASA, GASB, GASC, GASD, GASE, GASG, LEUA, LEUB, LEUC, LEUD, LEUE, LEUF, MAMA, MAMB, MAMC, MAMD, MAME, MAMF, MAMG, PROA, PROB, PROC, PROD, PROE, PROF, PANA, PANB, PANC, PAND, PULA, PULB, PULC, PULD, PULE, PULF, PULG, GENA, GENB, GEND, GENE, GENG, GENH, GENI and/or SEQ ID NO:1-67; a single nucleotide polymorphism (SNP) located within the gene encoding for IL10, TGF-β1, CYP1A1, ST3GAL4, IL4, AGTR1, ERBB2, HOXB13, Chr8, MSMB, RNASEL, CYP19, ABO, ABL1, ARID5B, Chr13, CCKBR, JAK2, NQO1, EGFR, EPHX1, CHRNA3, IRF4, PIK3CA, CXCL8, BRCA2, BRCA1, ATM, TCF2, TGFB1, KiSS1, APC, PGR, MTHFR, MUTYH, CDH1, IL1B, IL6, TLR4, TNF-α, MDM2, MLH1, BRAF, TP53, BCAR1, STK11, KRAS, FGFR2, SRD5A2, EIF3H and/or MSH6; and/or SEQ ID NOs: 1-67. In another aspect, the one or more HPV strain genotypes are HPV-16, HPV-18, HPV-31, HPV-33, HPV-35, HPV-39, HPV-45, HPV-51, HPV-52, HPV-56, HPV-58, HPV-59, HPV-66, HPV-68 and/or HPV-73 and/or SEQ ID NOs:68-82. In an additional aspect, the kit also contains probes to the one or more one or more cancer susceptibility marker genes and/or probes to the one or more HPV strain genotypes.

The present invention further improves on the disclosed methods and processes, presenting an innovative multiplex method for simultaneous HPV genotyping and several different SNPs identification for different types of cancers predisposition.

These and other features, aspects and advantages of the present teachings will become better understood with reference to the following description, examples and appended claims.

### BRIEF DESCRIPTION OF THE DRAWINGS

Those of skill in the art will understand that the drawings, described below, are for illustrative purposes only. The drawings are not intended to limit the scope of the present teachings in any way.

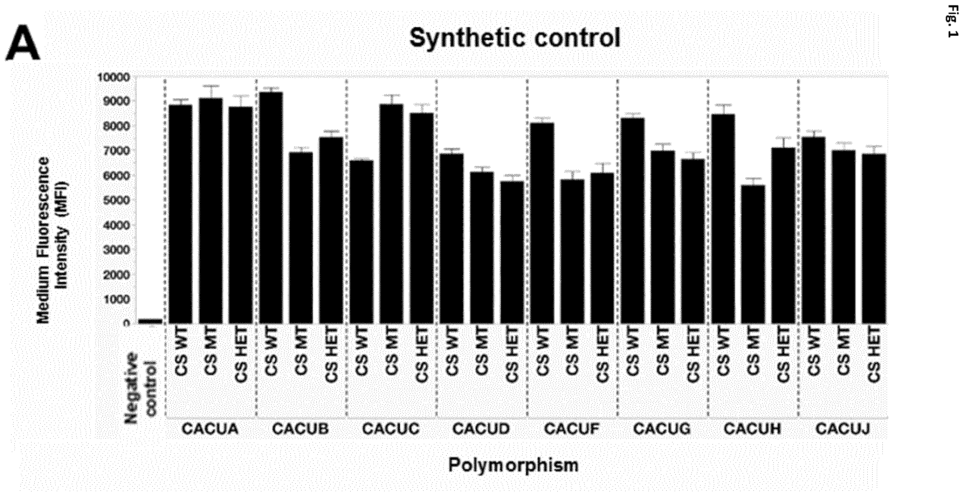

Figures 1 A-E illustrate the species specificity in the detection of SNPs associated with cervical cancer risk SNPs (CACU) using synthetic controls, biological control and DNA from 2 uropathogenic microorganisms. A. Detection of CACU risk SNPs A-J using wild type (WT), mutant (MT) and heterozygous (HET) synthetic controls (CS WT, CS MT and CS HET). B. Detection of CACU risk SNPs A-J using DNA isolated from biological control M1. C. Detection of CACU risk SNPs A-J using DNA isolated from *C. lupus familiaris.* D. Detection of CACU risk SNPs A-J using DNA isolated from *Escherichia coli.* E. Detection of CACU risk SNPs A-J using DNA isolated from the Human Immunodeficiency Virus, HIV.

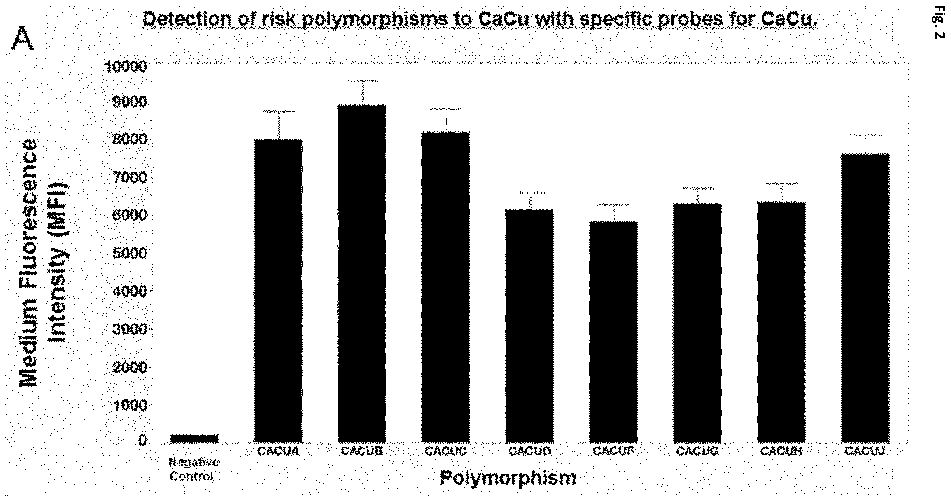

Figures 2A-B illustrate the intra-assay specificity in the detection of CACU risk SNPs A-J using the M1 biological control. A. Detection of CACU risk SNPs A-J using specific CACU probes. B Detection of CACU risk SNPs A-J using specific probes for HPV detection.

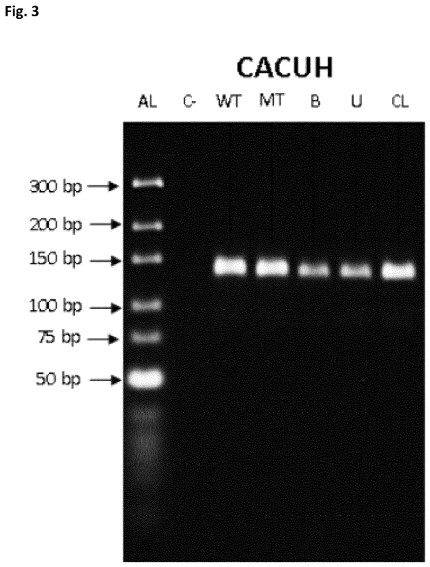

Figure 3 illustrates the results of representative PCR amplifications of the cervical cancer susceptibility SNP designated as CACUH in which AL indicates allelic ladder, C indicates no template control, WT indicates positive wild type DNA control, MT indicates positive mutant DNA control, B indicates DNA extracted from blood samples, U indicates DNA extracted from urine samples and CL indicates DNA extracted from cell lines that present the SNP.

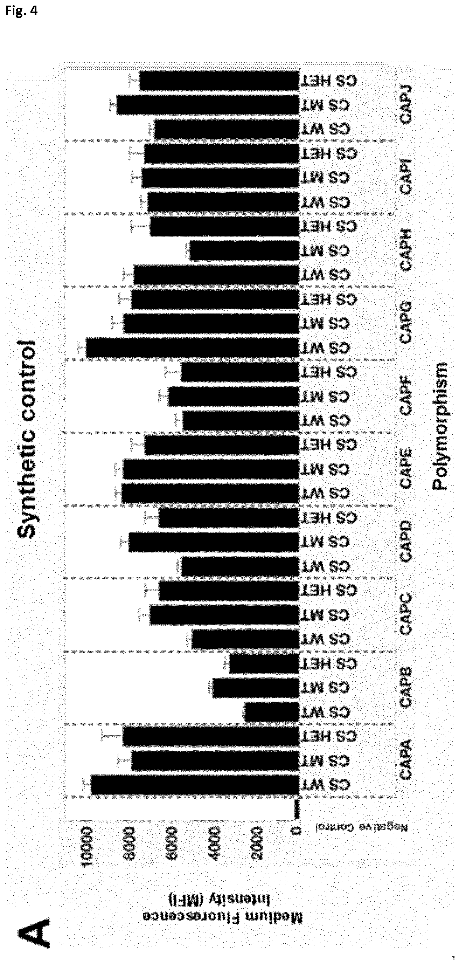

Figures 4A-E illustrate the species specificity in the detection of prostate cancer risk SNPs (CAP) using synthetic controls, a biological control sample and DNA from 7 uropathogenic microorganisms. A. Detection of CAP risk SNPs A-J using synthetic controls (CS WT, CS MT and CS HET). B. Detection of CAP risk SNPs A-J using DNA isolated from biological control M4. C. Detection of CAP risk SNPs A-J using 100 ng of DNA isolated from *C. lupus familiaris.* D. Detection of CAP risk SNPs A-J using 100 ng of DNA isolated from *Escherichia coli.* E. Detection of CAP risk SNPs A-J using 100 ng of DNA isolated from HIV.

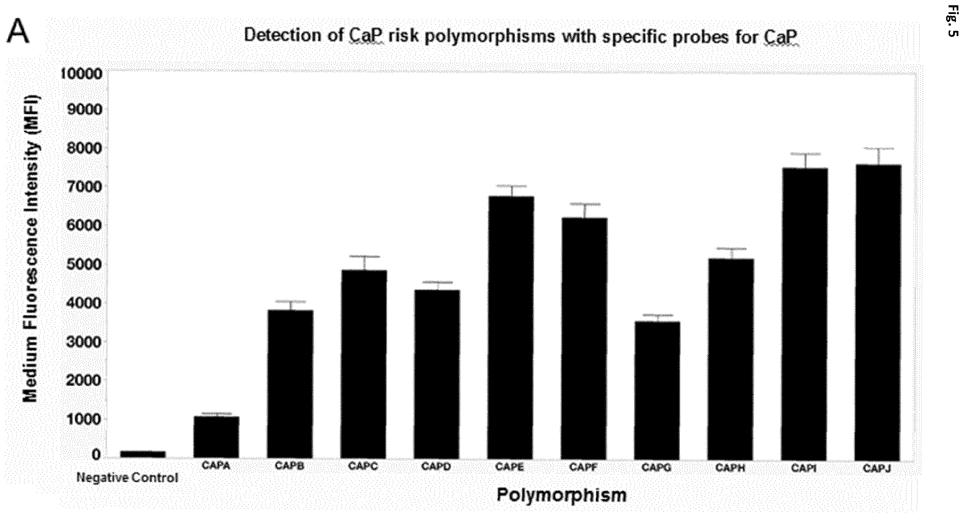

Figures 5A-B illustrate the intra-assay specificity in the detection of CAP risk SNPs A-J using the M4 biological control sample. A. Detection of CAP risk SNPs A-J using specific CAP probes. B. Detection of CAP risk SNPs A-J using specific probes for HPV detection.

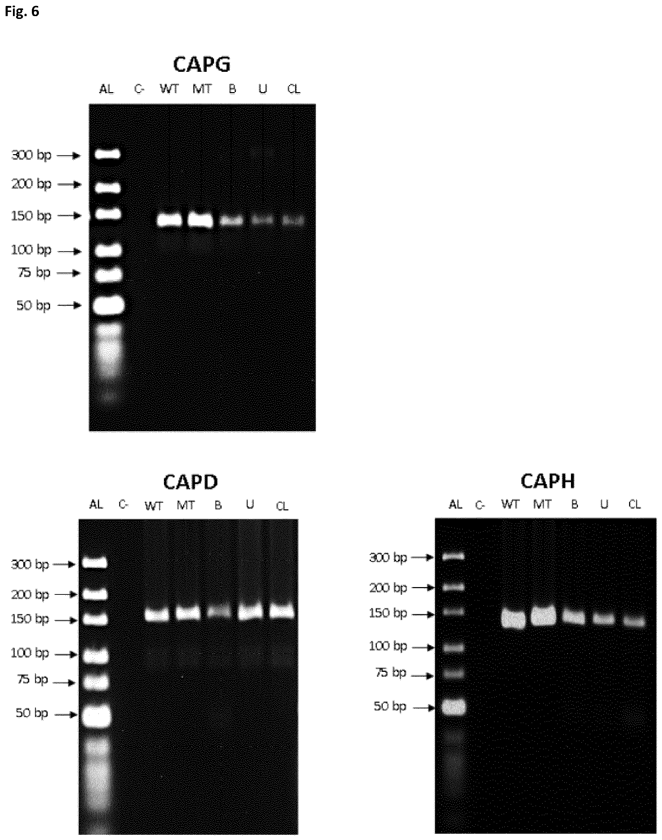

Figure 6 shows the results of representative PCR amplifications of prostate cancer susceptibility SNPs designated as CAPG, CAPD and CAPH, in which AL indicates allelic ladder, C- indicates no template control, WT indicates positive wild type DNA control, MT indicates positive mutant DNA control, B indicates DNA extracted from blood samples, U indicates DNA extracted from urine samples and CL indicates DNA extracted from cell lines that present the SNP.

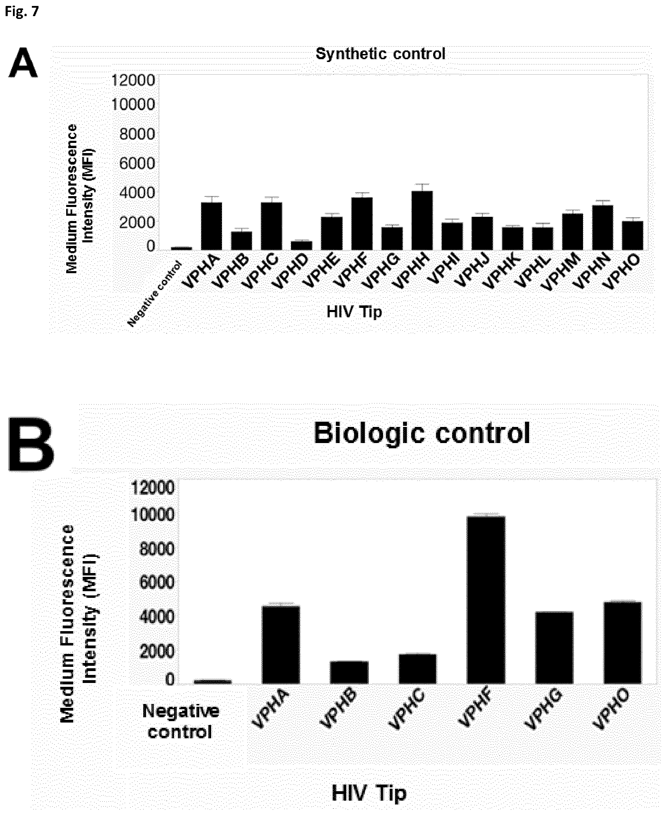

Figures 7A-E illustrate the species specificity in the detection of HPV DNA using synthetic controls and biological controls positive for HPVA, HPVB, HPVC, HPVF, HPVG and HPVO. A. Detection of HPVs A-O DNA using synthetic controls. B. Detection of HPV DNA using biological controls. C. Detection of HPV DNA using DNA isolated from *C. lupus familiaris.* D. Detection of HPV DNA using DNA isolated from *Escherichia coli.* E. Detection of HPV using DNA isolated from HIV.

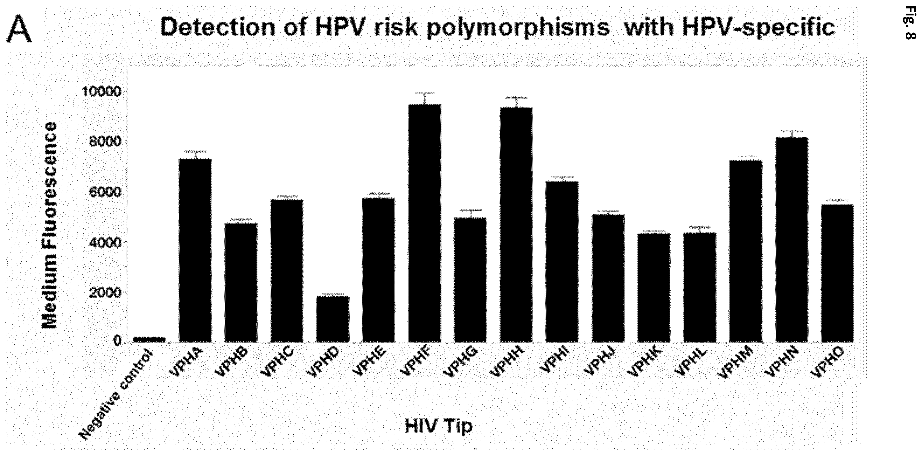

Figures 8A-C illustrate the intra-assay specificity in the detection of HPV DNA using synthetic controls. A. Detection of HPV DNA using specific HPV probes. B. Detection of HPV DNA using specific CACU probes. C. Detection of HPV DNA using specific CAP probes.

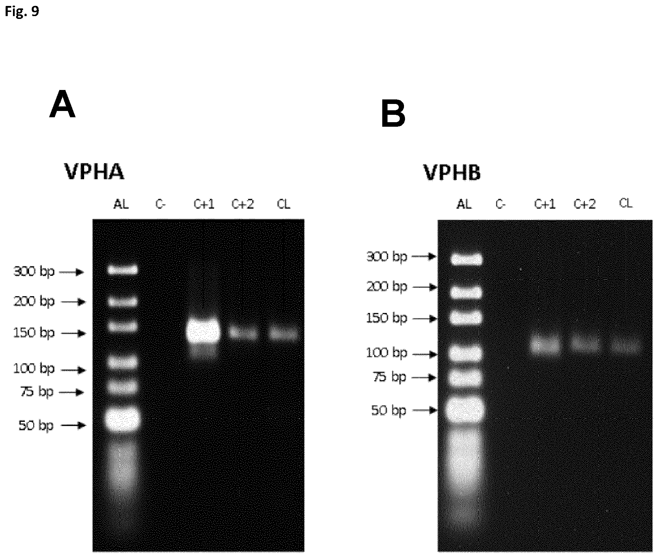

Figures 9A-B illustrate the results of representative PCR runs identifying two types of HPV in which AL indicates allelic ladder, C- indicates no template control, C+1 indicates synthetic positive control, C+2 indicates viral DNA and CL indicates DNA extracted from a cell line known to contain the corresponding virus. A. Representative PCR runs identifying HPVA. B. Representative PCR runs identifying HPVB.

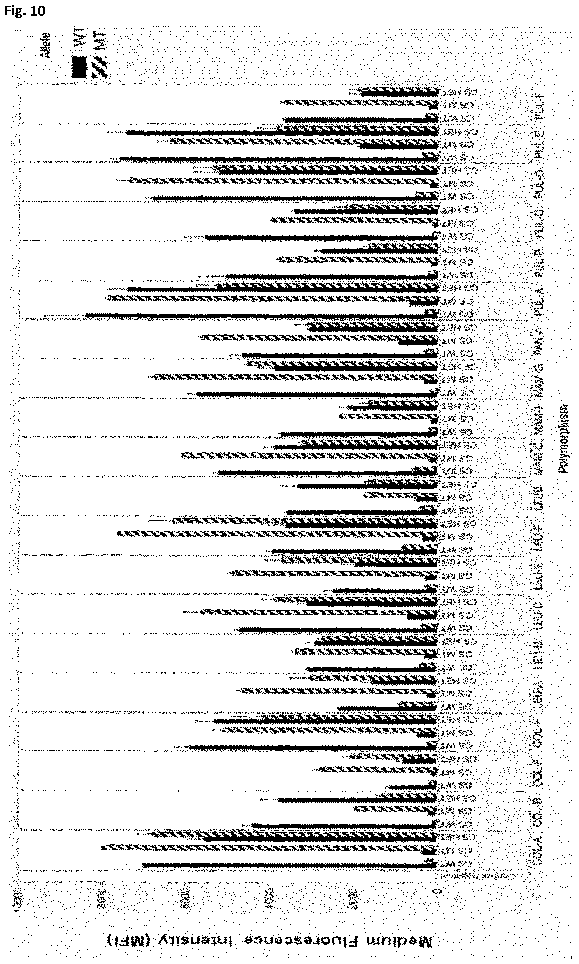

Figure 10 shows the identification of 20 cancer risk polymorphisms using synthetic controls by the GanPlex MR test (Panel A). The graph shows the MFI values recorded in each of the cancer risk polymorphisms when the Wilde type (CS WT), Mutant (MT) and Heterozygous (HET) synthetic controls were used. The bars represent the Average ± 1 D.E. of 2 replicas.

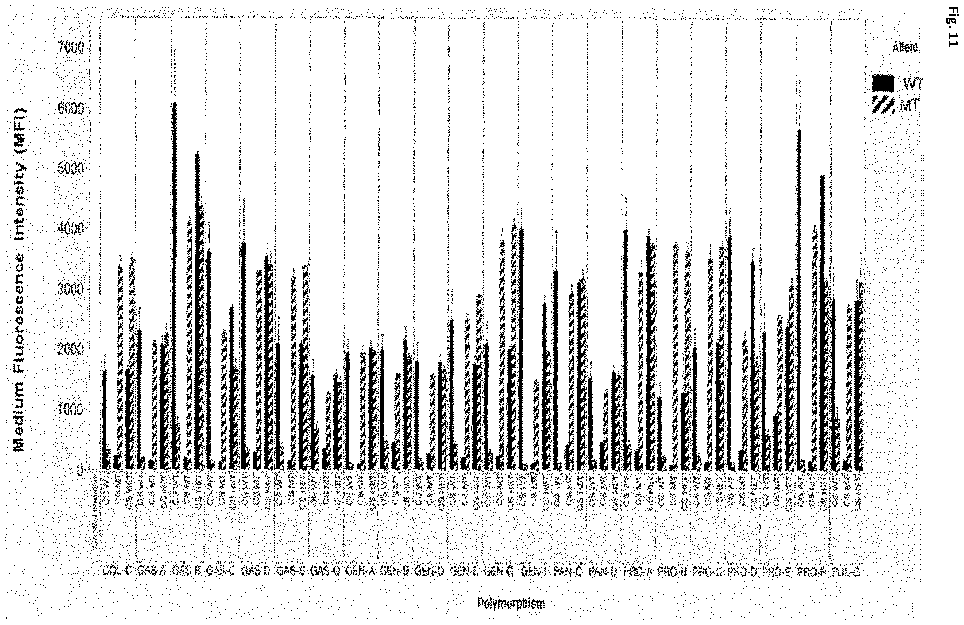

Figure 11 shows the identificatoin of 22 cancer risk polymorphisms using synthetic controls by the GanPlex MR test (Panel B). The graph shows the MFI values recorded in each of the cancer risk polymorphisms when the Wilde type (CS WT), Mutant (MT) and Heterozygous (HET) synthetic controls were used. The bars represent the Average ± 1 D.E. of 2 replicas..

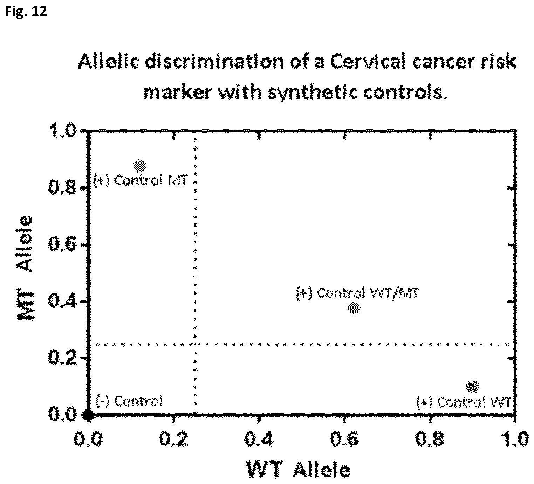

Figure 12 illustrates the allelic discrimination assay of a cervical cancer risk SNP showing the fluorescence associated with the wild type allele (WT) on the X axis, the fluorescence associated with a mutant allele (MT) on the Y axis, and delimiting fluorescence areas using a synthetic WT control, a MT synthetic control and a heterozygous (WT/MT) synthetic control.

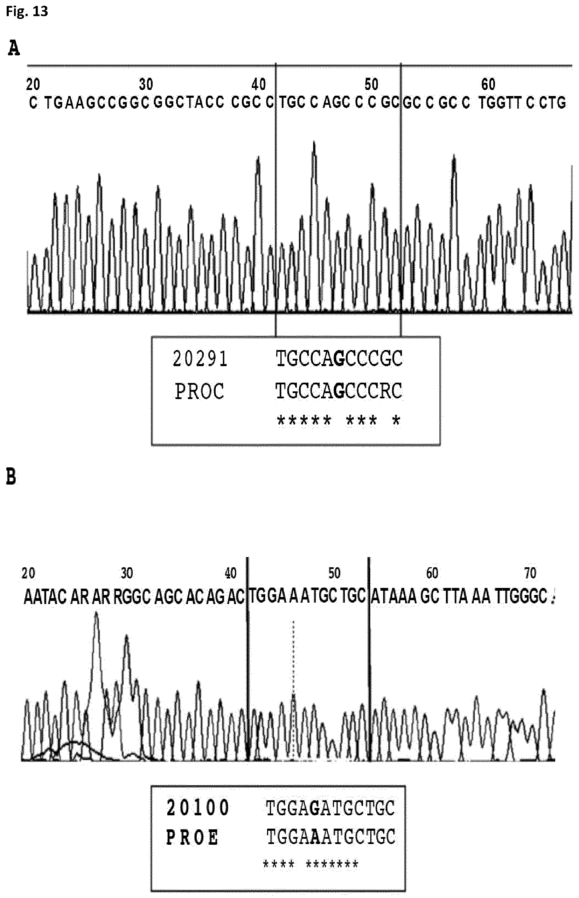

Figures 13A-B illustrate exemplary electropherograms according to various embodiments described herein.

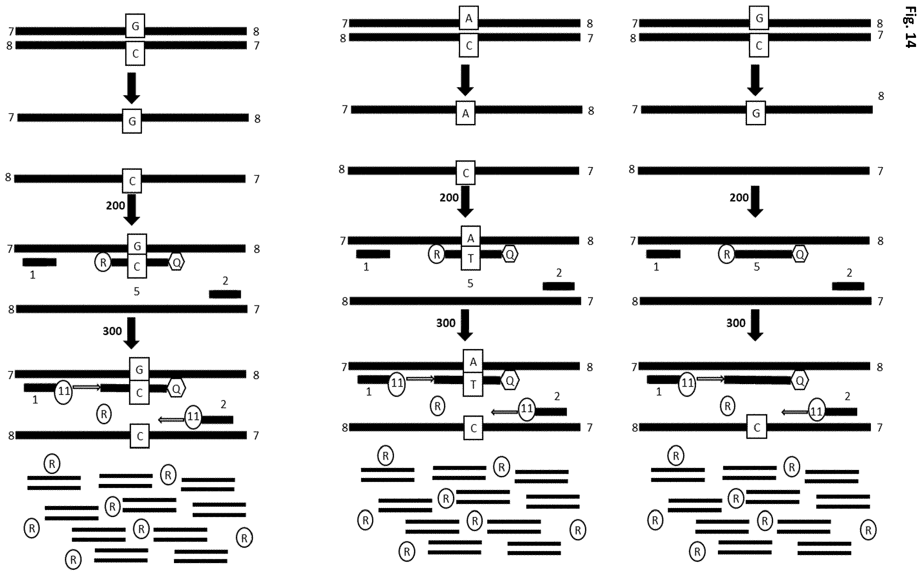

Figure 14 is a flow chart that illustrates the detection of target sequences previously amplified by PCR, using qPCR in the presence of SYBR™ Green or TaqMan™ probes labeled with different fluorophores, in which (1) indicates the forward oligonucleotide, (2) the reverse oligonucleotide, (5) the probe sequence, (6) the target sequence, (7) the 5 prime-end, (8) the 3 prime-end, (100) the denaturation step (95°C temperature), (200) the alignment step, and (300) the extension step (72°C temperature).

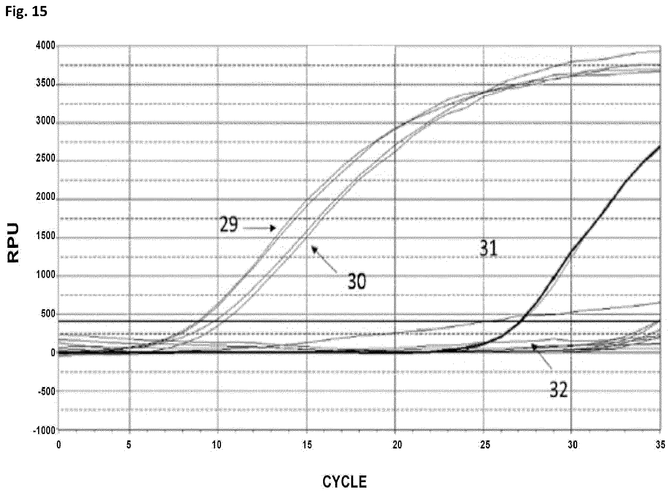

Figure 15 illustrates an exemplary of amplification plot showing the target sequence identified by qPCR using TaqMan™ probes labeled with three different fluorophores. The plot shows the analysis of one biological sample including their respective synthetic controls formed to identify one of the SNPs associated to prostate cancer, in which (29) indicates the amplification from Wild type positive synthetic control, (30) the amplification from Mutant positive synthetic control, (31) the amplification from Mutant biological sample, and (32) the amplification from Negative control.

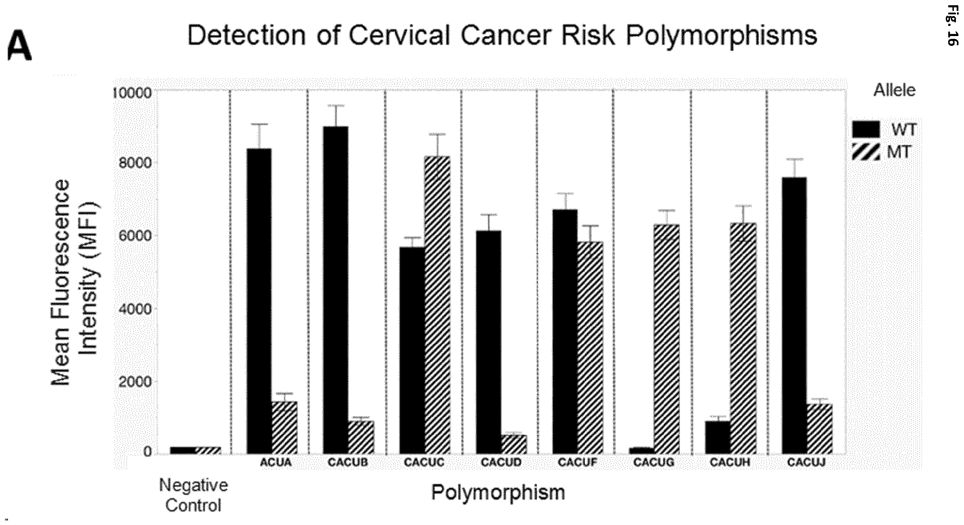

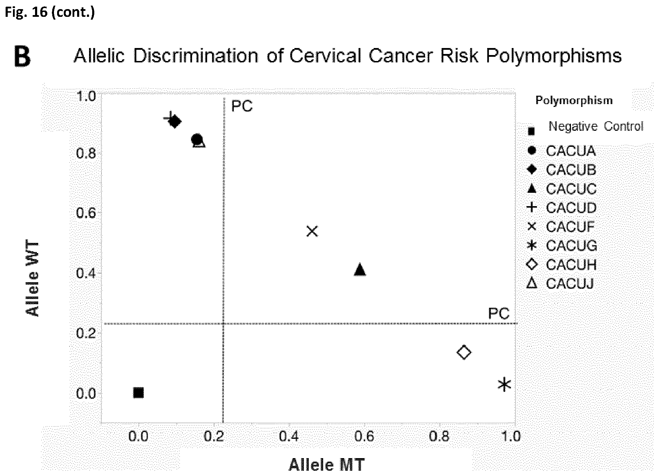

Figures 16 A-B show the identification and allelic discrimination of risk polymorphisms to CACU in M1 biological control sample by the Kimera-Test™ test. A: The MFI values recorded in each of the CACU risk polymorphisms identified in the biological control sample M1 are shown. The bars represent the Average ± 1 D.E. of 4 replicas. B: Allele discrimination graph originated from the obtained RA values. The M1 biological control sample presents the CACUA, CACUB, CACUD and CACUJ polymorphisms in genotype Ho.WT, CACUH and CACUG in genotype Ho.MT, CACUF and CACUC in genotype HET.

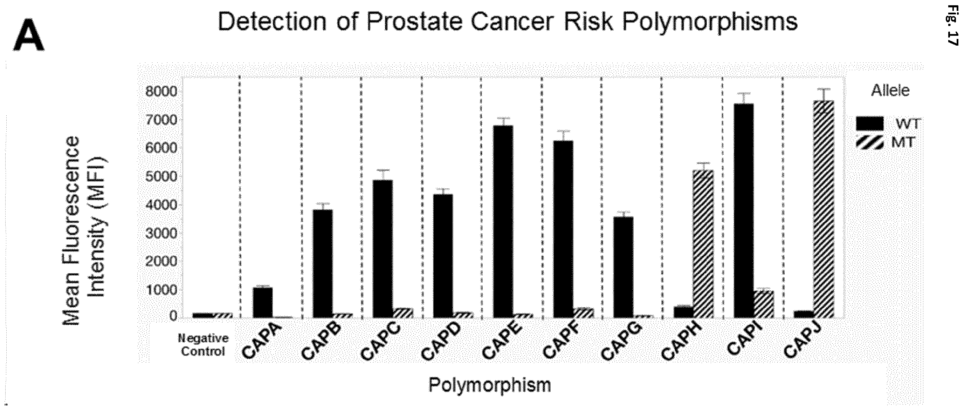

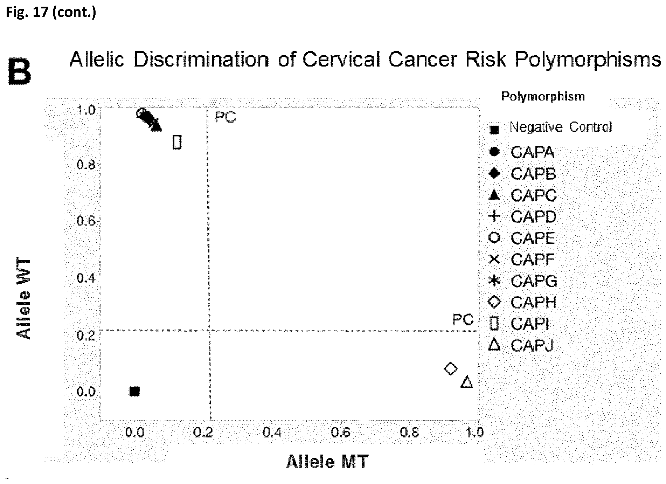

Figures 17 A-B show the identification and allelic discrimination of polymorphisms of risk to CAP in biological control sample by the Kimera-TestTM test. A: The MFI values recorded in each of the CAP risk polymorphisms identified in the M4 biological control sample are shown. The bars represent the Average ± 1 D.E. of 4 replicas. B: Allele discrimination graph originated from the obtained RA values. The M4 biological control sample presents CAPA, CAPB, CAPC, CACUD, CAPE, CAPF, CAPG and CAPI polymorphisms in genotype Ho.WT, CAPH and CAPJ in genotype HET.

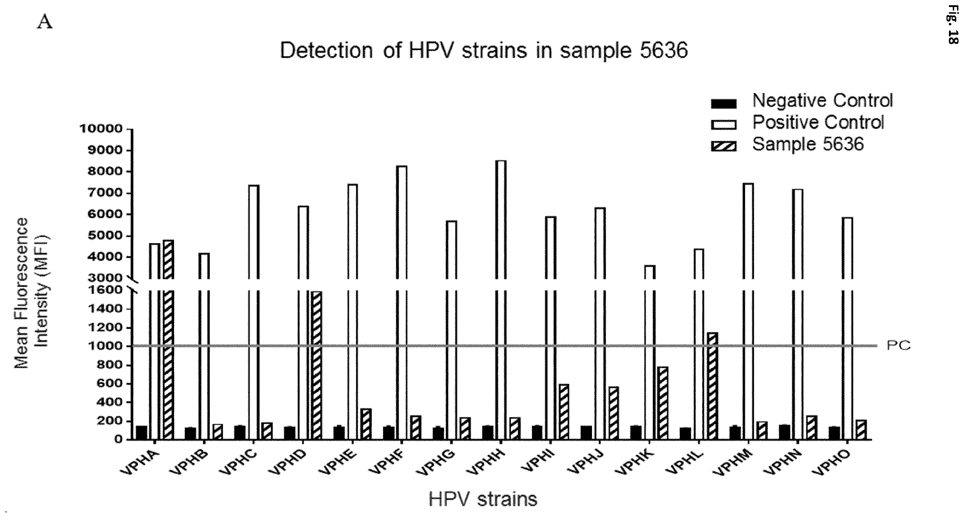

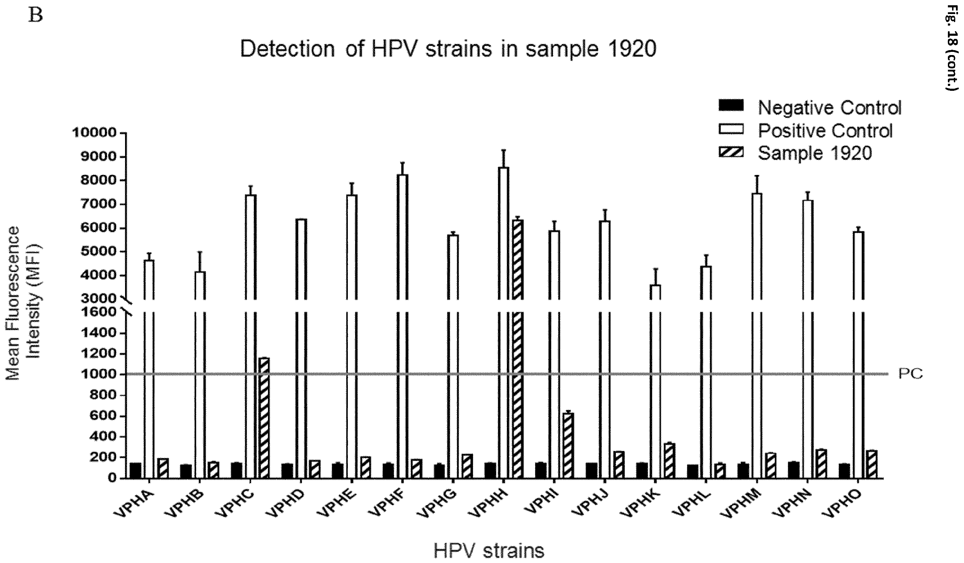

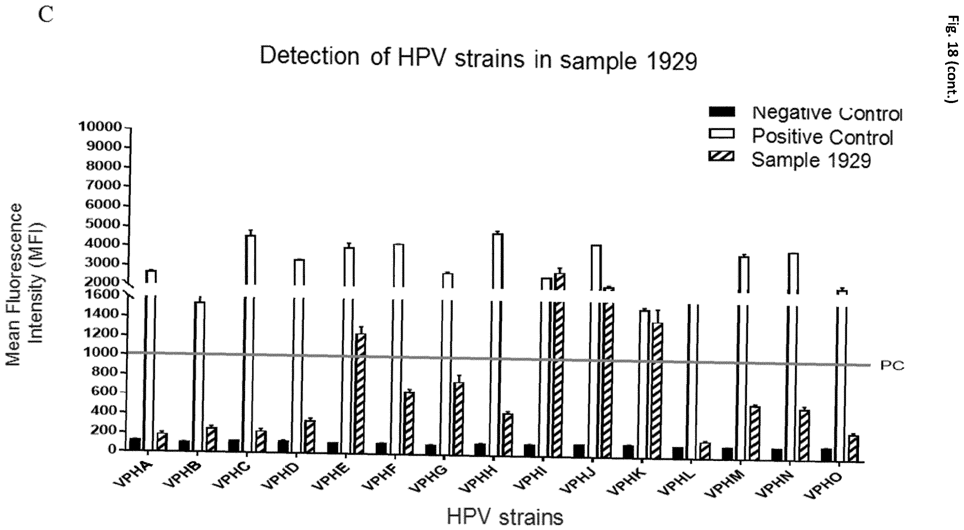

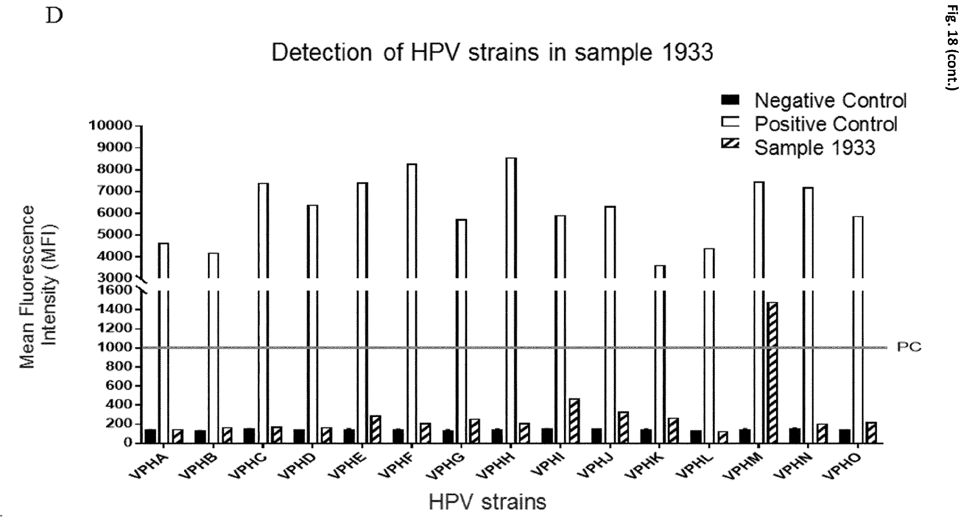

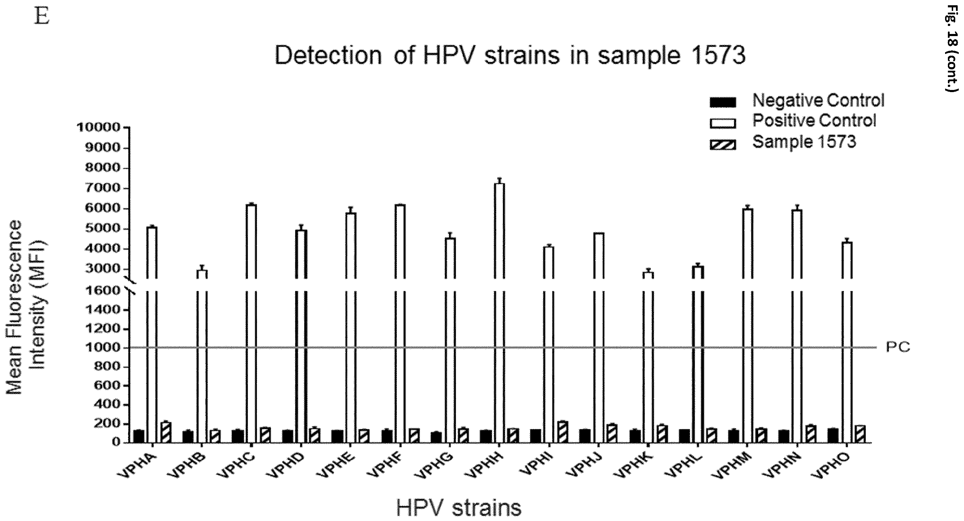

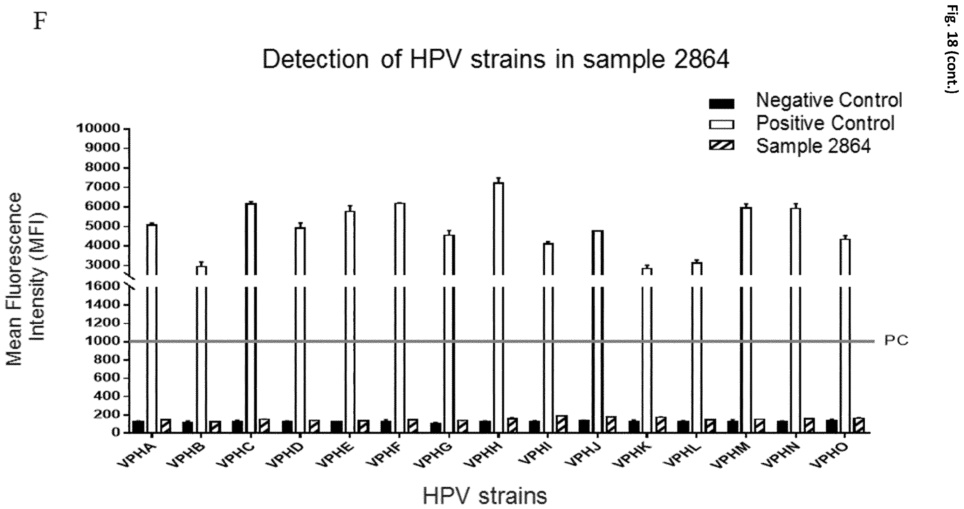

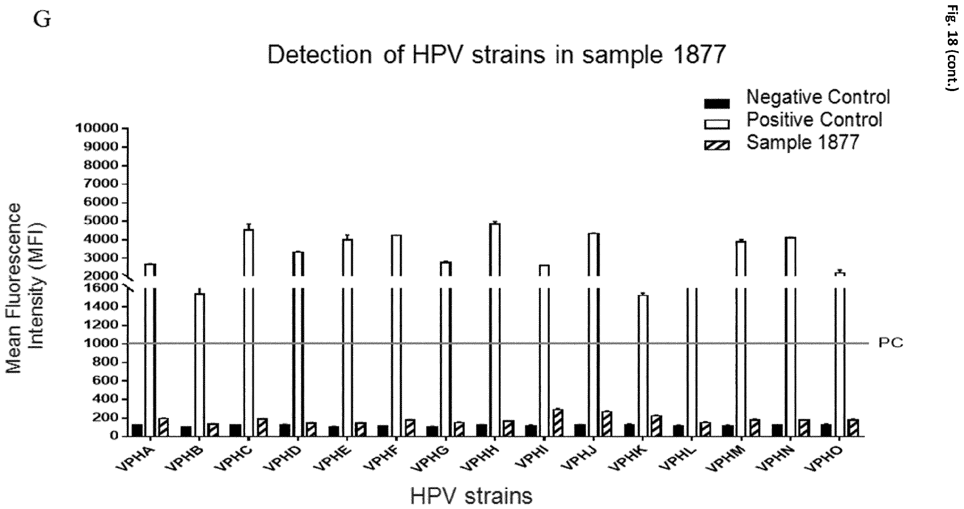

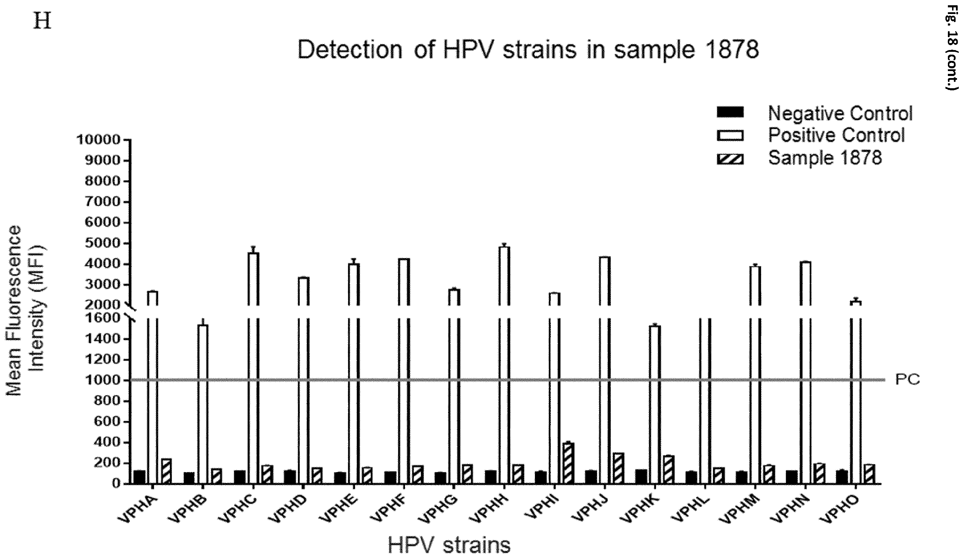

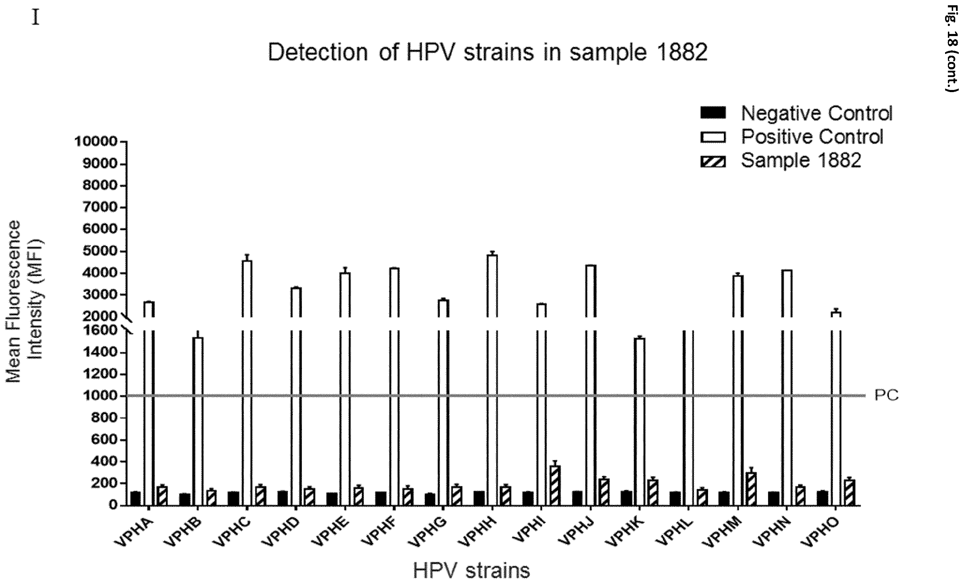

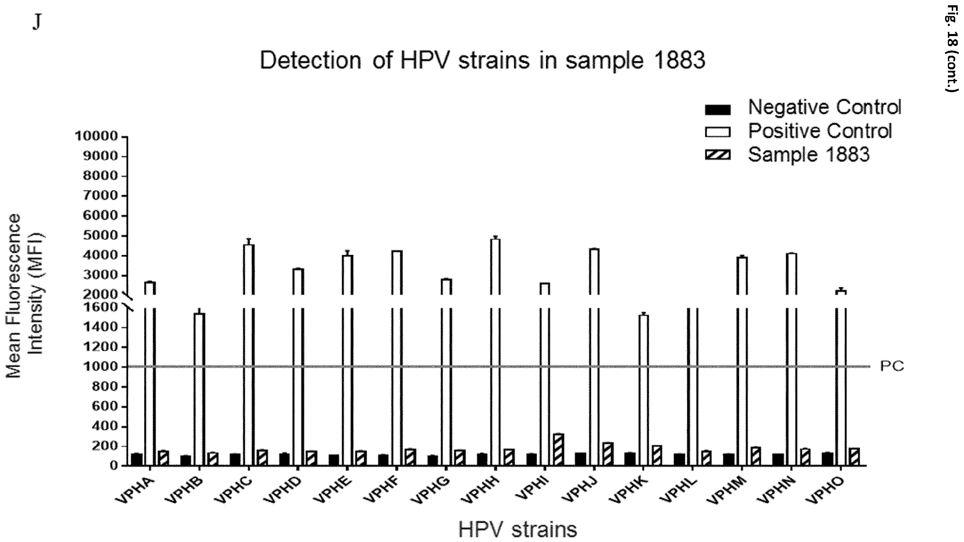

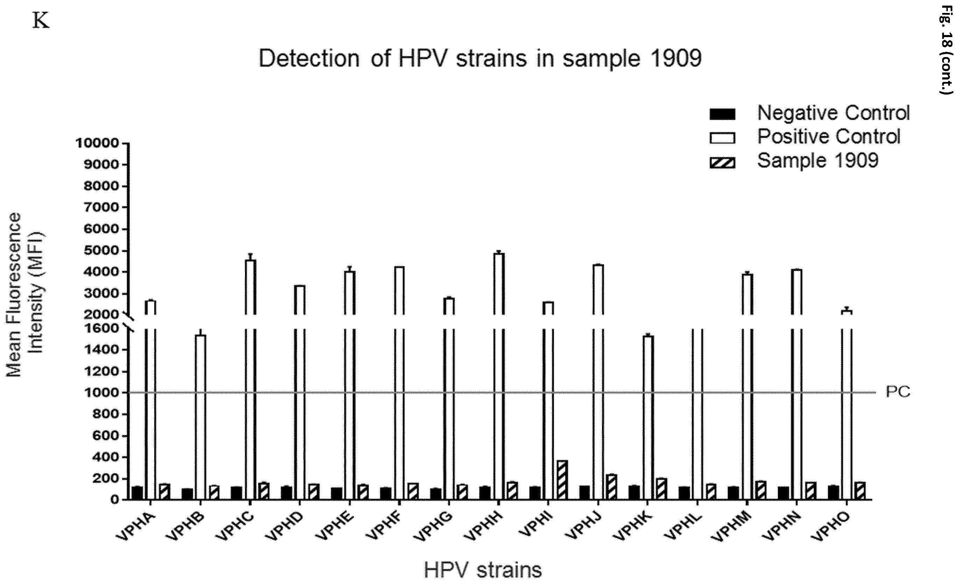

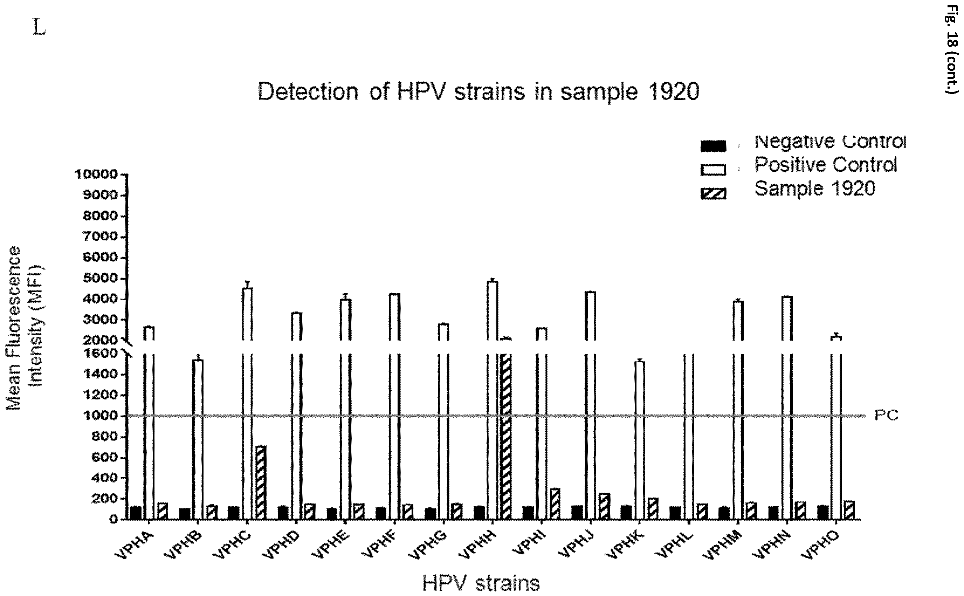

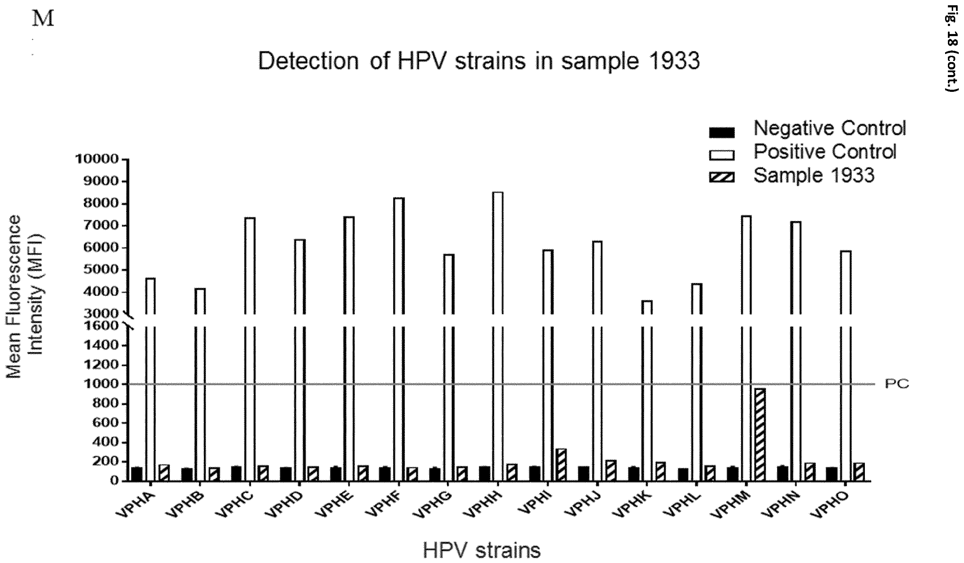

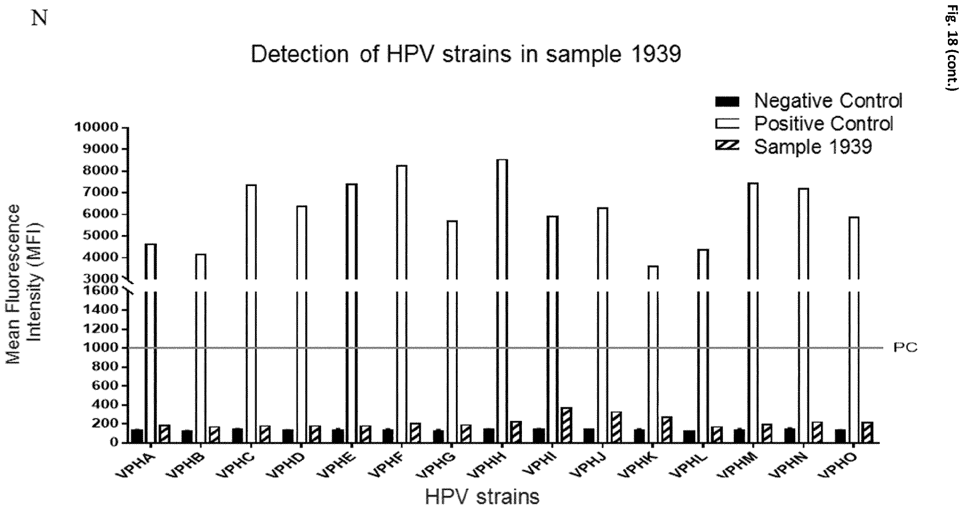

Figures 18A-N show the detection of HPV strains. A. sample 5636. B. sample 1920. C. 1929. D. 1933. E. sample 1573. F. sample 2864. G. sample 1877. H. sample 1878. I. sample 1882. J. sample 1883. K. sample 1909. L. sample 1920. M. sample 1933. N. sample 1939.

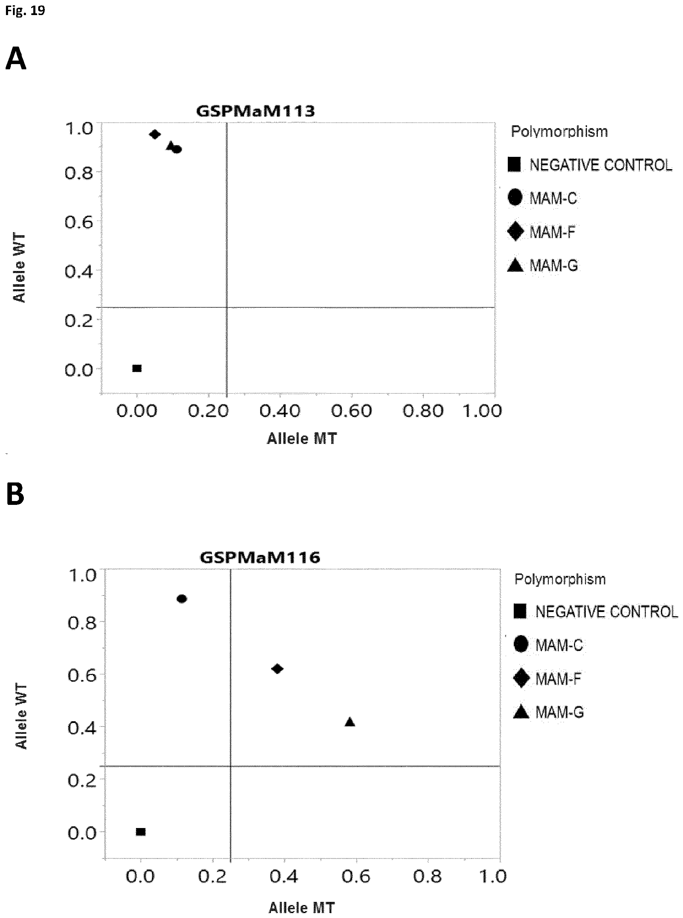

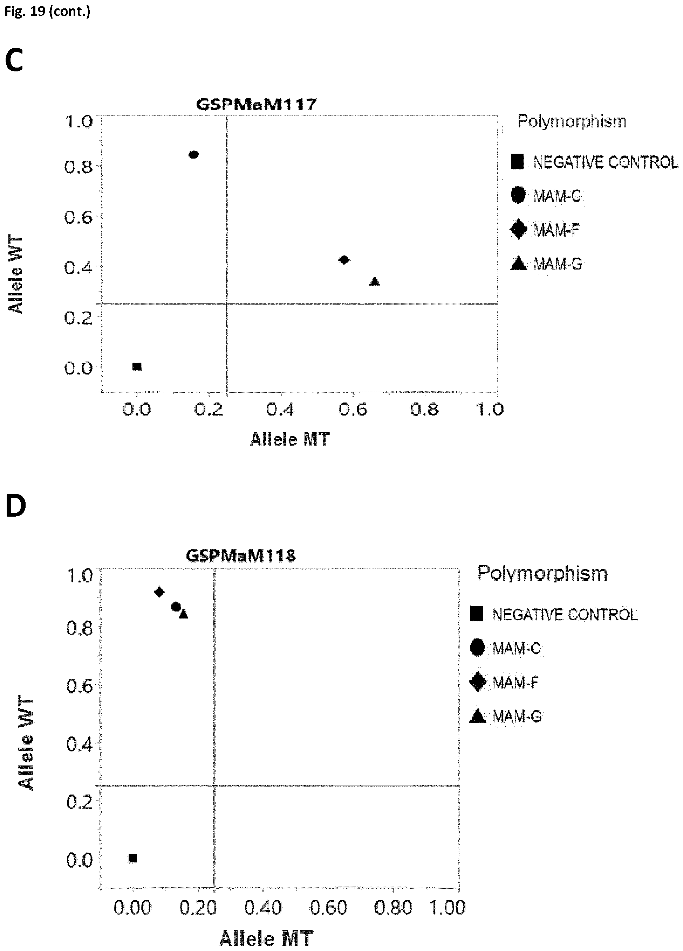

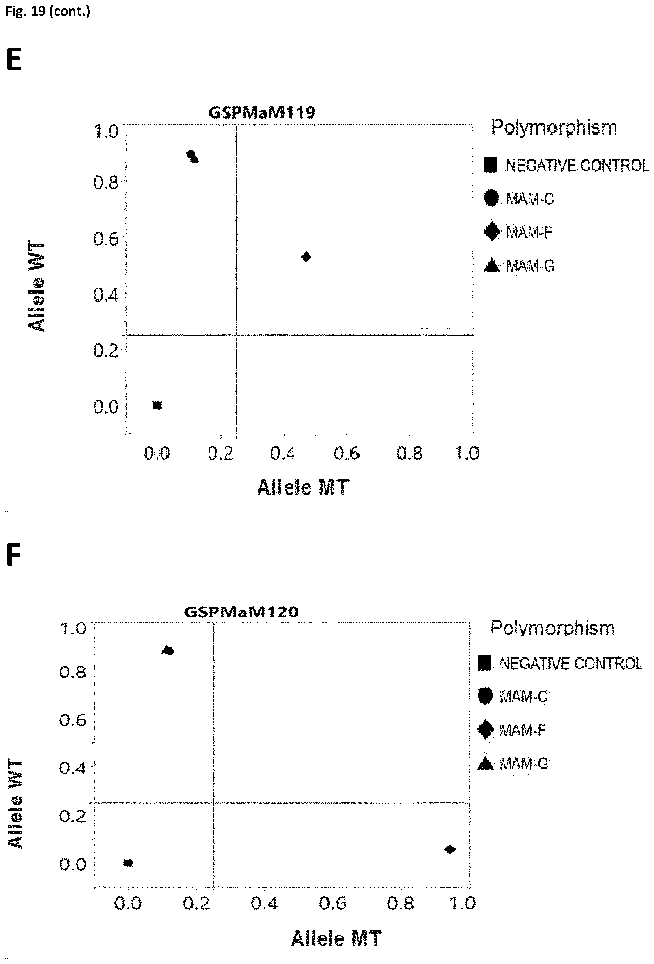

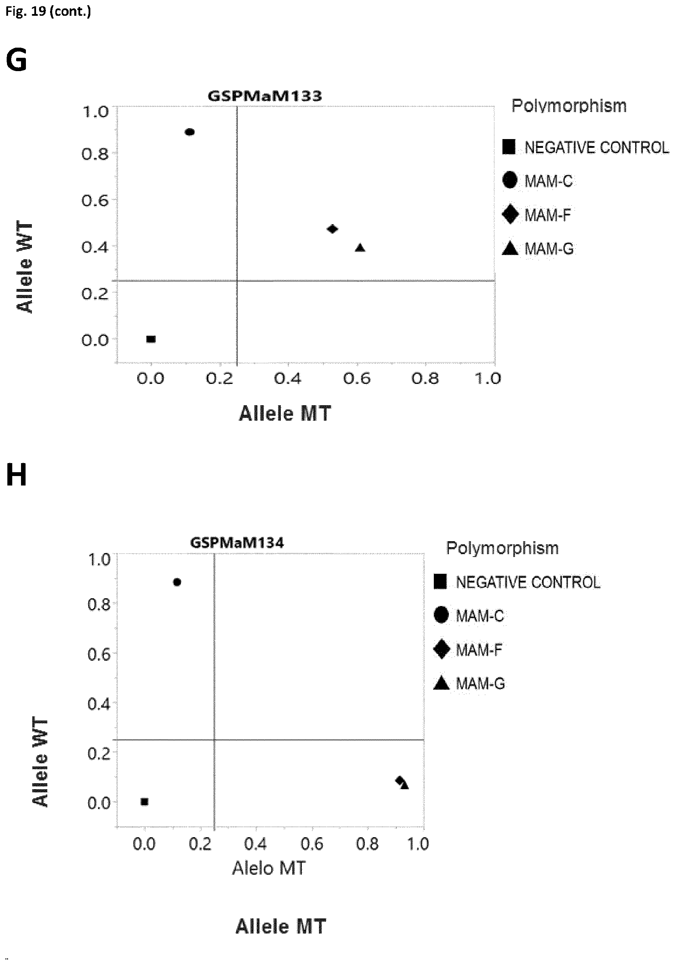

Figures 19A-I show the determination of susceptibility to breast cancer. A. sample 113. B sample 116. C. sample 117. D. sample 118. E. sample 119. F sample 120. G. sample 133. H. sample 134.

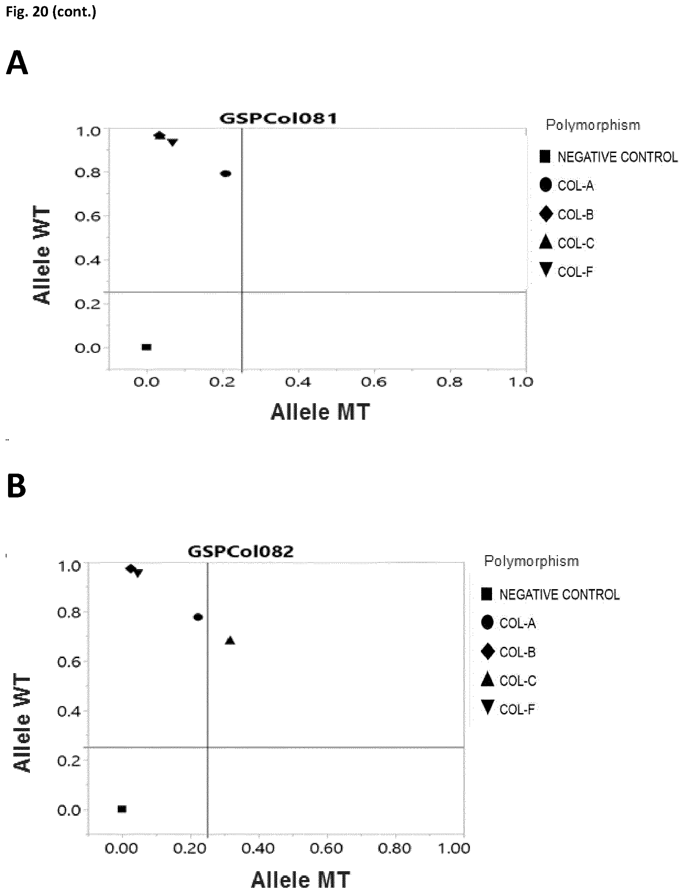

Figures 20A-V show the determination of susceptibility to colon cancer. A sample 81. B sample 82. C sample 84. D sample 85. E. sample 86. F. sample 87. G sample 88. H. sample 89. I sample 90. J. sample 92. K. sample 93. L. sample 96. M. sample 97. N. sample 100. O. sample 101. P. sample 102. Q. sample 103. R. sample 104. S. sample 105. T. sample 106. U. sample 107. V. sample 108.

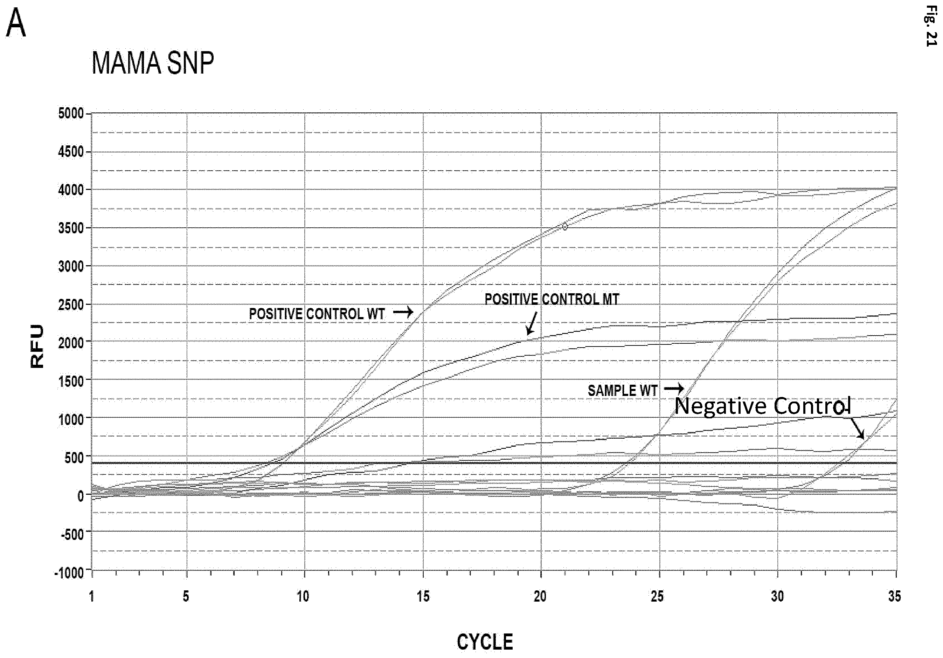

Figures 21A-E show the qPCR results from a breast cancer sample for breast cancer specific gene markers. A. MAMA SNP. B. MAMB SNP. C. MAMC SNP. D.MAMD SNP. E. MAMD SNP.

### DETAILED DESCRIPTION OF THE INVENTION

The present invention is based on the seminal discovery that the simultaneous detection of HPV infection and the genotyping of cancer susceptibility markers can be used to determine cancer susceptibility in a subject.

Before the present compositions and methods are described, it is to be understood that this invention is not limited to particular compositions, methods, and experimental conditions described, as such compositions, methods, and conditions may vary. It is also to be understood that the terminology used herein is for purposes of describing particular embodiments only, and is not intended to be limiting, since the scope of the present invention will be limited only in the appended claims.

As used in this specification and the appended claims, the singular forms "a", "an", and "the" include plural references unless the context clearly dictates otherwise. Thus, for example, references to "the method" includes one or more methods, and/or steps of the type described herein which will become apparent to those persons skilled in the art upon reading this disclosure and so forth. For example, the term "an HPV strain genotype" may include "a plurality of HPV strain genotypes". Similarly, the plural form may include the singular form.

All publications, patents, and patent applications mentioned in this specification are herein incorporated by reference to the same extent as if each individual publication, patent, or patent application was specifically and individually indicated to be incorporated by reference.

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, it will be understood that modifications and variations are encompassed within the spirit and scope of the instant disclosure. The preferred methods and materials are now described.

The term "about" when used before a numerical designation, e.g., pH, temperature, amount, concentration, and molecular weight, including range, indicates approximations which may vary by ± 5%, ±1% or ±0.1%.

As used in the specification and claims, the singular form "a", "an" and "the" include plural references unless the context clearly dictates otherwise.

The term "and/or" is intended to mean either or both of two components of the invention.

Various embodiments of the present invention are directed to methods and kits for simultaneously determining whether HPV strain genotypes and genetic markers for cancer susceptibility are present in a sample from a subject

In one embodiment, the present invention provides methods for assessing cancer susceptibility in a subject by simultaneously identifying one or more cancer susceptibility marker genes and a human papillomavirus (HPV) genotype strain, thereby assessing cancer susceptibility in the subject.

As used herein "determining a cancer susceptibility" and "assessing a cancer susceptibility" in a patient can be used interchangeably, and refer to the evaluation of the probability of said patient to develop a certain type of cancer. The evaluation of the risk of cancer development of the present invention includes the detection of a HPV strain DNA, and the detection of SNPs that are associated with cancer susceptibility.

The term "cancer" refers to a group diseases characterized by abnormal and uncontrolled cell proliferation starting at one site (primary site) with the potential to invade and to spread to others sites (secondary sites, metastases) which differentiate cancer (malignant tumor) from benign tumor. Virtually all the organs can be affected, leading to more than 100 types of cancer that can affect humans. Cancers can result from many causes including genetic predisposition, viral infection, exposure to ionizing radiation, exposure environmental pollutant, tobacco and or alcohol use, obesity, poor diet, lack of physical activity or any combination thereof. Particular cancers referenced herein are cervical, prostate, colon, pancreatic, lung or head and neck cancer.

Exemplary cancers described by the National Cancer Institute include: Acute Lymphoblastic Leukemia, Adult; Acute Lymphoblastic Leukemia, Childhood; Acute Myeloid Leukemia, Adult; Adrenocortical Carcinoma; Adrenocortical Carcinoma, Childhood; AIDS-Related Lymphoma; AIDS-Related Malignancies; Anal Cancer; Astrocytoma, Childhood Cerebellar; Astrocytoma, Childhood Cerebral; Bile Duct Cancer, Extrahepatic; Bladder Cancer; Bladder Cancer, Childhood; Bone Cancer, Osteosarcoma/Malignant Fibrous Histiocytoma; Brain Stem Glioma, Childhood; Brain Tumor, Adult; Brain Tumor, Brain Stem Glioma, Childhood; Brain Tumor, Cerebellar Astrocytoma, Childhood; Brain Tumor, Cerebral Astrocytoma/Malignant Glioma, Childhood; Brain Tumor, Ependymoma, Childhood; Brain Tumor, Medulloblastoma, Childhood; Brain Tumor, Supratentorial Primitive Neuroectodermal Tumors, Childhood; Brain Tumor, Visual Pathway and Hypothalamic Glioma, Childhood; Brain Tumor, Childhood (Other); Breast Cancer; Breast Cancer and Pregnancy; Breast Cancer, Childhood; Breast Cancer, Male; Bronchial Adenomas/Carcinoids, Childhood: Carcinoid Tumor, Childhood; Carcinoid Tumor, Gastrointestinal; Carcinoma, Adrenocortical; Carcinoma, Islet Cell; Carcinoma of Unknown Primary; Central Nervous System Lymphoma, Primary; Cerebellar Astrocytoma, Childhood; Cerebral Astrocytoma/Malignant Glioma, Childhood; Cervical Cancer; Childhood Cancers; Chronic Lymphocytic Leukemia; Chronic Myelogenous Leukemia; Chronic Myeloproliferative Disorders; Clear Cell Sarcoma of Tendon Sheaths; Colon Cancer; Colorectal Cancer, Childhood; Cutaneous T-Cell Lymphoma; Endometrial Cancer; Ependymoma, Childhood; Epithelial Cancer, Ovarian; Esophageal Cancer; Esophageal Cancer, Childhood; Ewing's Family of Tumors; Extracranial Germ Cell Tumor, Childhood; Extragonadal Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastric (Stomach) Cancer, Childhood; Gastrointestinal Carcinoid Tumor; Germ Cell Tumor, Extracranial, Childhood; Germ Cell Tumor, Extragonadal; Germ Cell Tumor, Ovarian; Gestational Trophoblastic Tumor; Glioma. Childhood Brain Stem; Glioma. Childhood Visual Pathway and Hypothalamic; Hairy Cell Leukemia; Head and Neck Cancer; Hepatocellular (Liver) Cancer, Adult (Primary); Hepatocellular (Liver) Cancer, Childhood (Primary); Hodgkin's Lymphoma, Adult; Hodgkin's Lymphoma, Childhood; Hodgkin's Lymphoma During Pregnancy; Hypopharyngeal Cancer; Hypothalamic and Visual Pathway Glioma, Childhood; Intraocular Melanoma; Islet Cell Carcinoma (Endocrine Pancreas); Kaposi's Sarcoma; Kidney Cancer; Laryngeal Cancer; Laryngeal Cancer, Childhood; Leukemia, Acute Lymphoblastic, Adult; Leukemia, Acute Lymphoblastic, Childhood; Leukemia, Acute Myeloid, Adult; Leukemia, Acute Myeloid, Childhood; Leukemia, Chronic Lymphocytic; Leukemia, Chronic Myelogenous; Leukemia, Hairy Cell; Lip and Oral Cavity Cancer; Liver Cancer, Adult (Primary); Liver Cancer, Childhood (Primary); Lung Cancer, Non-Small Cell; Lung Cancer, Small Cell; Lymphoblastic Leukemia, Adult Acute; Lymphoblastic Leukemia, Childhood Acute; Lymphocytic Leukemia, Chronic; Lymphoma, AIDS- Related; Lymphoma, Central Nervous System (Primary); Lymphoma, Cutaneous T-Cell; Lymphoma, Hodgkin's, Adult; Lymphoma, Hodgkin's; Childhood; Lymphoma, Hodgkin's During Pregnancy; Lymphoma, Non-Hodgkin's, Adult; Lymphoma, Non-Hodgkin's, Childhood; Lymphoma, Non-Hodgkin's During Pregnancy; Lymphoma, Primary Central Nervous System; Macroglobulinemia, Waldenstrom's; Male Breast Cancer; Malignant Mesothelioma, Adult; Malignant Mesothelioma, Childhood; Malignant Thymoma; Medulloblastoma, Childhood; Melanoma; Melanoma, Intraocular; Merkel Cell Carcinoma; Mesothelioma, Malignant; Metastatic Squamous Neck Cancer with Occult Primary; Multiple Endocrine Neoplasia Syndrome, Childhood; Multiple Myeloma/Plasma Cell Neoplasm; Mycosis Fungoides; Myelodysplasia Syndromes; Myelogenous Leukemia, Chronic; Myeloid Leukemia, Childhood Acute; Myeloma, Multiple; Myeloproliferative Disorders, Chronic; Nasal Cavity and Paranasal Sinus Cancer; Nasopharyngeal Cancer; Nasopharyngeal Cancer, Childhood; Neuroblastoma; Non-Hodgkin's Lymphoma, Adult; Non-Hodgkin's Lymphoma, Childhood; Non-Hodgkin's Lymphoma During Pregnancy; Non-Small Cell Lung Cancer; Oral Cancer, Childhood; Oral Cavity and Lip Cancer; Oropharyngeal Cancer; Osteosarcoma/Malignant Fibrous Histiocytoma of Bone; Ovarian Cancer, Childhood; Ovarian Epithelial Cancer; Ovarian Germ Cell Tumor; Ovarian Low Malignant Potential Tumor; Pancreatic Cancer; Pancreatic Cancer, Childhood', Pancreatic Cancer, Islet Cell; Paranasal Sinus and Nasal Cavity Cancer; Parathyroid Cancer; Penile Cancer; Pheochromocytoma; Pineal and Supratentorial Primitive Neuroectodermal Tumors, Childhood; Pituitary Tumor; Plasma Cell Neoplasm/Multiple Myeloma; Pleuropulmonary Blastoma; Pregnancy and Breast Cancer; Pregnancy and Hodgkin's Lymphoma; Pregnancy and Non-Hodgkin's Lymphoma; Primary Central Nervous System Lymphoma; Primary Liver Cancer, Adult; Primary Liver Cancer, Childhood; Prostate Cancer; Rectal Cancer; Renal Cell (Kidney) Cancer; Renal Cell Cancer, Childhood; Renal Pelvis and Ureter, Transitional Cell Cancer; Retinoblastoma; Rhabdomyosarcoma, Childhood; Salivary Gland Cancer; Salivary Gland'Cancer, Childhood; Sarcoma, Ewing's Family of Tumors; Sarcoma, Kaposi's; Sarcoma (OsteosarcomaVMalignant Fibrous Histiocytoma of Bone; Sarcoma, Rhabdomyosarcoma, Childhood; Sarcoma, Soft Tissue, Adult; Sarcoma, Soft Tissue, Childhood; Sezary Syndrome; Skin Cancer; Skin Cancer, Childhood; Skin Cancer (Melanoma); Skin Carcinoma, Merkel Cell; Small Cell Lung Cancer; Small Intestine Cancer; Soft Tissue Sarcoma, Adult; Soft Tissue Sarcoma, Childhood; Squamous Neck Cancer with Occult Primary, Metastatic; Stomach (Gastric) Cancer; Stomach (Gastric) Cancer, Childhood; Supratentorial Primitive Neuroectodermal Tumors, Childhood; T-Cell Lymphoma, Cutaneous; Testicular Cancer; Thymoma, Childhood; Thymoma, Malignant; Thyroid Cancer; Thyroid Cancer, Childhood; Transitional Cell Cancer of the Renal Pelvis and Ureter; Trophoblastic Tumor, Gestational; Unknown Primary Site, Cancer of, Childhood; Unusual Cancers of Childhood; Ureter and Renal Pelvis, Transitional Cell Cancer; Urethral Cancer; Uterine Sarcoma; Vaginal Cancer; Visual Pathway and Hypothalamic Glioma, Childhood; Vulvar Cancer; Waldenstrom's Macro globulinemia; and Wilms' Tumor.

The terms "subject," "individual" and "patient" are used interchangeably herein and refer to a mammal and, particularly, to a human.

As used herein, "cancer susceptibility markers" and "cancer susceptibility genetic markers" are used interchangeably and refer to any genetic markers that increase the risk of the carrier of such marker to develop the cancer associated with said marker. Cancer susceptibility genetic markers generally refer to inherited mutations or somatic mutations in tumor suppressor genes or in oncogenes, and that are responsible for higher risk of cancer development. However, cancer susceptibility markers in a subject can also be identified as single nucleotide polymorphisms (SNPs). A SNP is a DNA sequence variation occurring when a single nucleotide - A, T, C, or G - in the genome differs between members of a species. As used herein, the term SNP is intended to refer to those DNA sequence variations associated with cancer, in particular, with cervical cancer or prostate cancer.

SNPs have been reported to be associated with cervical and prostate cancers in patients. Thus, a number of SNPs in identified genes, have been reported to be associated with cervical cancer and similarly, a number of SNPs have been reported to be associated with prostate cancer. Such SNPs may be targeted in the methods and kits herein to detect the presence of genetic markers indicating susceptibility to cervical cancer or prostate cancer.

In addition, SNPs are associated with cancer propensity in different organs, including other organs such as breast, lung, pancreatic, gastric, and colon cancers. Moreover, SNPs have been associated with cancers, including leukemia, and specifically chronic myeloid lymphoblastic leukemia, acute myeloid lymphoblastic leukemia, and chronic lymphocytic leukemia. Those of skill in the art will recognize that the methods and kits of the present invention may be used to detect the presence of genetic markers indicating susceptibility to those cancers as well. In another aspect, simultaneous detection of HPV infection (genotyping) and Prostate cancer genetic propensity, and simultaneous detection of HPV infection (genotyping) and Cervical cancer genetic propensity, can be accomplished using the methods and kits of the present invention. DNA tests can be used to identify HPV strain genotypes as well as cancer susceptibility markers in a subject.

In certain aspects, the cancer susceptibility maker gene is CACUA. CACUB, CACUC, CACUD, CACUE, CACUF, CACUG, CACUH, CACUI, CACUJ, CAPA, CAPB, CAPC, CAPD, CAPE, CAPF, CAPG, CAPH, CAPI, CAPJ, COLA, COLB, COLC, COLD, COLE, COLF, COLG, GASA, GASB, GASC, GASD, GASE, GASG, LEUA, LEUB, LEUC, LEUD, LEUE, LEUF, MAMA, MAMB, MAMC, MAMD, MAME, MAMF, MAMG, PROA, PROB, PROC, PROD, PROE, PROF, PANA, PANB, PANC, PAND, PULA, PULB, PULC, PULD, PULE, PULF, PULG, GENA, GENB, GEND, GENE, GENG, GENH and/or GENI; is a SNP located in the gene encoding for IL10, TGF-β1, CYP1A1, ST3GAL4, IL4, AGTR1, ERBB2, HOXB13, Chr8, MSMB, RNASEL, CYP19, ABO, ABL1, ARID5B, Chr13, CCKBR, JAK2, NQO1, EGFR, EPHX1, CHRNA3, IRF4, PIK3CA, CXCL8, BRCA2, BRCA1, ATM, TCF2, TGFB1, KiSS1, APC, PGR, MTHFR, MUTYH, CDH1, IL1B, IL6, TLR4, TNF-α, MDM2, MLH1, BRAF, TP53, BCAR1, STK11, KRAS, FGFR2, SRD5A2, EIF3H and/or MSH6; and/or SEQ ID NOs:1-67 *see Table 22). In some aspects the cancer susceptibility gene is specific for cervical cancer (CACUA. CACUB, CACUC, CACUD, CACUE, CACUF, CACUG, CACUH, CACUI, and/or CACUJ); specific for prostate cancer (CAPA, CAPB, CAPC, CAPD, CAPE, CAPF, CAPG, CAPH, CAPI, and/or CAPJ); specific for colon cancer (COLA, COLB, COLC, COLD, COLE, COLF, and/or COLG); specific for gastric cancer (GASA, GASB, GASC, GASD, GASE, and/ or GASG); specific for leukemia (LEUA, LEUB, LEUC, LEUD, LEUE, and/or LEUF); specific for breast cancer (MAMA, MAMB, MAMC, MAMD, MAME, MAMF, and /or MAMG), PROA, PROB, PROC, PROD, PROE, PROF; specific for pancreatic cancer (PANA, PANB, PANC and/or PAND), specific for lung cancer (PULA, PULB, PULC, PULD, PULE, PULF, and/or PULG); and/or GENA, GENB, GEND, GENE, GENG, GENH and/or GENI. In other aspects the one or more cancer susceptibility gene is CACUA, CACUB, CACUC, CACUD, CACUF, CACUG, CACUH and/or CACUJ; CAPA, CAPB, CAPC, CAPD, CAPE, CAPF, CAPG, CAPI, and/or CAPJ; MAMC, MAMF, MAMG; and/or COLA, COLB, COLC and/or COLF.

### Table 22

**Table 22: Cancer susceptibility SNP sequences**

| SEQ ID NO | SNP name | SNP location | SNP Sequence |
|---|---|---|---|
| SEQ ID NO:1 | CACUA | rs1800872 located at the gene IL-10 | AGCAAGTGCAGACTACTCTTACCCACTTCCCCAAGCACAGTTGGGGTG GGGGACAGCTGAAGAGGTGGAAACATGTGCCTGAGAATCCTAATGAAA TCGGGGTAAAGGAGCCTGGAACACATCCTGTGACCCCGCCTGTMCTGTA GGAAGCCAGTCTCTGGAAAGTAAAATGGAAGGGCTGCTTGGGAACTTT GAGGATATTTAGCCCACCCCCTCATTTTTACTTGGGGAAACTAAGGCCC AGAGACC |
| SEQ ID NO:2 | CACUB | rs1800468 located at the gene TGFB1 | CATCTACAGTGGGGCCGACCGCTATCGCCTGCACACAGCTGCTGGTGGCA CCGTGCACCTGGAGATCGGCCTGCTGCTCCGCAACTTCGACCGCTACGGC GTGGAGTGCTGAGGGACTCTGCCTCCAACRTCACCACCATCCACACCCCG GACACCCAGTGATGGGGGAGGATGGCACAGTGGTCAAGAGCACAGACTC TAGAGACTGTCAGAGCTGACCCCAGCTAAGGCATGGCACCGCTTCTGTCC T |
| SEQ ID NO:3 | CACUC | rs1800469 located at the gene TGFB1 | GGTCCCTCTGGGCCCAGTTTCCCTATCTGTAAATTGGGGACAGTAAATGT ATGGGGTCGCAGGGTGTTGAGTGACAGGAGGCTGCTTAGCCACATGGGA GGTGCTCAGTAAAGGAGAGCAATTCTTACAGGTGTCTGCCTCCTGACCCT TCCATCCYTCAGGTGTCCTGTTGCCCCCTCCTCCCACTGACACCCTCCGGA GGCCCCCATGTTGACAGACCCTCTTCTCCTACCTTGTTTCCCAGCCTGAC |
| SEQ ID NO:4 | CACUF | rs10893506 located at the gene ST3GAL4 | CTTCCTGCTTTCTGGTGGAAGGAGGGGCAGACAGTGGGTGTGTCCTGCT CCAGTGTCTAGGCAGGAGAGTTTGTGAAGCYGACCGGACACCTGTGGCT CTTATTTCCTAGGTGGCCCGAGGCAGCCGGGATGACAGCTCTCCCCAGGA ATCCTGCTGCCTGCTGAGAAACATGGTCAGCAAGTCCCGTGAGTGTCATC CGAGGGCTCCCCCACCCTGGAGGACAGGCCTCAGAAGCCGTCTTCAGCA GG |
| SEQ ID NO:5 | CACUG | rs2424913 located at the gene DNMT3B | CTGGGGAATTCTGGCTGGACTCAGTCCAGAGTCCACCTCATCACCTGTT CACTTCCAGTTGTCCTGAAGCTGGCTACCAGGTCTCCTTGGCCACCTGAA GGCCTAATCCTTCTGGCCCCGCCAGACCCYAGGCCTCCAGTCACCTAAGG CCCAGTGAGTGTCCTCTCTTGCTTCTAGGTCCGAACTCGAAATAACAACA GTGTCTCCAGCCGGGAGAGGCACAGGCCTTCCCCACGTTCCACCCGAGG C |
| SEQ ID NO:6 | CACUH | rs2243250 located at the gene IL-4 | TTCCTGAAACCTCAGAATAGACCTACCTTGCCAAGGGCTTCCTTATGGGT AAGGACCTTATGGACCTGCTGGGACCCAAACTAGGCCTCACCTGATACG |
| | | | ACCTGTCCTTCTCAAAACACCTAAACTTGGGAGAACATTGTYCCCCAGTG CTGGGGTAGGAGAGTCTGCCTGTTATTCTGCCTCTATGCAGAGAAGGAGC CCCAGATCAGCTTTTCCATGACAGGACAGTTTCCAAGATGCCACCTGTAC T |
| SEQ ID NO:7 | CACUJ | rs1800871 located at the gene IL-10 | CCACAGAAGCTTACAACTAAAAGAAACTCTAAGGCCAATTTAATCCAAG GTTTCATTCTATGTGCTGGAGATGGTGTACAGTAGGGTGAGGAAACCAA ATTCTCAGTTGGCACTGGTGTACCCTTGTACAGGTGATGTAAYATCTCTG TGCCTCAGTTTGCTCACTATAAAATAGAGACGGTAGGGGTCATGGTGAGC ACTACCTGACTAGCATATAAGAAGCTTTCAGCAAGTGCAGACTACTCTTA CC |
| SEQ ID NO:8 | CAPA | rs5186 located at the gene AGT1R | GCTCATCCACCAAGAAGCCTGCACCATGTTTTGAGGTTGAGTGACATGTT CGAAACCTGTCCATAAAGTAATTTTGTGAAAGAAGGAGCAAGAGAACAT TCCTCTGCAGCACTTCACTACCAAATGAGCMTTAGCTACTTTTCAGAATT GAAGGAGAAAATGCATTATGTGGACTGAACCGACTTTTCTAAAGCTCTG AACAAAAGCTTTTCTTTCCTTTTGCAACAAGACAAAGCAAAGCCACATTT TG |
| SEQ ID NO:9 | CAPB | rs13821319 7 located at the gene HOXB 13 | AAACTCAGTGGGCGGCTGGGGTACTCTTCCCCGGCCGTGGAGTCTCCG CGGGGTACGCGGCCAGGGTGGCTGCCTGGGCACAGGGTTTCAGCGAGCT CCGGGACACTCGGCAGGAGTAGTACCCGCCTYCAAAGTAACCATAAGGC ACGGGAGCTGGGGACGTCCCCTGGGGCACCCCAGGGCATGGGTGGCATT GCTTTGGCGGCTCCGCCGAGCCTGGCAGATCCAAGGGGGCATAGTTGAC AGCA |
| SEQ ID NO:10 | CAPC | rs9282858 located at the gene SRD5A2 | CYAGCAGAGCCCAGTGCTGGCAGGCAGCGCCACTTTGGTCGCCCTTGGG GCACTGGCCTTGTACGTCGCGAAGCC TCCGGCTACGGGAAGCACACGGAGAGCCTGAAGCCGGCGGCTACCCGCC TGCCARCCCRCGCCGCCTGGTTCCTGCAGGAGCTGCCTTCCTTCGCGGTG CCCGCGGGGATCCTCGCCCGGCAGCCCCTCTCCCTCTTCGGGCCACCTGG GACGGTACTTCTGGGCCTCTTCTGCS |
| SEQ ID NO:11 | CAPD | rs16901979 located at the chromosom e 8 | AATTTAAAATGATACAAAGTTCAAAGTTTAACAAATACATTTGAAGCCAT TTGCAACAAATACATCTGAAGCTAATTGCTGGCTCTAGAAAGTGTGGGGT CTTTGTTGTGGAGCAGTGTTAATGATTTAGCATTACTTATMTCTGGCAAA TGGTATTTTTGAGATAACATGTTATGGAAGAAAGTGAACTGAACTTGGAA GTTTGAAGATCTCGATTGAAGTATCATTTCTGCCTCAACTACTTGCATTA |
| SEQ ID NO:12 | CAPE | rs4242382 located at the Chromoso me 8 | CCATATTTGGGCTATATTAAGTAATAAGCCCACATGCTTTCTGTTGAGAA AATACAAAAAGATGTTTCCCTCTGTCATAAAGAAAAAGAGGTAACCCAG GGAACATTTTGTCCCTCTAGTTATCTTCCCRCAGGCCCATCAAGAATCAG GCAGTAGGTGAAAAAGAAACACAGAGAACCTAGGAACACAATAGGAAG ACCACCATGGGCCCTTAGGGAGTCAGCGAAGGCTTATGATGCAAAAAGA AGGT |
| SEQ ID NO:13 | CAPF | rs10993994 located at the gene MSMB | GGGGACAAGGTAAACACATAACTTGGGTGGAGATGTGATTGATTAATAA CTCATAAATCATCTGAAACCATACTTTCCTTTTGATAGTCAACCCTCTGTA AACACTCAATGTGTTCTCACCTTGTTATCATTCCCAATGAYGTCGAATGC GTGGTTGCCCTCTCCAGTATAAAAGTTTGATGCAGCTTTGCCTGGATGTA CCTGTCTATAAGGAGTCCTGCTTATCACAATGGTAGGTAACTGGTTTTAT |
| SEQ ID NO:14 | CAPG | CAPG rs12757998 located at the gene RNASEL | TCTTTGCTTCCACTTTAATCTCTTGTCTTTTATTTTTGTCACACTTCTCC ATCCCCTTTTTCCTCCCTCCTCTACGCCTCCCTAAACTTTTTCTCCTCTTT CTATCCTACTTCACCTCTTCTCTTTTCCTCATATACTCATACTCCTCTGCAT TCCYGCCTTCATATTTCCCCCCTCCCTCCTTTCCCTCAACTTTCTCTCCCCA GTCCCATCTTCTTGTTCTTCCTATTGTTGTCACTTTTTTCTC |
| SEQ ID NO:15 | CAPH | rs10459592 located at the gene CYP19 | CAACATGCCATTGAATCTGTCTTTTCAGCCAGGTTTTGTAATCCCTTTACT CTCAGAACAGAGTTTCAGGGATGGTAGGTAGTCTGTAGCTGAAGAGGTG TAGGTTGAACAAACTTGTAKAGTGAGGAAAGAGTGAGTCATCATTAAAT GCATTTTCAGTGGCAGATGACATACTCCAAGGTTTGCCTTTACTTAGACA GGGCCTAGAGTTTCTGTGACTTGCAATGGCTCTCAATCAGACTTGTGCTG G |
| SEQ ID NO:16 | CAPI | rs1447295 located at the Chromoso me 8 | AGACACTATACTAGATGATGGGACAACTAAAGGTAATGAACAGTTCTG TCTCTATGTAAAAATAATAATGATGATGATGATGAGATGGGACTTCAATT GAGGAAGTGCCATTGGGGAGGTATGTAAAAMGTGCTATGGAAAAAAAG CAACAGGAACCCCTTGATAGAAAAAAAAATGCTGGTGGGGGTAGGGATT TCTGCCTGTGTTCTTCAGAATGGGGTATGGAAAATCTGGGAGGAAAAG AAATT |
| SEQ ID NO:17 | CAPJ | rs6983267 located at the Chromoso me 8 | TCTCAGCTCCCTATCCATAAAACAGAGGACGAATAAACTCTCCTCCTAC CACTAAGAGGTGTAGCCAGAGTTAATACCCTCATCGTCCTTTGAGCTCAG CAGATGAAAGKCACTGAGAAAAGTACAAAGAATTTTTATGTGCTATTGA CTTTATTTTATTTTATGTGGGGGAGGGAGCCGGCCCCAGCTGGAAAGCTG CTTTCTCTGAATCAAAGGGCAGGAACCCAGCAAGTTCTCAGGATTGGGG C |
| SEQ ID NO:18 | COLA | rs1801155 located at the gene APC | CTCCAATATGTTTTTCAAGATGTAGTTCATTATCATCTTTGTCATCAGCTG AAGATGAAATAGGATGTAATCAGACGACACAGGAAGCAGATTCTGCTAA TACCCTGCAAATAGCAGAAAWAAAAGAAAAGATTGGAACTAGGTCAGC TGAAGATCCTGTGAGCGAAGTTCCAGCAGTGTCACAGCACCCTAGAACC AAATCCAGCAGACTGCAGGGTTCTAGTTTATCTTCAGAATCAGCCAGGCA CAA |
| SEQ ID NO:19 | COLB | rs35502531 located at the gene MLH1 | GAGCCAGCACCGCTCTTTGACCTTGCCATGCTTGCCTTAGATAGTCCAGA GAGTGGCTGGACAGAGGAAGATGGTCCCAAAGAAGGACTTGCTGAATAC ATTGTTGAGTTTCTGAAGAAGNGGCTGAGATGCTTGCAGACTATTTCTCT TTGGAAATTGATGAGGTGTGACAGCCATTCTTATACTTCTGTTGTATTCTT CAAATAAAATTTCCAGCCGGGTGCGGTGGCTCATGGCTGTAATCCCAGCA |
| SEQ ID NO:20 | COLC | rs16892766 located at the gene EIF3H | ACATTGCAACTTACCACAATAAAGTAAATATCACAAGATAAATGACAAT GTTGGAATGGAAACTACTTAGGGACTCAGAACGGTCAGACGCAAACAGT TTCAAGACTATTMGCTGTTAAAGGTTATGCCTTATGTCACCCAAAAGGGT TTTCCCCTAGATTTATAGCACAAACTCATGGAAGATTTATTGCCGTCTTA ATTTTTTCCCCAATTTTAACTTTAMGAACAGTCAGCCTGGAATKCCTCCT AA |
| SEQ ID NO:21 | COLD | rs1042821 located at the gene MSH6 | CCGATCTCCGCAGCCCTCCGTTGAGGTTCTTCGCCTTGGGCGGTGACGCG GAGCGCGCCAAGGGCCTGGGCCCAGGCCCAGCCTCGCTCCAGGCCGCAT CCCCGCCTGGGGAAGGAGAGGCCYCGGGGGCAGCGGCGGCACGGCCGC CTTCGCGTGAGGCCCTGGCCGAGGCCTTGTTGGCATCACTCAGCGCCGGA GACTTGGGGAAGAAGCTGTACAGGGTGCTCTGTCGCGACATACCGACAG CCGG |
| SEQ ID NO:22 | COLE | rs1801133 located at the gene MTHFR | AGGTGGAGGCCAGCCTCTCCTGACTGTCATCCCTATTGGCAGGTTACCCC AAAGGCCACCCCGAAGCAGGGAGCTTTGAGGCTGACCTGAAGCACTTGA AGGAGAAGGTGTCTGCGGGAGYCGATTTCATCATCACGCAGCTTTTCTTT GAGGCTGACACATTCTTCCGCTTTGTGAAGGCATGCACCGACATGGGCAT CACTTGCCCCATCGTCCCCGGGATCTTTCCCATCCAGGTGAGGGGCCCAG G |
| SEQ ID NO:23 | COLF | rs36053993 located at the gene MUTYH | GAACAGCCTGGGGCCCTTGGGGCCCAAATTCTGCTGGTGCAGAGGCCCA ACTCAGGTACCTGGATACTGGGCGTGGAGGGCAGTGGCATGAGTAACAA GAGAGAATGGAGGGAATCGGCAGCTGAGGCCTGACCCCTGCCTGGCTGC CCTCCCTCTCAGRTCTGCTGGCAGGACTGTGGGAGTTCCCGTCCGTGACC TGGGAGCCCTCAGAGCAGCTTCAGCGCAAGGCCCTGCTGCAGGAACTAC AGCG |
| SEQ ID NO:24 | COLG | rs34612342 located at the gene MUTYH | CTGGAGGTGAGAGCCACCCTAGGGTAGGGAAATAGGAACAATAGAGG GACTGACGGGTGATCTCTTTGACCTCTGATCCTACCCACAGGAGGTGAAT CAACTCTGGGCTGGCCTGGGCTRCTATTCTCGTGGCCGGCGGCTGCAGGA GGGAGCTCGGAAGGTAAGGGGATGGCAGGAGGGTAGGAACCCAGGAGT CTTGGGTGTCTTATAATCTTGAGTCTTGCACTCCAATCAGGTGGTAGAGG AGCT |
| SEQ ID NO:25 | GASA | rs16260 located at the gene CDH1 | AACAAAAGAACTCAGCCAAGTGTAAAAGCCCTTTCTGATCCCAGGTCTTA GTGAGCCACCGGCGGGGCTGGGATTCGAACCCAGTGGAATCAGAACCGT GCAGGTCCCATAACCCACCTAGACCCTAGCAACTCCAGGCTAGAGGGTC AMCGCGTCTATGCGAGGCCGGGTGGGCGGGCCGTCAGCTCCGCCCTGGG GAGGGGTCCGCGCTGCTGATTGGCTGTGGCCGGCAGGTGAACCCTCAGC CAAT |
| SEQ ID NO:26 | GASB | rs1143627 located at the gene IL-1B | ATTAGTCCCCTCCCCTAAGAAGCTTCCACCAATACTCTTTTCCCCTTTCCT TTAACTTGATTGTGAAATCAGGTATTCAACAGAGAAATTTCTCAGCCTCC TACTTCTGCTTTTGAAAGCYATAAAAACAGCGAGGGAGAAACTGGCAGA TACCAAACCTCTTCGAGGCACAAGGCACAACAGGCTGCTCTGGGATTCTC TTCAGCCAATCTTCATTGCTCAAGTATGACTTTAATCTTCCTTACAACTA |
| SEQ ID NO:27 | GASC | rs4073 located at the gene CXCL8 | ATCATGATAGCATCTGTAATTAACTGAAAAAAATAATTATGCCATTAAA AGAAAATCATCCATGATCTTGTTCTAACACCTGCCACTCTAGTACTATAT CTGTCACATGGTACTATGATAAAGTTATCTAGAAATAAAAAAGCATACA WTTGATAATTCACCAAATTGTGGAGCTTCAGTATTTTAAATGTATATTAA AATTAAATTATTTTAAAGATCAAAGAAAACTTTCGTCATACTCCGTATTT G |
| SEQ ID NO:28 | GASD | rs4986790 located at the gene TLR4 | TTCTGGGAGAATTTAGAAATGAAGGAAACTTGGAAAAGTTTGACAAATC TGCTCTAGAGGGCCTGTGCAATTTGACCATTGAAGAATTCCGATTAGCAT ACTTAGACTACTACCTCGATGRTATTATTGACTTATTTAATTGTTTGACAA ATGTTTCTTCATTTTCCCTGGTGAGTGTGACTATTGAAAGGGTAAAAGAC TTTTCTTATAATTTCGGATGGCAACATTTAGAATTAGTTAACTGTAAATT |
| SEQ ID NO:29 | GASE | rs4986791 located at the gene TLR4 | CTTCCAACAAAGGTGGAATGCTTTTCAGAAGTTGATCTACCAAGCCTT GAGTTTCTAGATCTCAGTAGAAATGGCTTGAGTTTCAAAGGTTGCTGTTC TCAAAGTGATTTTGGGACAAYCAGCCTAAAGTATTTAGATCTGAGCTTCA ATGGTGTTATTACCATGAGTTCAAACTTCTTGGGCTTAGAACAACTAGAA CATCTGGATTTCCAGCATTCCAATTTGAAACAAATGAGTGAGTTTTCAGT |
| SEQ ID NO:30 | GASG | rs1800629 located at the gene TNFA | CTGAAGCCCCTCCCAGTTCTAGTTCTATCTTTTCCTGCATCCTGTCTGGA AGTTAGAAGGAAACAGACCACAGACCTGGTCCCCAAAAGAAATGGAGG CAATAGGTTTTGAGGGGCATGRGGACGGGGTTCAGCCTCCAGGGTCCTA CACACAAATCAGTCAGTGGCCCAGAAGACCCCCCTCGGAATCGGAGCAG GGAGGATGGGGAGTGTGAGGGGTATCCTTGATGCTTGTGTGTCCCCAACT TTC |
| SEQ ID NO:31 | LEUA | rs34549764 located at the gene ABL1 | CAGCTCTACGTCTCCTCCGAGAGCCGCTTCAACACCCTGGCCGAGTTGGT TCATCATCATTCAACGGTGGCCGACGGGCTCATCACCACGCTCCATTATC CAGCCCCAAAGCGCAACAAGCCCACTGTCTATGGTGTGTCCCCCAACTAC GACAAGTGGGAGATGGAACGCACGGACATCACCATGAAGCACARGCTGG |
| | | | GCGGGGGCCAGTACGGGGAGGTGTACGAGGGCGTGTGGAAGAAATACA GCC |
| SEQ ID NO:32 | LEUB | rs7089424 located at the gene ARID5B | TTGGAGGTGGAGTTGGCTTTCTTCCCACTGCGTTCATTGTTACTCAGAGT GGTAGCAGTGTTTGGTTATAGTTTAGTTKTGTGATGTTTTTTTCTTGAGT GATGAAGCAATAGTGAGGGCAAAAGCTGATAGTAACATTTTGATACATA TTTTTGTATTTGTCTATGCCTCTATTTTAAGTGGTTTGCTTTTGAAAACAG CTGGTTCAAACCCTTGGTTTTGGGTTTGAAATATTCTTTGTTTTGTTT |
| SEQ ID NO:33 | LEUC | LEUC rs4982731 located at the gene ARID5B | AGAGACAGGTACCACGTACTGAGGCAGCAAAGGACTCAGTGAGTGTCA GGAGAAGCCTCTTACCCCTCTGGCCCTCAACTGCTTTATCTGTAAAATGT GGCTCACCCTAYCTTGCTGGTCTGAGGGCAGAGCCCACCAGATGATCTCT TGAGGTCTCTCCCAGCACATCTTGAGATCTGCGATTTTACAGAGCCAATT TTGGTTCTACCTCTAAACAATCGTGTGACTCCAGGTAAGTCCCTTCCCCTC |
| SEQ ID NO:34 | LEUD | rs872071 located at the gene IRF4 | TTGAGATCCTGATAGCCTGTTACAGGAATGAAGTAAAGGTCAGTTTTTTT TTGTATTGATTTTCACAGCTTTGAGGAACATGCATAAGAAATGTAGCTGA AGTAGAGGGGRCGTGAGAGAAGGGCCAGGCCGGCAGGCCAACCCTCCTC CAATGGAAATTCCCGTGTTGCTTCAAACTGAGACAGATGGGACTTAACA GGCAATGGGGTCCACTTCCCCCTCTTCAGCATCCCCCGTACCCCACTTTCT G |
| SEQ ID NO:35 | LEUE | rs77375493 located at the gene JAK2 | ATTCTTTGTACTTTTTTTTTCCTTAGTCTTCTTTGAAGCAGCAAGTATGA TGAGCAAGCTTTCTCACAAGCATTTGGTTTTAAATTATGGAGTATGTDTC TGTGGAGACGAGAGTAAGTAAAACTACAGGCTTTCTAATGCCTTTCTCAG AGCATCTGTTTTTGTTTATATAGAAAATTCAGTTTCAGGATCACAGCTAG GTGTCAGTGTAAACTATAATTTAACAGGAGTTAAGTATTTTTGAAACT |
| SEQ ID NO:36 | LEUF | rs1800566 located at the gene NQO1 | AACTTCTGTTGTTTATAGTACAACTGCATGGAATTGGTTGACTTACCTCTC TGTGCTTTCTGTATCCTCAGAGTGGCATTCTGCATTTCTGTGGCTTCCAAG TCTTAGAAYCTCAACTGACATATAGCATTGGGCACACTCCAGCAGACGCC CGAATTCAAATCCTGGAAGGATGGAAGAAACGCCTGGAGAATATTTGGG ATGAGACACCACTGTATTTTGCTCCAAGCAGCCTCTTTGACCTAAACTT |
| SEQ ID NO:37 | MAMA | rs1801516 located at the gene ATM | TTTTTACTCAAACTATTGGGTGGATTTGTTTGTATATTCTAGGTGAAAACT GACTTTTGTCAGACTGTACTTCCATACTTGATTCATGATATTTTACTCCAA RATACAAATGAATCATGGAGAAATCTGCTTTCTACACATGTTCAGGGATT TTTCACCAGCTGTCTTCGACACTTCTCGCAAACGAGCCGATCCACAACCC CTGCAAACTTGGATTCAGGTATTCTATTAAATTTTTAACATTAATACT |
| SEQ ID NO:38 | MAMB | rs80357713 located at the gene BRCA1 | AAAGATATATATATATGTTTTCTAATGTGTTAAAGTTCATTGGAACAGA AAGAAATGGATTTATCTGCTCTTCGCGTTGAAGAAGTACAAAATGTCATT AATGCTATGCAGAAAATCTTNAGTGTCCCATCTGGTAAGTCAGCACAAG AGTGTATTAATTTGGGATTCCTATGATTATCTCCTATGCAAATGAACAGA ATTGACCTTACATACTAGGGAAGAAAAGACATGTCTAGTAAGATTAGGC TA |
| SEQ ID NO:39 | MAMC | rs80359380 located at the gene BRCA2 | CTACTAGTTTAGCTTGTGTTGAAATTGTAAATACCTTGGCATTAGATAAT CAAAAGAAACTGAGCAAGCCTCAGTCAATTAATACTGTATCTGCACATTT ACAGAGTAGTGTAGTTGTTTCTGATTGTAAAAATAGTCATATAACCCCTN CAGATGTTATTTTCCAAGCAGGATTTTAATTCAAACCATAATTTAACACC TAGCCAAAAGGCAGAAATTACAGAACTTTCTACTATATTAGAAGAATCA G |
| SEQ ID NO:40 | MAMD | rs144848 located at the gene BRCA2 | CATCTGGGCTCCATTTAGACCTGAAAGGGTTAGTTGAGACCATTCACAGG |
| | | | CCAAAGACGGTACAACTTCCTTGGAGATTTTGTCACTTCCACTCTCAAAG GGCTTCTGATKTGCTACATTTGAATCTAATGGATCAGTATCATTTGGTTCC ACTTCAGATACAAATGAGTATTTTTCTTTCACTTGGTTTTTAGATTTTTCA CATTCATCAGCGTTTGCTTCATGGAAAATTTTTTTCCTAGTCTTGCTA |
| SEQ ID NO:41 | MAME | rs2981582 located at the gene FGFR2 | TGAAATCTGTCATCAGTAGGGAATATTGGTAGCTGAGTTATTTTTCGAGT GGTAATCCGAGAATAAAACGGCAGATCCCAGCACTCATCGCCACTTAAT GAACCTGTTTGYGGAGAGTCCACCTGGTGCCTGCCTGGCTTTAGGAACCC GCAGCAGTCCGAGTGGTGTCTGGGGTAAGCTGAGCTGCTCTGGGAACAC ATCTCGTGCGTGGGGTGAATGAACAGCACACTTACCCAGTGGGGTAGGC TGG |
| SEQ ID NO:42 | MAMF | rs1800470 located at the gene TGFB1 | CCAAGCCTCCCCTCCACCACTGCGCCCTTCTCCCTGAGGACCTCAGCTTTC CCTCGAGGCCCTCCTACCTTTTGCCGGGAGACCCCCAGCCCCTGCAGGGG CGGGGCCTCCCCACCACACCAGCCCTGTTCGCGCTCTCGGCAGTGCCGGG GGCGCCGCCTCCCCCATGCCGCCCTCCGGGCTGCGGCTGCTGCBGCTGC TGCTACCGCTGCTGTGGCTACTGGTGCTGACGCCTGGCCGGCCGGCCGC |
| SEQ ID NO:43 | MAMG | rs5780218 located at the gene KiSS1 | TGCAGTGTGCCTACCTTGCCTCAGTCCTGGCCTGGGCAGGAGTCTGGCGG AGCCTCTGAGGTGACGAGACCACCTGGCTGGGTGAATGTCCAGAGGGTG GGCAGCTGGGCTCCCGGTCTCNAGAGTTCTCCCCAGCTCCCTGATCACAT CCCTTTTATAAAGCCCGGGGTGACGTCTCCAAGCACAGGCCCACTCTGCC GCCCCTCCCTCCTCCTCAGACCCTCCTGAAGAGCATGGCACCTTGGGAGG C |
| SEQ ID NO:44 | PROA | rs5186 located at the gene AGT1R | GCTCATCCACCAAGAAGCCTGCACCATGTTTTGAGGTTGAGTGACATGTT CGAAACCTGTCCATAAAGTAATTTTGTGAAAGAAGGAGCAAGAGAACAT TCCTCTGCAGCACTTCACTACCAAATGAGCMTTAGCTACTTTTCAGAATT GAAGGAGAAAATGCATTATGTGGACTGAACCGACTTTTCTAAAGCTCTG AACAAAAGCTTTTCTTTCCTTTTGCAACAAGACAAAGCAAAGCCACATTT TG |
| SEQ ID NO:45 | PROB | rs13821319 7 located at the gene HOXB 13 | AAACTCAGTGGGGCGGCTGGGGTACTCTTCCCCGGCCGTGGAGTCTCCG CGGGGTACGCGGCCAGGGTGGCTGCCTGGGCACAGGGTTTCAGCGAGCT CCGGGACACTCGGCAGGAGTAGTACCCGCCTYCAAAGTAACCATAAGGC ACGGGAGCTGGGGACGTCCCCTGGGGCACCCCAGGGCATGGGTGGCATT GCTTTGGCGGCTCCGCCGAGCCTGGCAGATCCAAGGGGGCATAGTTGAC AGCA |
| SEQ ID NO:46 | PROC | rs9282858 located at the gene SRD5A2 | CYAGCAGAGCCCAGTGCTGGCAGGCAGCGCCACTTTGGTCGCCCTTGGG GCACTGGCCTTGTACGTCGCGAAGCC TCCGGCTACGGGAAGCACACGGAGAGCCTGAAGCCGGCGGCTACCCGCC TGCCARCCCRCGCCGCCTGGTTCCTGCAGGAGCTGCCTTCCTTCGCGGTG CCCGCGGGGATCCTCGCCCGGCAGCCCCTCTCCCTCTTCGGGCCACCTGG GACGGTACTTCTGGGCCTCTTCTGCS |
| SEQ ID NO:47 | PROD | rs523349 located at the gene SRD5A2 | TTGGCGTTCCTCGGTGCGCGCTCTACGCTGCGCTCCTGGACGCCGGGAGC AGGGCAGTGCGCTGCACTGGGCGCCCGCAAGGGAAAAACGCTACCTGTG GAAGTAATGTASGCAGAAGAGGCCCAGAAGTACCGTCCCAGGTGGCCCG AAGAGGGAGAGGGGCTGCCGGGCGAGGATCCCCGCGGGCACCGCGAAG GAAGGCAGCTCCTGCAGGAACCAGGCGGCGCGGGCTGGCAGGCGGGTA GCCGCC |
| SEQ ID NO:48 | PROE | rs4430796 located at the gene TCF2 | CTTTTGAGAAGTTCCAGTCTGCCTTTCCTGCCTCCTTCTCCTTTCTGAAG AAATTCTCATTGAATACAGAGAGGCAGCACAGACTGGARATGCTGCATA AAGCTTAAATTGGGCAGGGCCCAAGCGTTGTTGGGTCTTTGGAGACAAT |
| | | | GGCTCCTGAGAATTTTTTAGGCTTTCCAGGAACTACAGAGAGTTGCTTC ATGTCAGGAACACAAATTCTTAAAGAGCTAGTCACCAAGTATGGGGGC CA |
| SEQ ID NO:49 | PROF | rs12757998 located at the gene RNASEL | TCTTTGCTTCCACTTTAATCTCTTGTCTTTTATTTTTGTCACACTTCTCC ATCCCCTTTTTCCTCCCTCCTCTACGCCTCCCTAAACTTTTTCTCCTCTTT CTATCCTACTTCACCTCTTCTCTTTTCCTCATATACTCATACTCCTCTGCAT TCCYGCCTTCATATTTCCCCCCTCCCTCCTTTCCCTCAACTTTCTCTCCCCA GTCCCATCTTCTTGTTCTTCCTATTGTTGTCACTTTTTTCTC |
| SEQ ID NO:50 | PANA | rs505922 located at the gene ABO | CTGACATGAGACCAACTAAGACAGCATAACCCGTGGGGCAGGACAGACT CCTGGGGTGGCAGCTGTGTTCATCATCTTGACTGGGTCAAGATGTATCCA GCTGTACCTTTYATGTGCGGTTTATTGTATACATCCGTTGAAACATATTAA GACAAAAAAGGGAAAACAAAGACCACAAAGGAGGGACAGGGAAGAAC CCGGAAGGCGCTGGCAGCACAGGGAGGAGGCAGGTGTTTGCAGTCAGAC CTGA |
| SEQ ID NO:51 | PANB | rs11549465 located at the gene CCKBR | TTAGGGCTTCGCTTTGACGGCGACAGTGACAGCGACAGCCAAAGCAGGG TCCGAAACCAAGGCGGGCTGCCAGGTGGGGCTGGACCACGTGAGCAAAA TCTGGGVGAGGCGGAGCTTTGGAGGGCGACGGGGCCTGCTGGAGTGGGT GGGACTGAAATGAAGGTGAGGGTGAGAAGGAAGCTGGAAATGGAGTTG AGCTGGGAGCGGAGGTCAGGTGGGGACTGGGCTGGAGACTGGGGGGAC TCGCCTT |
| SEQ ID NO:52 | PANC | rs9543325 located at the Chromoso me 13 | CATTCAAAGGAACTCTTGTGGGCTCAAGGTTGGAGTTGATAGATTCAGC AAGAATTTTCCCTCTTCAACTTCGTCAGCCCAATCCTCAGTAGGTAAAG TTGTCTTCTTGTCTGATGCACGTGCTGCCCTTGCTGCACYTCTATCACACC TGTGCCTTCTCTATCATGACACTTAATCTACCGTCCTGTAGATGGGAGTCC TGGCAGGACACAGATAACCTCTTGGAGCAGTACTCTCCATTCCTCTAT |
| SEQ ID NO:53 | PAND | rs7190458 located at the gene BCAR1 | GCAGGAGGTCGCACAGCAGGTTGCTGTAGTGGGTCACCTGGCTGCGCAC GTCAGCAGCCTTGGCCTGCCGTGACAGTGTGTCCCCGATGAACACCAGCT TGTGGGCGCTRAGGATGACGAACTTGCTGTGCGCCACAAAGATCTTGGG CGGCTGGTTGGTGGCCACGGCGGTAAAGAAGGCGTCCACGGCGTTGGTC AGTGTGGTCAGGTTGGCCTCACACTGCTCCAGGTAGAAGAGCAGCAGCT GCCG |
| SEQ ID NO:54 | PULA | rs4646903 located at the CYP1A1 | TGGGTTTACTGTAGGGAGGAAGAAGAGGAGGTAGCAGTGAAGAGGTGT AGCCGCTGCACTTAAGCAGTCTGTTTGAGGGACAAGACTCTATTTTTGA GACAGGGTCCCCAGGTCATCCAGGCTGGAGTGCACTGGTACCATTTGTT TCACTGTAACCTCCACCTCCYGGGCTCACACGATTCTCCCACCTCAGCCT CTGAGTAGTTGGGGCCGCAGGCACACGCCACCACAGCTTTTTTTTTTTTT |
| SEQ ID NO:55 | PULB | rs12143456 9 located at the EGFR | GTGTGCCGCCTGCTGGGCATCTGCCTCACCTCCACCGTGCARCTCATCAY GCAGCTCATGCCCTTCGGCTGCCTCCTGGACTATGTCCGGGAACACAAAG ACAATATTGGCTCCCAGTACCTGCTCAACTGGTGTGTGCAGATCGCAAAG GTAATCAGGGAAGGGAGATACGGGGAGGGGAGATAAGGAGCCAGGATC CTCACATGCGGTCTGCGCTCCTGGGATAGCAAGAGTTTGCCATGGGGATA TG |
| SEQ ID NO:56 | PULC | rs2234922 located at the EPHX1 | AGCCTGACCGTGCAGGGTCTTCTCTCCCTCCACCCTGACTGTGCTCTGT CCCCCCAGGGCTGGACATCCACTTCATCCACGTGAAGCCCCCCCAGCTGC CCGCAGGCCRTACCCCGAAGCCCTTGCTGATGGTGCACGGCTGGCCCGGC TCTTTCTACGAGTTTTATAAGATCATCCCACTCCTGACTGACCCCAAGAA CCATGGCCTGAGCGATGAGCACGTTTTGAAGTCATCTGCCCTTCCATC |
| SEQ ID NO:57 | PULD | rs1051730 located at the CHRNA3 | GTCCTACGATAAGGCGAAAATCGATCTGGTCCTGATCGGCTCTTCCATGA ACCTCAAGGACTATTGGGAGAGCGGCGAGTGGGCCATCATCAAAGCCCC AGGCTAYAAACACGACATCAAGTACAACTGCTGCGAGGAGATCTACCCC GACATCACATACTCGCTGTACATCCGGCGCCTGCCCTTGTTCTACACCAT CAACCTCATCATCCCCTGCCTGCTCATC TCCTTCCTCACTGTGCTCGTCTTC |
| SEQ ID NO:58 | PULE | rs10488600 3 located at the PIK3CA | TATATAAGATATTATTTATTTACAGAGTAACAGACTACTAGAGACAAT GAATTAAGGGAAAATGACAAAGAACAGCTCAAAGCAATTTCTACACGAG ATCCTCTCTCTGAAATCACTRAGCAGGAGAAAGATTTTCTATGGAGTCAC AGGTAAGTGCTAAAATGGAGATTCTCTGTTTCTTTTTCTTTATTACAGAAA AAATAACTGAATTTGGCTGATCTCAGCATGTTTTTACCATACCTATTGG |
| SEQ ID NO:59 | PULF | rs12191327 9 located at the PIK3CA | GAAAGACCCTAGCCTTAGATAAAACTGAGCAAGAGGCTTTGGAGTATTT CATGAAACAAATGAATGATGCACDTCATGGTGGCTGGACAACAAAAATG GATTGGATCTTCCACACAATTAAACAGCATGCATTGAACTGAAAAGATA ACTGAGAAAATGAAAGCTCACTCTGGATTCCACACTGCACTGTTAATAAC TCTCAGCAGGCAAAGACCGATTGCATAGGAATTGCACAATCCATGAACA GCAT |
| SEQ ID NO:60 | PULG | rs1048943 located at the CYP1A1 | CCACTTCAGCTGTCTCCCTCTGGTTACAGGAAGCTATGGGTCAACCCATC TGAGTTCCTACCTGAACGGTTTCTCACCCCTGATGGTGCTATCGACAAGG TGTTAAGTGAGAAGGTGATTATCTTTGGCATGGGCAAGCGGAAGTGTATC GGTGAGACCNTTGCCCGCTGGGAGGTCTTTCTCTTCCTGGCTATCCTGCT GCAACGGGTGGAATTCAGCGTGCCACTGGGCGTGAAGGTGGACATGACC C |
| SEQ ID NO:61 | GENA | rs2279744 located at the MDM2 | GGAGGGCGGGATTTCGGACGGCTCTCGCGGCGGTGGGGTGGGGGTGGT TCGGAGGTCTCCGCGGGAGTTCAGGGTAAAGGTCACGGGGGCCGGGGGC TGCGGGGCCGCTKCGGCGCGGGAGGTCCGGATGATCGCAGGTGCCTGTC GGGTCACTAGTGTGAACGCTGCGCGTAGTCTGGGCGGGATTGGGCCGGT TCAGTGGGCAGGTTGACTCAGCTTTTCCTCTTGAGCTGGTCAAGTTCAGA CACG |
| SEQ ID NO:62 | GENB | rs11348802 2 located at the BRAF | ATTACAGAATTATAGAAATTAGATCTCTTACCTAAACTCTTCATAATGCT TGCTCTGATAGGAAAATGAGATCTACTGTTTTCCTTTACTTACTACACCTC AGATATATTTCTTCATGAAGACCTCACAGTAAAAATAGGTGATTTTGGTC TAGCTACAGWGAAATCTCGATGGAGTGGGTCCCATCAGTTTGAACAGTT GTCTGGATCCATTTTGTGGATGGTAAGAATTGAGGCTATTTTTCCACTGA |
| SEQ ID NO:63 | GEND | rs12191266 4 located at the TP53 | GCAGTTTCTACTAAATGCATGTTGCTTTTGTACCGTCATAAAGTCAAACA ATTGTAACTTGAACCATCTTTTAACTCAGGTACTGTGTATATACTTACTTC TCCCCCTCCTCTGTTGCTGCAGATCCGTGGGCGTGAGCNCTTCGAGATGT TCCGAGAGCTGAATGAGGCCTTGGAACTCAAGGATGCCCAGGCTGGGAA GGAGCCAGGGGGGAGCAGGGCTCACTCCAGGTGAGTGACCTCAGCCCCT T |
| SEQ ID NO:64 | GENE | rs1136201 located at the ERBB2 | GAATGCCAAACACCTTCATGTCCCCGTGGCCCCCTTTGTCCCTCCCAC CCCAAACTAGCCCTCAATCCCTGACCCTGGCTTCCGCCCCCAGCCCTCTG ACGTCCATCRTCTCTGCGGTGGTTGGCATTCTGCTGGTCGTGGTCTTGGG GGTGGTCTTTGGGATCCTCATCAAGCGACGGCAGCAGAAGATCCGGAAG TACACGATGCGGAGACTGCTGCAGGAAACGGAGGTGAGGCGGGGTGAA GTC |
| SEQ ID NO:65 | GENG | rs741765 located at the STK11 | TGCGTAGGAAGTGCAGCAGGACAACCCTGACAGACCCTGCAAGCCAGGG AGCCCCCGAGGGTGGGGCTGGGGGAGGCGCAGGGCAGCACTGCCCTCTG GGGTGGGAGTTCRGAGGGTGAACAGGGCCCAGAAGCAGGGTGGAGCTG AGGGACCTGGCAAACCAGGCCCTGCGGGGAGGTCGGCCTCTCCACTCAG TCCTCTCAATGCCTGCTGGGGTCTCGGGGCCAATAGCCCCCCACCTGACA CCCCC |
| SEQ ID NO:66 | GENH | rs12191352 9 located at the KRAS | TTGAAATAATTTTTCATATAAAGGTGACAGTATTTGATAGTGTATTAACC TTATGTGTGACATGTTCTAATATAGTCACATTTTCATTATTTTTATTATAA GGCCTGCTGAAAATGACTGAATATAAACTTGTGGTAGTTGGAGCTGNTG NCGTAGGCAAGAGTGCCTTGACGATACAGCTAATTCAGAATCATTTTGTG GACGAATATGATCCAACAATAGAGGTAAATCTTGTTTTAATATGCATATT |
| SEQ ID NO:67 | GENI | rs11244544 1 located at the KRAS | TAATATGCATATTAAAACAAGATTTACCTCTATTGTTGGATCATATTCGTC CACAAAATGATTCTGAATTAGCTGTATCGTCAAGGCACTCTTGCCTACGN CACCAGCTCCAACTACCACAAGTTTATATTCAGTCATTTTCAGCAGGCCT TATAATAAAAATAATGAAAATGTGACTATATTAGAACATGTCACACATA AGGTTAATACACTATCAAATACTCCACCAGTACCTTTTAATACAAACTCA |

As used herein, "HPV" refers to human papillomavirus, DNA viruses from the papillomavirus family which include more than 170 types or strains. HPV infection spreads by sustained direct contact such as skin-to-skin contact. Most HPV infections cause no symptoms and resolve spontaneously. However, in some people HPV infection persists and may result in warts or precancerous lesions that increase the risk of several cancers including cervical, vulva, vagina, penis, anus, mouth and throat cancers. HPV strains are classified depending on the genital cancer risk associated with said HPV infection. As such, HPV-16, HPV-18, HPV-31 and HPV-45 are the highest risk HPV types for genital cancers, while HPV-33, HPV-35, HPV-39, HPV-51, HPV-52, HPV-56, HPV-58 and HPV-59 are classified as high risk HPV types, and HPV-66, HPV-68 and HPV-73 are classified as probably high-risk HPV types. As used herein, "high-risk HPV strains" refers to both highest risk and high risk HPV types without further distinction. HPV-16 is also associated with the development of oro-laryngo-pharyngeal cancers. Nearly all cases of cervical cancer are associated with an HPV infection. Table 23 lists the fragment of L1 of HPV used to identify the strain genotypes.

**Table 23: HPV strain sequences**

| SEQ ID NO | HPV name | HPV strain | Fragment of gene L1 Sequence |
|---|---|---|---|
| SEQ ID NO:68 | HPVA | Fragment of gene L1 of HPV-16 | GCTGGTRCTGTTGGTGAAAATGTACCAGACGATTTATACATTAAAGGCTC TGGGTCTACTGCAAATTTAGCCAGTTCAAATTATTTTCCTACACCTAGTG GTTCTATGGTTACCTCTGATGCCCAAATATTYAATAAACCTTATTGGTTAC |
| | | | AACGAGCACAGGGCCACAATAATGGCATTTGTTGGGGTAACCAACTATT TGTTACTGTTGTTGATACTACACGCAGTACAAATATGTCATTATGTGCTG |
| SEQ ID NO:69 | HPVB | Fragment of gene L1 of HPV-18 | GTGGGCATCCATTTTATAATAAATTAGATGACACTGAAAGTTCCCATGCC GCYACGTCTAATGTTTCTGAGGACGTTAGGGACAATGTGTCTGTAGATTA TAAGCAGACACAGTTATGTATTTTGGGCTGTGCCCCTGCTATTGGGGAAC ACTGGGCTAAAGGCACTGCTTGTAAATCGCGTCCTTTATCACAGGGCGAT TGCCCCCCTTTAGAACTTAAAAACACAGTTTTGGAAGATGGTGATATGGT |
| SEQ ID NO:70 | HPVC | Fragment of gene L1 of HPV-31 | ACACTGAAAACTCTAATAGATATGCCGGTGGTCCTGGCACTGATAATAG GGAATGTATATCRATGGATTATAAACAAACACARCTGTGTTTACTTGGTT GCAAACCACCTATTGGAGAGCATTGGGGTAARGGTAGTCCTTGTAGTAA YAATGCTATTACCCCTGGTGATTGTCCTCCATTAGAATTAAARAATWCAG TTATACAAGATGGGGATATGGTTGATACAGGCTTTGGAGCTATGGATTTT AC |
| SEQ ID NO:71 | HPVD | Fragment of gene L1 of HPV-33 | ATGGGCATGTGTAGGCCTTGAAATAGGTAGAGGGCAGCCATTAGGCGTT GGCATAAGTGGTCATCCTYTATTAAACAAATTTGATGACACTGAAACCRG TAACARGTATCCTGGACAACCGGGTGCTGATAATAGGGAATGTTTATCCA TGGATTATAAACAAACACAGTTATGTTTACTTGGATGTAAGCCTCCAACA GGGGAACATTGGGGTAAAGGTGTTGCTTGTACTAATGCAGCACCTGCCA AT |
| SEQ ID NO:72 | HPVE | Fragment of gene L1 of HPV-35 | TAAGGCATGGTGAAGAATATGATTTACAGTTTATTTTCAGTTATGTAAA ATAACACTAACAGCAGATGTTATGACATATATTCATAGTATGAACCCGTC CATTTTAGAGGATTGGAATTTTGGCCTTACACCACCGCCTTCTGGTACCTT AGAGGACACATATCGCTATGTAACATCACAGGCTGTAACTTGTCAAAAA CCCAGTGCACCAAAACCTAAAGATGATCCATTAAAAAATTATACTTTTTG |
| SEQ ID NO:73 | HPVF | Fragment of gene L1 of HPV-39 | GTGTATTTGCCTCCACCTTCTGTGGCGAAGGTTGTCAATACTGATGATTAT GTTACACGCACAGGCATATATTATTATGCTGGCAGCTCTAGATTATTAA CAGTAGGACATCCATATTTTAAAGTGGGTATGAATGGTGGTCGCAAGCA GGACATTCCAAAGGTGTCTGCATATCAATATAGGGTATTTCGCGTGACAT TGCCCGATCCTAATAAATTCAGTATTCCAGATGCATCCTTATATAATCCA G |
| SEQ ID NO:74 | HPVG | Fragment of gene L1 of HPV-45 | CGTGGCACTGTTAGRTTTAGTAGATTGGGTCAAAGGGCAACCATGTTTAC ACGTAGTGGTAAACAAATAGGGGGTAGGGTACATTTTTACCATGATATA AGCCCCATTGCTGCTACAGAGGAAATTGAATTGCAGCCTTTAMTTAGTGC TACAGATGATAGTGACCTGTTTGATGTATATGCAGACTTYCCACCTCCTG CGTCCACTACACCTAGCACTATAAACAAATCATTTACATATCCAAAGTAT T |
| SEQ ID NO:75 | HPVH | Fragment of gene L1 of HPV-51 | GAGTGGGTTRGGTATATTTTGGGTGGCCTAGGTATTGGTACKGGGTCTG GATCTGGGGGGCGTACTGGATATATCCCTTTAGGTGGTGGGGGTCGCCCA GGCGTGGTGGATATTGCTCCTGCAAGGCCACCTATTATAATTGAACCTAT AGCACCTACTGAACCTTCTATAGTAAATTTGGTTGAVGACTCTAGTATTA TTCAGTCTGGGTCTCCTATACCTACCTTTACTGGTACCGATGGCTTTGAA |
| SEQ ID NO:76 | HPVI | Fragment of gene L1 of HPV-52 | GTACAAAGCAGTGCTTTTTTCCTACTCCTAGTGGTTCTATGGTAACCTCA GAATCCCAATTATTTAATAAACCGTACTGGTTACAACGTGCGCAGGGCCA CAATAATGGCATATGTTGGGGCAATCAGTTGTTTGTCACAGTTGTGGATA CCACTCGTAGCACTAACATGACTTTATGTGCTGAGGTTAAAAAGGAAAG CACATATAAAAATGAAAATTTTAAGGAATACCTTCGTCATGGCGAGGAA T |
| SEQ ID NO:77 | HPVJ | Fragment of gene L1 of HPV-56 | AAATAATAATGTTATAGAAGATAGTAGGGACAATATATCAGTTGATGGC AAGCAAACACAGTTGTGTATTGTTGGATGTACTCCCGCTATGGGTGAACA TTGGACTAAAGGTGCTGTGTGTAAGTCCACACAAGTTACCACAGGGGAC TGCCCGCCTCTTGCATTAGTTAATACACCTATAGAGGATGGGGACATGAT AGACACAGGATTTGGCGCTATGGACTTTAAGGTGTTGCAGGAATCTAAG GCT |
| SEQ ID NO:78 | HPVK | Fragment of gene L1 of HPV-58 | TGCGTCGCAGACGTAAACGTTTTCCATATTTTTTGCAGATGTCCGTGTGG CGGCCTAGTGAGGCCACTGTGTACCTGCCTCCTGTGCCTGTGTCTAAGGT TGTAAGCACTGATGAATATGTGTCACGCACAAGCATTTATTATTATGCTG GCAGTTCCAGACTTTTGGCTGTTGGCAATCCATATTTTTCCATCAAAAGTC CCAATAACAATAAAAAAGTATTAGTTCCCAAGGTATCAGGCTTACAGT |
| SEQ ID NO:79 | HPVL | Fragment of gene L1 of HPV-59 | TTCCTAAGGTGTCTGCATATCAATACAGAGTATTTAGGGTTAAKTTACCT GATCCYAATAAATTYGGCCTTCCAGATAACACAGTATATGATCCTAACTC TCAACGCYTGGTCWGGGCCTGTGTAGGTGTTGAAATCGGTCGGGGCCAA CCTTTAGGGGTAGGACTYAGTGGTCATCCATTMTATAATAAATTRGATGA CACTGAAAACTCTCATGTAGCATCTGCTGTTGATAMCAAAGATACACGT GA |
| SEQ ID NO:80 | HPVM | Fragment of gene L1 of HPV-66 | ATATATACAGGGAGCYACATTTGCACTATGGCCTGTATATTTTTTAAAC GTAGGCGCCGTAAACGTATTCCCTATTKTTTGCAGATGGCGATGTGGCG GCCTAGTGACAATAAGGTGTACCTACCTCCAACACCTGTTTCAAAGGTTG TGGCAACGGATACATATGTAAAACGTACCAGTATATTTATCATGCAGGT AGCTCTAGGTTGCTTGCTGTTGGCCATCCTTATTACTCTGTTTCYAAATC |
| SEQ ID NO:81 | HPVN | Fragment of gene L1 of HPV-68 | ATGGCATTGTGGCGMKCTAGCGACAACATGGTGTATTTGCCTCCCCCTC AGTGGCGAAGGTTGTCAATACAGATGATTAYGTRACACGCACTGGCATK TATTAYTATGCTGGTACATCTAGGTTATTAACTGTAGGCCATCCATATTTT AARGTYCCTATGTCTGGGRGCCGYAAGCAGGRCATTCCTAAGGTGTCTGC ATATCAATAYAGRGTGTTTMGRRTTWCCYTRCCYGATCCTAATAAATTTA |
| SEQ ID NO:82 | HPVO | Fragment of gene L1 of HPV-16 | CCTTTTATAATCCTGATAAGGAGCGCCTAGTATGGGCCTGTTCTGGTGTG GAGGTTGGACGTGGACAACCCTTAGGTATAGGTACTAGTGGCAATCCATT TATGAATAAATTRGATGATACTGAAAATGCTCCTAAATACATTGCTGGAC AAAATACAGATGGTAGAGAATGTATGTCAGTGGATTATAAACAAACACA GTTGTGTATTTTAGGTTGTAGGCCTCCCTTAGGGGAACATTGGGGTCCAG G |

Infections with high-risk HPV strains have been associated with cancers. It has been reported that HPV-16 and HPV-18 cause approximately 80% of cervical cancers worldwide, whereas the remaining 20% of cervical cancers are predominantly associated with other HPV-31, HPV-33, HPV-45 and HPV-58. High-risk HPV strains have also been reported to be associated with prostate cancer. The most common of such HPV strains is HPV-16, HPV-31, HPV-33 and HPV-18 along with HPV-58. HPV-11 and HPV-6 to a lesser extent. DNA testing has been used in identifying HPV strains. Thus, viral DNA may be targeted in the methods and kits herein to detect the presence of high-risk HPV serotypes.

HPV infection have also been reported to be associated with higher risks of developing non genital or oro-laryngo-pharyngeal cancers.

In one aspect, identifying the one or more cancer susceptibility marker genes is by amplifying a cell free DNA sample from the subject using primers to the one or more cancer susceptibility marker genes; and detecting the amplified DNA. In another aspect, amplifying the cell free DNA sample also uses primers to the one or more HPV strain genotypes.

The term "sample", may include whole blood, plasma, serum, buffy coat, a body fluid, lymphocytes, tissue, cultured cells and the like and, in particular, the term "sample" may refer to a urine sample, saliva sample, blood sample, blood cell-free DNA and specimen of or from the skin, mucous membrane or other body area or surface to be examined by means of a swab.

A urine sample from the patient may be collected in a urine collection sample cup. The cup may be pre-filled with a liquid suitable for preserving the urine sample. In various embodiments, the kit may contain a urine collection sample cup, pre-filled with a preservative liquid. The preservative liquid may include such preservatives as hydrochloric acid, boric acid, acetic acid, toluene or thymol.

The urine, blood or saliva sample may be a random specimen collected at any time following standards methods of collection.

The cell-free DNA may be extracted from the sample, including a urine sample, by any method known in the art including by using organic solvents such as a mixture of phenol and chloroform, followed by precipitation with ethanol. Among other methods of extracting cell-free DNA, one such method includes, for example, using polylysine-coated silica particles. Alternatively, the cell-free DNA may be extracted from the urine using a commercially available kit such as, for example, QlAamp® DNA minikit (Qiagen, Germantown, MD).

The extracted DNA is amplified by one of the alternative methods for DNA amplification well known in the art, which include for example: the Xmap® technology of Luminex that allows the simultaneous analysis of up to 500 bioassays through the reading of biological test on the surface of microscopic polystyrene bead; the multiplex PCR that allows the simultaneous amplification of several DNA sequences; the multiplex ligation-dependent probe amplification (MLPA) for the amplification of multiple targets using a single pair of primers; the quantitative PCR (qPCR), which measures and quantify the amplification in real time; the ligation chain reaction (LCR) that uses primers covering the entire sequence to amplify, thereby preventing the amplification of sequences with a mutation; the rolling circle amplification (RCA), wherein the two ends of the sequences are joined by a ligase prior to the amplification of the circular DNA; the helicase dependent amplification (HDA) which relies on a helicase for the separation of the double stranded DNA; the loop mediated isothermal amplification (LAMP) which employs a DNA polymerase with high strand displacement activity; the nucleic acid sequence based amplification, specifically designed for RNA targets; the strand displacement amplification (SDA) which relies on a strand-displacing DNA polymerase, to initiate replication at nicks created by a strand-limited restriction endonuclease or nicking enzyme at a site contained in a primer; and the multiple displacement amplification(MDA), based on the use of the highly processive and strand displacing DNA polymerase from the bacteriophage Ø29. PCR methods as used herein have been used in diagnostic tests and are well known in the art.

The extracted genomic DNA is for example amplified using multiplex PCR reaction carried out following the conditions detailed in Table 1.

**Table 1: Conditions for the amplification of genomic DNA by multiplex PCR**

| | Initial denaturation | | Denaturation | | Alignment | | Extension | | Final Extension | |
|---|---|---|---|---|---|---|---|---|---|---|
| | 1 cycle | | 40 cycles | | | | | | 1 cycle | |
| | °C | min. | °C | Sec. | °C | Sec. | °C | Sec. | °C | min. |
| Breast, prostate, lung, colon, gastric, leukemia, pancreas, prostate, cervical | 95 | 5 | 95 | 30 | 61-65 | 60 | 72 | 20 | 72 | 5 |

A primer is a short strand of RNA or DNA (generally about 18-22 bases) that serves as a starting point for DNA synthesis. Primers are used in methods of amplifying DNA. In one aspect, the primers to the one or more cancer susceptibility marker genes are designed to amplify cancer susceptibility marker genes CACUA. CACUB, CACUC, CACUD, CACUE, CACUF, CACUG, CACUH, CACUI, CACUJ, CAPA, CAPB, CAPC, CAPD, CAPE, CAPF, CAPG, CAPH, CAPI, CAPJ, COLA, COLB, COLC, COLD, COLE, COLF, COLG, GASA, GASB, GASC, GASD, GASE, GASG, LEUA, LEUB, LEUC, LEUD, LEUE, LEUF, MAMA, MAMB, MAMC, MAMD, MAME, MAMF, MAMG, PROA, PROB, PROC, PROD, PROE, PROF, PANA, PANB, PANC, PAND, PULA, PULB, PULC, PULD, PULE, PULF, PULG, GENA, GENB, GEND, GENE, GENG, GENH and/or GENI and/or SEQ ID NOs: 1-67 and the primers to the one or more HPV strain genotypes are designed to amplify HPV-16, HPV-18, HPV-31, HPV-33, HPV-35, HPV-39, HPV-45, HPV-51, HPV-52, HPV-56, HPV-58, HPV-59, HPV-66, HPV-68, HPV-73 and/or SEQ ID NOs:68-82.

As used herein "amplified DNA" or "PCR product" refers to an amplified fragment of DNA of defined size. Various techniques are available and well known in the art to detect PCR products. PCR product detection methods include, but are not restricted to, gel electrophoresis using agarose or polyacrylamide gel and adding ethidium bromide staining (a DNA intercalant), labeled probes (radioactive or non-radioactive labels, southern blotting), labeled deoxyribonucleotides (for the direct incorporation of radioactive or non-radioactive labels) or silver staining for the direct visualization of the amplified PCR products; restriction endonuclease digestion, that relies agarose or polyacrylamide gel or High-performance liquid chromatography (HPLC); dot blots, using the hybridization of the amplified DNA on specific labeled probes (radioactive or non-radioactive labels); high-pressure liquid chromatography using ultraviolet detection; electrochemiluminescence coupled with voltage-initiated chemical reaction/photon detection; and direct sequencing using radioactive or fluorescently labeled deoxyribonucleotides for the determination of the precise order of nucleotides with a DNA fragment of interest, oligo ligation assay (OLA), PCR, qPCR, DNA sequencing, fluorescence, gel electrophoresis, magnetic beads, allele specific primer extension (ASPE) and/or direct hybridization.

Oligonucleotide ligation assay (OLA) methods are also well known in the art. Briefly, this method allows the detection of target sequences previously amplified by PCR. The OLA method requires specific probes that include a TAG sequence. Anti-TAG sequence is coupled to the surface of a magnetic microsphere for the detection.

As used herein, a "probe" or "OLA probe" refers to the polynucleotide CAPable of selectively hybridizing to a target sequence of amplified DNA during the oligonucleotide ligation assay. The OLA probe of the invention includes a common region, at the 3 prime end, that may include a dye label; a sequence specific region that selectively recognizes the target sequence and a Tag, at the 5 prime end, for the recognition by the anti-tag sequences bound to the magnetic microspheres. In certain aspects, the probes are designed to detect CACUA. CACUB, CACUC, CACUD, CACUE, CACUF, CACUG, CACUH, CACUI, CACUJ, CAPA, CAPB, CAPC, CAPD, CAPE, CAPF, CAPG, CAPH, CAPI, CAPJ, COLA, COLB, COLC, COLD, COLE, COLF, COLG, GASA, GASB, GASC, GASD, GASE, GASG, LEUA, LEUB, LEUC, LEUD, LEUE, LEUF, MAMA, MAMB, MAMC, MAMD, MAME, MAMF, MAMG, PROA, PROB, PROC, PROD, PROE, PROF, PANA, PANB, PANC, PAND, PULA, PULB, PULC, PULD, PULE, PULF, PULG, GENA, GENB, GEND, GENE, GENG, GENH, GENI, HPV-16, HPV-18, HPV-31, HPV-33, HPV-35, HPV-39, HPV-45, HPV-51, HPV-52, HPV-56, HPV-58, HPV-59, HPV-66, HPV-68 HPV-73 and/or SEQ ID NOs: 1-82.

The assay methods and kits herein may be performed on a multiplexing system for magnetic beads read such as, for example, MAGPIX® (Luminex Corp. Madison, WI) or BioCode 2500 Analizer® (Applied BioCode, Inc).

In an additional aspect, the present invention provides a kit containing at least primers to one or more cancer susceptibility marker genes; primers to one or more human papillomavirus (HPV) strain genotypes; and instructions for use. In one aspect, the one or more cancer susceptibility marker genes are CACUA. CACUB, CACUC, CACUD, CACUE, CACUF, CACUG, CACUH, CACUI, CACUJ, CAPA, CAPB, CAPC, CAPD, CAPE, CAPF, CAPG, CAPH, CAPI, CAPJ, COLA, COLB, COLC, COLD, COLE, COLF, COLG, GASA, GASB, GASC, GASD, GASE, GASG, LEUA, LEUB, LEUC, LEUD, LEUE, LEUF, MAMA, MAMB, MAMC, MAMD, MAME, MAMF, MAMG, PROA, PROB, PROC, PROD, PROE, PROF, PANA, PANB, PANC, PAND, PULA, PULB, PULC, PULD, PULE, PULF, PULG, GENA, GENB, GEND, GENE, GENG, GENH and/or GENI; a single nucleotide polymorphism (SNP) located within the gene for IL10, TGF-β1, CYP1A1, ST3GAL4, IL4, AGTR1, ERBB2, HOXB13, Chr8, MSMB, RNASEL, CYP19, ABO, ABL1, ARID5B, Chr13, CCKBR, JAK2, NQO1, EGFR, EPHX1, CHRNA3, IRF4, PIK3CA, CXCL8, BRCA2, BRCA1, ATM, TCF2, TGFB1, KiSS1, APC, PGR, MTHFR, MUTYH, CDH1, IL1B, IL6, TLR4, TNF-α, MDM2, MLH1, BRAF, TP53, BCAR1, STK11, KRAS, FGFR2, SRD5A2, EIF3H and/or MSH6; and/or SEQ ID NOs: 1-67. In another aspect, the one or more HPV strain genotypes HPV-16, HPV-18, HPV-31, HPV-33, HPV-35, HPV-39, HPV-45, HPV-51, HPV-52, HPV-56, HPV-58, HPV-59, HPV-66, HPV-68 and/or HPV-73. In an additional aspect, the kit also contains probes to one or more one or more cancer susceptibility marker genes and/or probes to one or more HPV strain genotypes.

As various changes could be made in the above methods and compositions without departing from the scope of the invention, it is intended that all matter contained in the above description shall be interpreted as illustrative and not in a limiting sense.

### Other Embodiments

The detailed description set-forth above is provided to aid those skilled in the art in practicing the present invention. However, the invention described and claimed herein is not to be limited in scope by the specific embodiments herein disclosed because these embodiments are intended as illustration of several aspects of the invention. Any equivalent embodiments are intended to be within the scope of this invention. Indeed, various modifications of the invention in addition to those shown and described herein will become apparent to those skilled in the art from the foregoing description which do not depart from the spirit or scope of the present inventive discovery. Such modifications are also intended to fall within the scope of the appended claims.

Additionally, in some embodiments the methods may additionally include a treatment step. The term "treatment" refers to both 1) therapeutic treatments or measures that cure, slow down, lessen symptoms of, and/or halt progression of a diagnosed pathologic conditions or disorder, and 2) and prophylactic/ preventative measures. Those in need of treatment may include individuals already having a particular medical disorder as well as those who may ultimately acquire the disorder (i.e., those needing preventive measures).

"Cancer treatments" or "anti-cancer treatments" include surgery, chemotherapy, radiation therapy, targeted therapy, immunotherapy and combination thereof. Specific anti-cancer treatments are defined and administered depending on the cancer type and the stage of the disease. Cancer specific treatments and combinations are well described in the art.

"Chemotherapeutic agents and antineoplastic agents" are well known in the art and include: anti-microtubules agents; antimetabolite agents; topoisomerase inhibitors; alkylating agents; anthracyclines agents; and enzyme inhibitors agents.

Presented below are examples discussing the simultaneous detection of HPV infection and the genotyping of cancer susceptibility markers contemplated for the discussed applications. The following examples are provided to further illustrate the embodiments of the present invention, but are not intended to limit the scope of the invention. While they are typical of those that might be used, other procedures, methodologies, or techniques known to those skilled in the art may alternatively be used

### EXAMPLES

### EXAMPLE 1

### OBTAINING CELL-FREE DNA FROM A SAMPLE

Cell-free DNA was extracted from a sample using organic solvents such as a mixture of phenol and chloroform, followed by precipitation with ethanol. Cell-free DNA can also be obtained using polylysine-coated silica particles. Alternatively, the cell-free DNA may be extracted from the sample using a commercially available kit such as, for example, QIAamp® DNA minikit (Qiagen, Germantown, MD). The DNA sample must have a high purity, i.e. superior to 1.6 (260/280 nm) and its functionality must have been corroborated by the amplification of a general reference sample and with 100 ng of DNA a multiplex PCR reaction.

### EXAMPLE 2

### SAMPLE PREPARATION AND EVALUATION OF PRIMERS TO DETECT CACU RISK SNPs

Cell-free DNA samples from synthetic DNA or isolated from M1, *C. lupus familiaris*, *E. coli* and human immunodeficiency virus (HIV) were obtained and prepared to a final concentration of 50 ng/µL. Each analysis must include a negative control (water), a positive control - wild type (WT), a mutant positive control (MT) and a heterozygous positive control (HET).

Preparation of mixtures of synthetic controls for wild type (WT) and mutant (MT) for the end-point PCR amplification of high risk to CACU. Mixtures of oligonucleotide synthetic control WT and MT DNA for cervical cancer (CACU) SNPs CACUA, CACUB, CACUC, CACUD, CACUE, CACUF, CACUG, CACUH, and CACUJ were prepared to a concentration of 30 µM from the stock solution concentration of 200 µM. A sequential dilution was carried out until a final concentration of 0.03 µM was reached for each synthetic controls of WT and MT CACU SNPs A- J.

Preparation of the mixture of heterologous (HET) synthetic controls. HET synthetic controls were prepared by placing 5 µL of the mixture of synthetic controls WT at 0.03 µM and 5 µL from the mixture of MT synthetic controls at 0.03 µM of the CACU panel in a polypropylene tube of 0.2mL.

Preparation of the sense (L1) and reverse (L2) oligonucleotides. Separately, sense (L1) and reverse (L2) oligonucleotides were diluted to 100 µM of CACU SNPs A-J from the stock solution at a concentration of 200 µM.

Preparation of the mixture of oligonucleotides and Master Mix end-point PCR. The volume of each sense (L1) and reverse (L2) oligonucleotide, as indicated in the Table 2, were placed in a tube. To the tube that contains the sense and reverse oligonucleotides 15 µL of Master Mix 2X end -point PCR (Thermo Scientific) was added as described in Table 3. The mixture was homogenized with a pipette and the volume was adjusted for the number of reactions required or the PCR amplification of the CACU panel of 5 samples, plus an extra reaction (as described in Table 4). The MasterMix was added to the mixture of oligonucleotides for the end-point PCR amplification for 8 oligonucleotides panel CACU for the analysis of 5 samples, plus an extra reaction, as indicated in Table 5.

**Table 2. Volume of sense (L1) and reverse (L2) oligonucleotides required for the PCR reaction mixture of CACU array**

| Marker | Aliquot | C_{Final}(µM) |
|---|---|---|
| CACUA | L1 and L2 | 0.5 |
| CACUB | L1 and L2 | 0.5 |
| CACUC | L1 and L2 | 0.2 |
| CACUD | L1 and L2 | 0.4 |
| CACUF | L1 and L2 | 0.2 |
| CACUG | L1 and L2 | 0.2 |
| CACUH | L1 and L2 | 0.3 |
| CACUJ | L1 and L2 | 0.5 |
| Total volume of L1 and L2 | | |
| Final reaction volume | | |

**Table 3. Volume of master mix added to the oligonucleotide mixture for the PCR reaction mixture of CACU array.**

| Reagent | C Initial | C Final | Volume 1 rxn (µL) |
|---|---|---|---|
| Master mix | 2X | 1.5X | 15 |
| Final reaction volume | | | 20 µL |

**Table 4: Volume of sense (L1) and reverse (L2) oligonucleotides**

| Marker | Aliquot | C_{Initial} (µM) | C_{Final}(µM) | Volume 1 rxn (µL) | Volume 6 rxn (µL) |
|---|---|---|---|---|---|
| | | | | | |
| CACUA | L1 and L2 | 100 | 0.5 | 0.10 | 0.60 |
| CACUB | L1 and L2 | 100 | 0.5 | 0.10 | 0.60 |
| CACUC | L1 and L2 | 100 | 0.2 | 0.04 | 0.24 |
| CACUD | L1 and L2 | 100 | 0.4 | 0.08 | 0.48 |
| CACUF | L1 and L2 | 100 | 0.2 | 0.04 | 0.24 |
| CACUG | L1 and L2 | 100 | 0.2 | 0.04 | 0.24 |
| CACUH | L1 and L2 | 100 | 0.3 | 0.06 | 0.36 |
| CACUJ | L1 and L2 | 100 | 0.5 | 0.10 | 0.60 |
| Total volume of L1 and L2 | | | | 1.12 µL | 6.72 µL (3.36 µL Lland L2) |
| Final reaction volume | | | | 20 µL | 120 µL |

**Table 5. Added volume of Master Mix**

| Reagent | C_{Initial} | C_{Final} | Volume 1 rxn (µL) | Volume 6 rxn (µL) |
|---|---|---|---|---|
| Master mix | 2X | 1.5X | 15 | 90 |
| Final reaction Volume | | | 20 µL | 120 µL |

Preparation of PCR reaction mixture. Table 6 indicates that the number of PCR reaction mixtures was dependent on the total quantity of DNA samples that were analyzed. It included 4 controls per PCR trial of the 8 oligonucleotides CACU panel. The reaction mixtures were prepared in polypropylene tubes in the following order: water, Master mixture + oligonucleotides (L1 and L2), and DNA of the samples or of the synthetic controls. PCR amplification was performed using standard conditions. The PCR products were analyzed them by electrophoresis. The PCR products may be further analyzed by multiplex technology or sequencing.

**Table 6 components or each PCR reaction tube for the amplification or the CACU panel in DNA taken from urine and blood samples. *The indicated volume is measured starting with a DNA sample at 50 ng/µL initial concentration. In case the sample presents a minor concentration, the volume must be adjusted to 100 ng for the PCR reaction.**

| Reaction tube | C_{Initial} | C_{Final} | Sample volume (µL) | H₂O volume (µL) | Master Mix + Oligonucleotides volume (µL) |
|---|---|---|---|---|---|
| Negative control | --- | --- | --- | 3.88 | 16.12 |
| WT synthetic control | 0.03 µM | 0.003 µM | 2 | 1.88 | 16.12 |
| MT synthetic control | 0.03 µM | 0.003 µM | 2 | 1.88 | 16.12 |
| HET synthetic control | 0.03 µM | 0.003 µM | 2 | 1.88 | 16.12 |
| Biological sample DNA | 50 ng/µL | 100 ng | 2* | 1.88 | 16.12 |

The PCR products were evaluated by electrophoresis. Alternatively, the target sequences can be identified by qPCR using SYBR™ Green or TaqMan ™ probes labeled with different fluorophores as is illustrated in Figures 11 and 12.

For the detection and amplification of SNPs associated with the risk of cervical cancer, 8 pairs of specific oligonucleotides were used for each locus of interest. The initiating oligos flanked the locus of interest in the genes or in important sequences of the regulation of the genes (i.e. IL10, TGF-B1, CYP1A1, ST3GAL4 and IL4). The initiating oligos had a complementarity with their target sequence of 80%, 90% and 100%. The oligonucleotides "primers" designed for the SNPs at risk to cervical cancer had a range of alignment temperature (Tm) between 62 ° C and 64 ° C. The oligonucleotides "primers" for the detection of cervical cancer risk SNPs had a concentration range between 0.2 µM and 0.5 µM (Table 7).

**Table 7. Oligonucleotide primers conditions, OLA and OLA reporter probe**

| Panel | Primers (concentration range in µM) | OLA (concentration range in µM) | Reporter probe OLA (concentration range in µM) |
|---|---|---|---|
| CACU | 0.2 - 0.5 | 0.1 - 0.5 | 4 - 6 |

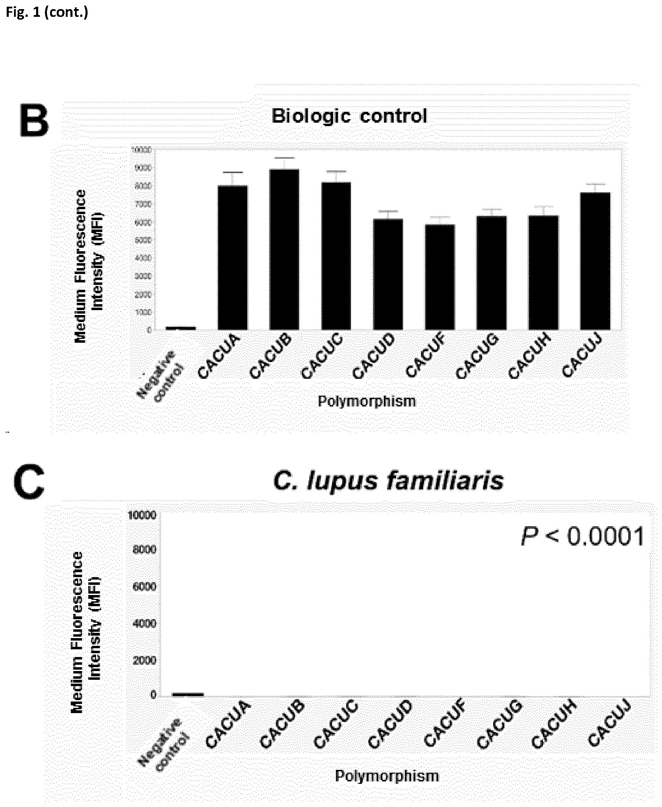

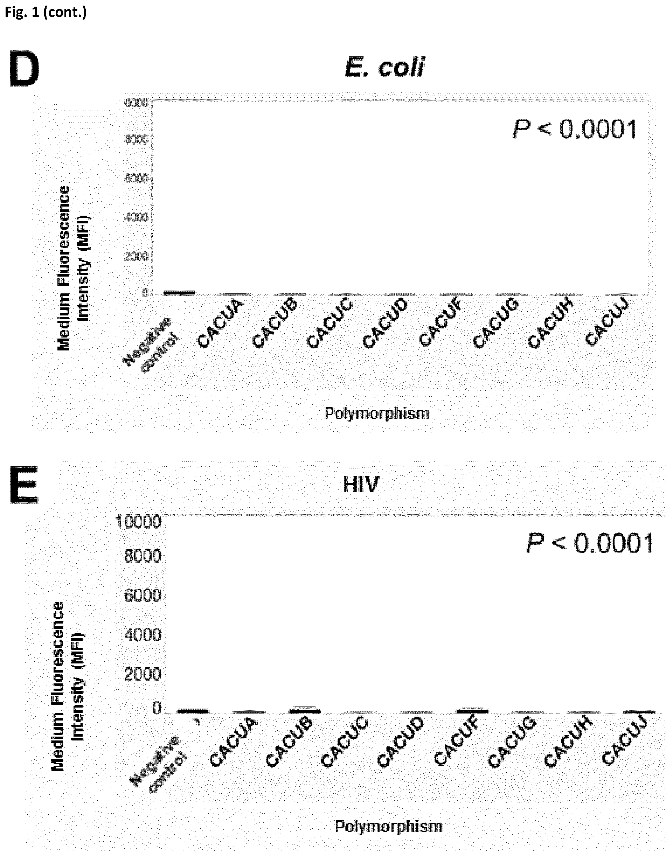

Figure 1 demonstrates that the oligonucleotides "primers" designed for the identification of risk SNPs to cervical cancer did not have cross-reactivity with other genomic DNA other than H. sapiens. Specifically, Figure 1A shows the detection of CACU risk SNPs A-J using synthetic controls (CS WT, CS MT and CS HET). Figure 1B shows the detection of CACU risk SNPs A-J using DNA isolated from a biological control M1. Figures 1C-E show that the CACU risk SNPs A-J were not detected in DNA isolated from *C. lupus familiaris*, *Escherichia coli.* or the Human Immunodeficiency Virus (HTV) (p<0.00001). The bars represent the Mean ± 1 Standard Deviation of 4 replicates. The P value represents the statistically significant difference of the MFI values recorded in each test with respect to the MFI values recorded in graph A and in graph B, determined by the Kruskal-Wallis test (α = 0.05). PC = 296.08 MFI (n = 60, 95% CI).

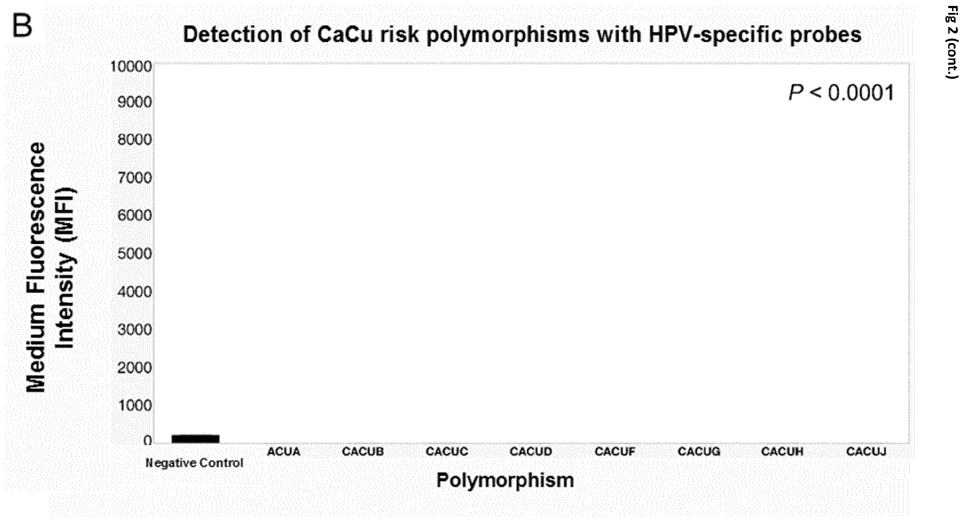

The PCR products were analyzed to determine intra assay specificity of the CACU probes for the detection of CACU risk SNPs A-J. Figure 2 illustrates the intra-assay specificity in the detection of CACU risk SNPs A-J using the M1 biological control. Figure 2A shows the detection of CACU risk SNPs A-J using specific CACU probes. Figure 2B shows the lack of detection of CACU risk SNPs A-J using specific probes for HPV detection. The bars represent the Mean ± 1 Standard Deviation of 4 replicates, the value of P represents the statistically significant difference between the MFI values recorded in Figure 2B with respect to the MFI values recorded in Figure 2A, determined by the Kruskal-Wallis test (α = 0.05), PC = 296.08 MFI (n = 64, 95% CI).

As illustrated in Figure 3, target sequences of DNA containing cancer susceptibility SNP were detected in blood and urine samples. The results of the PCR amplifications of CACUH shown that both urine (U) and blood (B), samples were suitable for DNA extraction and genotyping of such markers, with PCR products equivalent to those obtained with either a positive control (WT, wild type marker), a control DNA comprising the mutant marker (MT), or DNA extracted from a cell line that presented the SNP (CL).

These analyses demonstrated that blood and urine samples were suitable for the genotyping of cancer susceptibility markers and that DNA amplification combined with an OLA detected cancer susceptibility markers in a patient and thus established if the patient presents a susceptibility risk of developing the corresponding cancer.

### EXAMPLE 3

### EVALUATION OF THE CROSS REACTIVITY OF PRIMERS TO DETECT CAP RISK SNPs

Analysis of primers to detect prostate cancer risk polymorphisms was performed similarly as described in Example 3. Ten pairs of "primer" oligonucleotides specific to each locus of interest were used. The initiating oligos flanked the locus of interest in the genes or in important sequences of the regulation of the genes (i.e. AGTR1, HOXB13, SRD5A2, Chr8, MSMB, RNASEL and CYP19). The oligo "initiators" designed for the SNPs of risk to prostate cancer had complementarity with their target sequence of 80%, 90% and 100%. The oligonucleotides "primers" for SNPs at risk of prostate cancer had a range of alignment temperature (Tm) between 63 ° C and 65 ° C. Oligonucleotides "primers" for SNPs at risk of prostate cancer have a range of concentrations between 0.3 µM and 0.5 µM (Table 8).

**Table 8. Oligonucleotide primers conditions, OLA and OLA reporter probe**

| Panel | Primers (concentration range in µM) | OLA (concentration range in µM) | OLA reporter probe (concentration range in µM) |
|---|---|---|---|
| CAP | 0.3 - 0.5 | 0.1 - 0.4 | 4 - 6 |

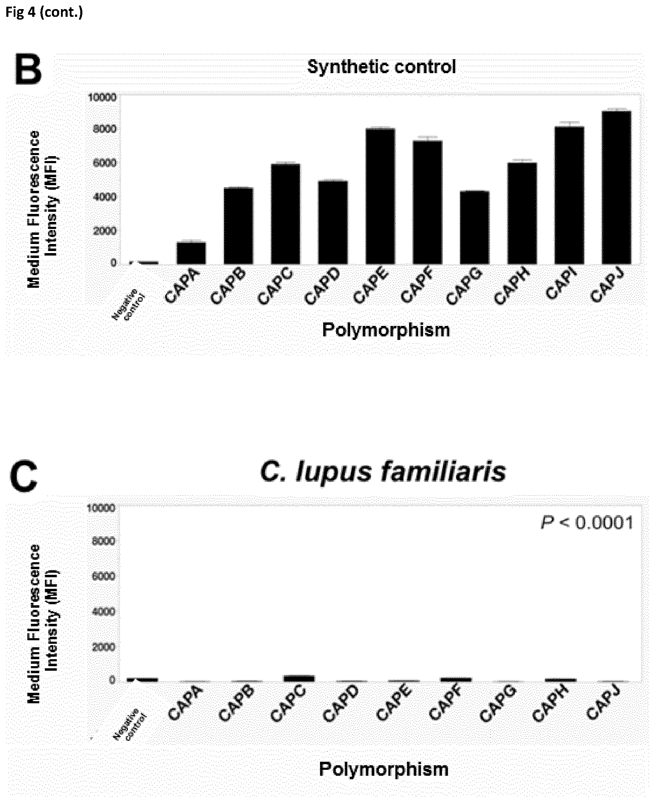

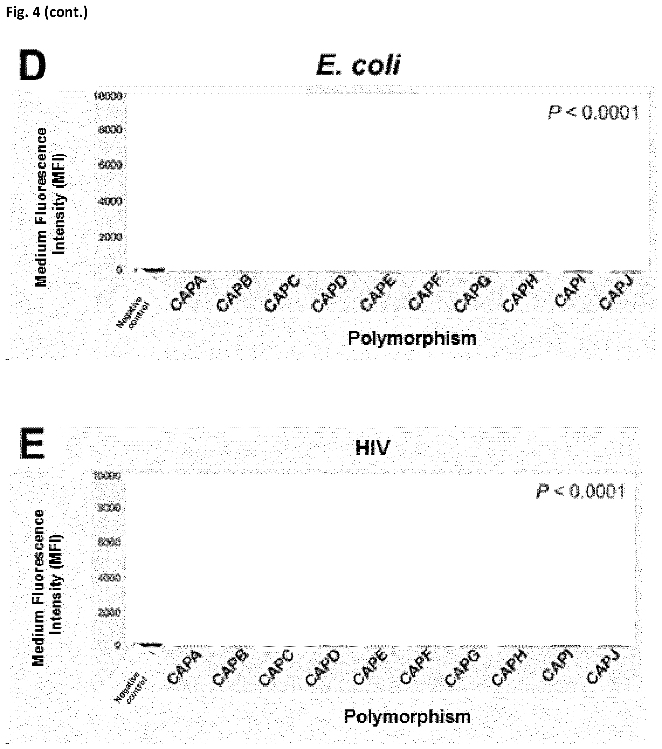

As illustrated in Figure 4, the primers used for the detection of prostate cancer risk SNPs (CAP) A-J were suitable for such detection in synthetic controls (Figure 4A) and in DNA isolated from a biological control (Figure 4B). However, there was no detection of CAP risk SNPs when the primers were used on DNA isolated *C. lupus familiaris* (Figure 4C, p<0.0001), *E. coli* (Figure 4D, p<0.0001) or HIV (Figure 4E, p<0.0001). The bars represent the Mean ± 1 Standard Deviation of 4 replicates, the P value represents the statistically significant difference of the MFI values recorded in each trial with respect to the MFI values recorded in Figure 4A and in Figure 4B, PC = 402.90 MFI (n = 80, 95% CI).

The oligonucleotides "primers" designed for the identification of risk SNPs to prostate cancer did not cross-react with other genomic DNA (Figure 4).

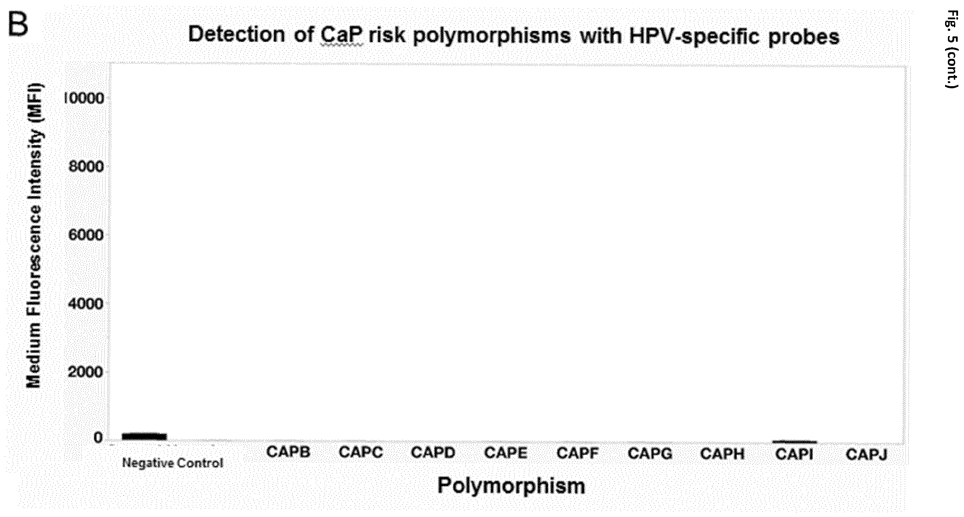

As illustrated in Figure 5, the oligonucleotides "primers" designed for the identification of prostate cancer SNPs were not cross-reactive with the HPV-specific "OLA reporter probes" (Figure 5). Figure 5A shows the detection of CAP risk SNPs A-J using specific CAP probes. Figure 5B shows the no detection of CAP risk SNPs A-J using specific probes for HPV detection. The bars represent the Mean ± 1 Standard Deviation of 4 replicates, the value of P represents the statistically significant difference between the MFI values recorded in graph B with respect to the MFI values recorded in Figure 5A, determined by the Kruskal-Wallis test (α = 0.05), PC = 264.35 MFI (n = 80, 95% CI).

### EXAMPLE 4

### SAMPLE PREPARATION AND EVALUATION OF CAP RISK SNPs PCR PRODUCTS

Preparation of DNA sample, synthetic control mixtures WT, MT, and HET and oligonucleotides (L1) sense and (L2) reverse of for the detection of prostate cancer (CAP) SNPs CAPA, CAPB, CAPC, CAPD, CAPE, CAPF, CAPG, CAPH, CAPI and CAPJ were prepared as previously described for CACU risk SNPs.

Preparation of oligonucleotide mixture and Master Mix of PCR end point. The volume of each oligonucleotide sense (L1) and reverse (L2) was placed in a new and sterile propylene tube as indicated in Table 9. As illustrated in Table 10, 15 µL of Master Mix 2X of PCR end point (Thermo Scientific) was added. As described indicated below, 5 samples were analyzed (1 DNA sample and 4 controls; negative, WT, MT and HET) plus one extra reaction (see Table 11). Master mix was added to the oligonucleotide mixture for PCR end point amplification from 10-oligonucleotide array of CAP SNPs as described in Table 12.

**Table 9. Volume of oligonucleotides sense (L1) and reverse (L2) required for reaction mixture of PCR 10-plex from CAP array.**

| Marker | Aliquot | C_{Initial} (µM) | C_{Final}(µM) | Volume 1 rxn (µL) |
|---|---|---|---|---|
| CAPE | L1 and L2 | 100 | 0.3 | 0.06 |
| CAPH | L1 and L2 | 100 | 0.3 | 0.06 |
| CAPJ | L1 and L2 | 100 | 0.3 | 0.06 |
| CAPA | L1 and L2 | 100 | 0.4 | 0.08 |
| CAPC | L1 and L2 | 100 | 0.4 | 0.08 |
| CAPI | L1 and L2 | 100 | 0.4 | 0.08 |
| CAPG | L1 and L2 | 100 | 0.5 | 0.10 |
| CAPB | L1 and L2 | 100 | 0.5 | 0.10 |
| CAPD | L1 and L2 | 100 | 0.5 | 0.10 |
| CAPF | L1 and L2 | 100 | 0.5 | 0.10 |
| Total volume of L1 and L2 | | | | 1.64 µL (0.82 µL L1 and L2) |
| Final volume of the reaction | | | | 20 µL |

**Table 10. Master mix volume added to oligonucleotide mixture for the reaction mix of PCR 10-oligonucleotide from CAP array.**

| Reagent | Cᵢₙᵢₜᵢₐₗ | C_{Final} | Volume 1 rxn (µL) |
|---|---|---|---|
| Master mix | 2X | 1.5X | 15 |
| Final reaction Volume | | | 20 µL |

**Table 11. Volume of sense (L1) and reverse (L2) oligonucleotides required for PCR amplification of the 10-oligonucleotide panel of CAP from 5 samples plus an extra reaction**

| Marker | Aliquot | C_{Initial} (µM) | C_{Final}(µM) | Volume 1 rxn (µL) | Volume 6 rxn (µL) |
|---|---|---|---|---|---|
| CAPE | L1 and L2 | 100 | 0.3 | 0.06 | 0.36 |
| CAPH | L1 and L2 | 100 | 0.3 | 0.06 | 0.36 |
| CAPJ | L1 and L2 | 100 | 0.3 | 0.06 | 0.36 |
| CAPA | L1 and L2 | 100 | 0.4 | 0.08 | 0.48 |
| CAPC | L1 and L2 | 100 | 0.4 | 0.08 | 0.48 |
| CAPI | L1 and L2 | 100 | 0.4 | 0.08 | 0.48 |
| CAPG | L1 and L2 | 100 | 0.5 | 0.10 | 0.60 |
| CAPB | L1 and L2 | 100 | 0.5 | 0.10 | 0.60 |
| CAPD | L1 and L2 | 100 | 0.5 | 0.10 | 0.60 |
| CAPF | L1 and L2 | 100 | 0.5 | 0.10 | 0.60 |
| Total volume of L1 and L2 | | | | 1.64 µL | 9.84 µL (4.92 µL L1 and L2) |
| Final reaction Volume | | | | 20 µL | 120 µL |

**Table 12: . Master mix volume**

| Reagent | Cᵢₙᵢₜᵢₐₗ | C_{Final} | Volume 1 rxn (µL) | Volume 6 rxn (µL) |
|---|---|---|---|---|
| Master mix | 2X | 1.5X | 15 | 90 |
| Final reaction Volume | | | 20 µL | 120 µL |

Preparation of PCR reaction mixture. Components of the PCR reaction were added in the following order: water, Master Mix + oligonucleotides (L1 and L2), and last, the DNA from the samples or synthetic controls (see Table 13). PCR was performed using standard conditions. The PCR products were analyzed by electrophoresis and may be further analyzed by multiplex technology or sequencing.

**Table 13. The components of each PCR reaction tube for amplification of 10-oligonucleotides panel of CAP in urine and blood DNA samples.* The indicated volume is taken from the DNA sample is at an initial concentration of 50 ng/µL. In case the sample presents a low concentration, the volume must be adjusted to 100ng for the PCR reaction.**

| Reaction tube | Cᵢₙᵢₜᵢₐₗ | C_{Final} | Sample volume (µL) | Volume of H₂O (µL) | Volume of Master Mix + Oligonucleotides (µL) |
|---|---|---|---|---|---|
| Negative control | --- | --- | --- | 3.36 | 16.64 |
| WT synthetic control | 0.03 µM | 0.003 µM | 2 | 1.36 | 16.64 |
| MT synthetic control | 0.03 µM | 0.003 µM | 2 | 1.36 | 16.64 |
| HET synthetic control | 0.03 µM | 0.003 µM | 2 | 1.36 | 16.64 |
| Biological sample ADN | 50 ng/µL | 100 ng | 2* | 1.36 | 16.64 |
| Final reaction Volume : 20 µL | | | | | |

As illustrated in Figure 6, cancer susceptibility SNPs were detected in both blood and urine samples. The results of the PCR amplifications of CAPG, CAPD and CAPH (prostate cancer susceptibility markers) show that both urine (U) and blood (B), samples were suitable for DNA extraction and genotyping of such markers, with PCR products equivalent to those obtained with either a positive control (WT, wild type marker), a control DNA comprising the mutant marker (MT), or DNA extracted from a cell line that presented the SNP (CL).

It was determined that blood and urine samples were suitable for the genotyping of cancer susceptibility markers and that DNA amplification combined with an OLA detected cancer susceptibility markers in a patient and thus established if the patient presented a susceptibility risk of developing the corresponding cancer.

### EXAMPLE 5

### PCR ANALYSIS OF HIGH-RISK HUMAN PAPILLOMA VIRUS

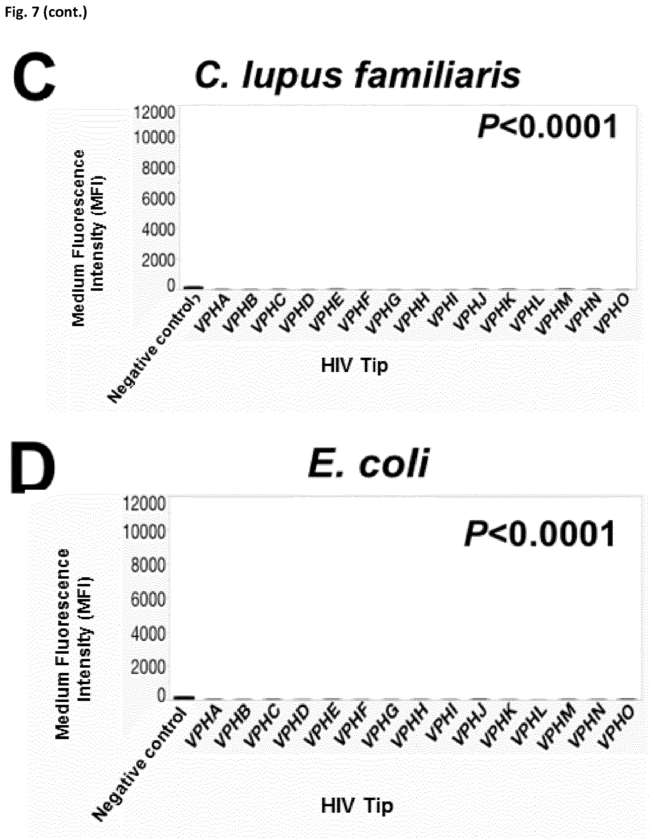

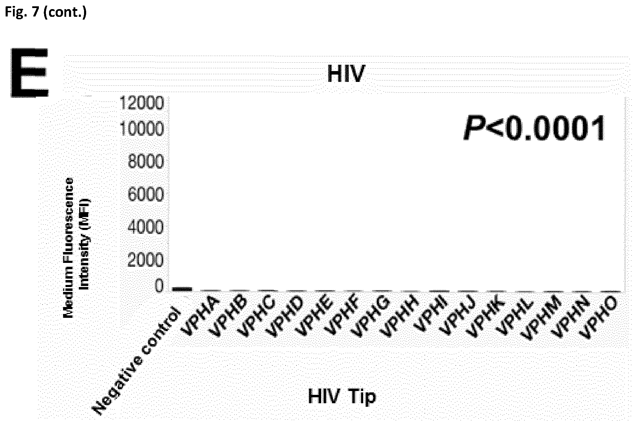

The oligonucleotides "primers" for the identification of the high-risk types of HPV do not cross-react with other genomic DNA other than HPV (Figure 7).

The detection and amplification of each of the high-risk types of HPV was determined by using specific oligonucleotides "primers" designed for each type of high-risk HPV, which contain nucleotide sequences complementary to the region of interest within the HPV L1 gene. The primers were designed to amplify SEQ ID NOs:67-82. The initiating oligos flanked the specific region of each type of HPV in the L1 gene. The oligonucleotides "primers" designed for the detection of the 15 types of high-risk HPV had complementarity with their target sequence of 80%, 90% and 100%. Degenerate "primer" oligonucleotides were designed to detect the HPV subtypes of interest. The oligonucleotides "primers" for the 15 types of HPV had a range of Alignment Temperature (Tm) between 61 ° C and 63 ° C. The oligonucleotides "primers" had a concentration range between 0.4 µM and 0.6 µM (Table 14).

**Table 14. Oligonucleotide primers conditions, OLA and OLA reporter probe.**

| Panel | Primers (concentration range in µM) | OLA (concentration range in µM) | OLA reporter probe (concentration range in µM) |
|---|---|---|---|
| HPV | 0.3 - 0.6 | *0.1-0.4* | *4-6* |

As illustrated in Figure 7, the primers used for the detection of high risk types of HPV were suitable for such detection in synthetic controls (Figure 7A) and in DNA isolated from a biological control (Figure 7B). However, there was no detection when the primers were used on DNA isolated from *C. lupus familiaris* (Figure 7C, p<0.0001), *E. coli* (Figure 7D, p<0.0001) or HIV (Figure 7E, p<0.0001). The bars represent the Mean ± 1 Standard Deviation of 4 replicates, the P value represents the statistically significant difference of the MFI values recorded in each test with respect to the MFI values recorded in the synthetic controls and in the biological controls determined by the Kruskal-Wallis test (α = 0.05), PC = 359.38 MFI (n = 60, 95% CI)

The oligonucleotides "primers" designed for the identification of high-risk types of HPV did not cross-react with other genomic DNA (Figure 7).

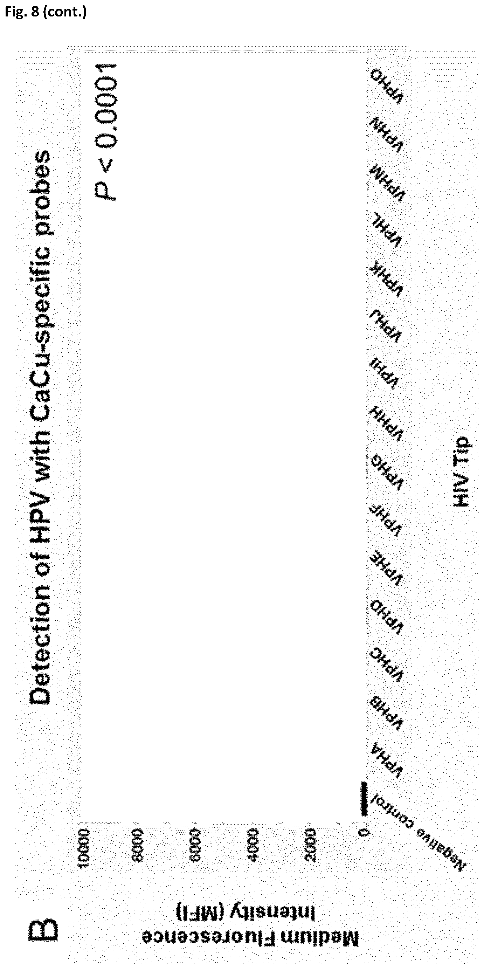

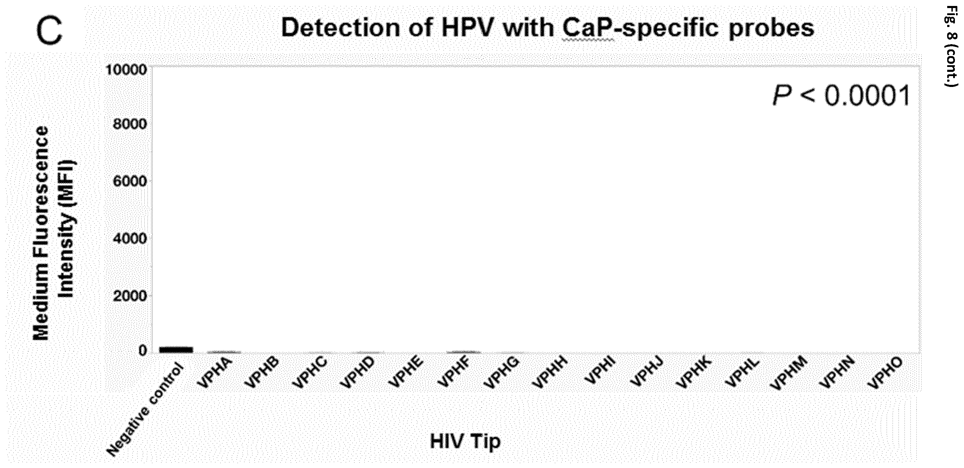

As illustrated in Figure 8, the oligonucleotides "primers" designed for the identification of the 15 types of high-risk HPV did not cross-react the "OLA reporter probes" specific for cervical cancer (Figure 8B) and prostate cancer (Figure 8C). The bars represent the Mean ± 1 Standard Deviation of 4 replicates, the value of P represents the statistically significant difference between the MFI values recorded in graph B with respect to the MFI values recorded in Figure 5A, determined by the Kruskal-Wallis test (α = 0.05), PC = 440.47 MFI (n = 60, 95% CI).

For the preparation of the DNA sample, the mixtures of synthetic HPV controls (identified as A, B, C, D, E, F, G, H, I, J, K, L, M, N, O), and the sense (L1) and reverse (L2) oligonucleotides CAPA, CAPB, CAPC, CAPD, CAPE, CAPF, CAPG, CAPH, CAPI and CAPJ, were prepared as previously described for the CACU risk SNPs . From the 200 µM stocks of the synthetic HPV controls an aliquot was prepared at a final concentration of 30 µM and from this concentration sequential dilutions were made to obtain a final concentration will be 0.003 µM. To prepare the mix of synthetic controls used in the HPV panel multiplex system 1 µL of the 0.003 µM dilution of each synthetic control was added in a tube to obtain a final volume of 15 µL. The final concentration of the synthetic controls mix was 0.0002 µM.

Preparation of oligonucleotide mixture and Master Mix PCR endpoint. The volume of each sense (L1) and reverse (L2) oligonucleotide as indicated in Table 15 was added in a tube. 15 µL of Master Mix 2X PCR endpoint (ThermoScientific) was added to the tube containing the sense and reverse oligonucleotides and homogenized.

Preparation of PCR reaction mixture. For this case 4 reactions and 1 extra reaction were performed, considering a negative control, synthetic control: that is a mix of synthetic sequences identical to the partial region of each of the HPV types identified, sample of DNA from a biological sample A and sample of DNA from a Biological sample B. The necessary amount of Master Mix was placed in a sterile 0.6 ml tube, for 5 reactions (as detailed in Table 16). To carry out the amplification by PCR endpoint of the HPV panel in 15-Plex format, oligonucleotides L1 and L2 must be available. Table 16 describes the concentrations and volumes for 5 reactions, as in Table 17. The reaction mixtures were prepared in tubes by adding the components of the PCR reaction in the following order: water, mixture of Master Mix + oligonucleotides (L1 and L2), and finally the DNA of the samples or of the synthetic controls (Table 18). Perform PCR amplification under standard conditions. The PCR products were analyzed by electrophoresis. Once the products of interest have been checked proceed to the analysis using the Luminex platform.

As illustrated in Figure 9, target sequences of viral HPV DNA were detected by multiplex PCR. The results of the PCR amplification of HPVA and HPVB show that DNA extracted from a cell line known to contain the corresponding virus (CL) was a suitable sample for the detection of HPV infection, with PCR products equivalent to those obtained with either a synthetic positive control (C+1), or indicates viral DNA (C+2). By detecting the presence of viral DNA of one or more of the HPV strains tested in a sample collected from a patient, a DNA amplification combined with an OLA detected HPV infection in a patient and thus established if the patient presented a susceptibility risk of developing the corresponding cancer.

| Marker | Aliquot | C_{Initial} (µM) | C_{Final}(µM) | Vol. 1rxn (µL) |
|---|---|---|---|---|
| HPVA | L1 and L2 | 200 | 0.5 | 0.05 |
| HPVB | L1 and L2 | 200 | 0.4 | 0.04 |
| HPVC | L1 and L2 | 200 | 0.6 | 0.06 |
| HPVD | L1 and L2 | 200 | 0.4 | 0.04 |
| HPVE | L1 and L2 | 200 | 0.4 | 0.04 |
| HPVF | L1 and L2 | 200 | 0.5 | 0.05 |
| HPVG | L1 and L2 | 200 | 0.5 | 0.05 |
| HPVH | L1 and L2 | 200 | 0.5 | 0.05 |
| HPVI | L1 and L2 | 200 | 0.5 | 0.05 |
| HPVJ | L1 and L2 | 200 | 0.4 | 0.04 |
| HPVK | L1 and L2 | 200 | 0.4 | 0.04 |
| HPVL | L1 and L2 | 200 | 0.6 | 0.06 |
| HPVM | L1 and L2 | 200 | 0.5 | 0.05 |
| HPVN | L1 and L2 | 200 | 0.5 | 0.05 |
| HPVO | L1 and L2 | 200 | 0.4 | 0.04 |
| Final volume of L1 and L2 | | | | 1.42 µL (0.71 µL de Lland L2) |
| Final reaction volume | | | | 20 µL |
| Table 15. Volume of sense (L1) and reverse (L2) oligonucleotides required for PCR amplification 15 HPV panel | | | | |
| oligonucleotides, for 1 reaction. | | | | |

**Table 16. Master mix volume required for 5 reactions.**

| Reagent | Cᵢₙᵢₜᵢₐₗ (µM) | C_{final} (µM) | Vol. 1rxn (µL) | Vol. 5 rxn(µL) |
|---|---|---|---|---|
| Master mix | 2X | 1.5X | 15 | 75 |
| final Volume | | | 20 | 100 |

**Table 17. Volume of sense (L1) and reverse (L2) oligonucleotides required for PCR amplification of panel 15 HPV oligonucleotides, 1 reaction.**

| Marker | Aliquot | C_{Initial} (µM) | C_{Final}(µM) | Vol. 1rx (µL) | Vol. 5rx (µL) |
|---|---|---|---|---|---|
| HPVA | L1 and L2 | 200 | 0.5 | 0.05 | 0.25 |
| HPVB | L1 and L2 | 200 | 0.4 | 0.04 | 0.2 |
| HPVC | L1 and L2 | 200 | 0.6 | 0.06 | 0.3 |
| HPVD | L1 and L2 | 200 | 0.4 | 0.04 | 0.2 |
| HPVE | L1 and L2 | 200 | 0.4 | 0.04 | 0.2 |
| HPVF | L1 and L2 | 200 | 0.5 | 0.05 | 0.25 |
| HPVG | L1 and L2 | 200 | 0.5 | 0.05 | 0.25 |
| HPVH | L1 and L2 | 200 | 0.5 | 0.05 | 0.25 |
| HPVI | L1 and L2 | 200 | 0.5 | 0.05 | 0.25 |
| HPVJ | L1 and L2 | 200 | 0.4 | 0.04 | 0.2 |
| HPVK | L1 and L2 | 200 | 0.4 | 0.04 | 0.2 |
| HPVL | L1 and L2 | 200 | 0.6 | 0.06 | 0.3 |
| HPVM | L1 and L2 | 200 | 0.5 | 0.05 | 0.25 |
| HPVN | L1 and L2 | 200 | 0.5 | 0.05 | 0.25 |
| HPVO | L1 and L2 | 200 | 0.4 | 0.04 | 0.2 |
| Volume of L1 and L2 | | | | 1.42 µL (0.71 µL de Lland L2) | 7.1 µL (3.55 µL de Lland L2) |
| Final reaction Volume | | | | 20 µL | 100 µL |

**Table 18. Components of each PCR reaction tube for panel amplification 15 HPV oligonucleotides in DNA samples from biological samples. * The indicated volume is taken from a DNA sample that is at an initial concentration of 50 ng/µL.**

| Reaction tube | C_{Initial} | C_{Final} | Sample volume (µL) | H₂O volume (µL) | Master Mix + oligonucleotides volume (µL) |
|---|---|---|---|---|---|
| Negative control | --- | --- | --- | 3.58 | 16.42 |
| Synthetic control | 0.0002 µM | 0.00002 µM | 2 | 1.58 | 16.42 |
| DNA of biological sample A | 50 ng/µL | 100 ng | 2* | 1.58 | 16.42 |
| DNA of biological simple B | 50 ng/µL | 100 ng | 2* | 1.58 | 16.42 |
| Final reaction Volume: 20 µL | | | | | |

### EXAMPLE 6

### AMPLIFICATION BY ENDPOINT PCR IN MULTIPLEX FORMAT OF SNPS ASSOCIATED WITH THE RISK OF ANY TYPE OF CANCER.

The DNA samples were prepared in the same way as in the previous examples, however, for the detection and amplification of SNP associated with risk of any type of cancer and of 7 different types of cancer (including breast cancer, prostate, colon, leukemia , lung, gastric and pancreas), 41 pairs of "primer" oligonucleotides specific to each locus of interest were used. The oligos "initiators" flanked the locus of interest in the genes or in important sequences of the regulation of the genes (i.e. IL10, TGF-β1, CYP1A1, ST3GAL4, IL4, AGTR1, ERBB2, HOXB13, Chr8, MSMB, RNASEL, CYP19, ABO, ABL1, ARID5B, Chr13, CCKBR, JAK2, NQO1, EGFR, EPHX1, CHRNA3, IRF4, PIK3CA, CXCL8, BRCA2, BRCA1, ATM, TCF2, TGFB1, KiSS1, APC, PGR, MTHFR, MUTYH, CDH1, IL1B, IL6, TLR4, TNF-α, MDM2, MLH1, BRAF, TP53, BCAR1, STK11, KRAS, FGFR2, SRD5A2, EIF3H and/or MSH6). The oligos "initiators" for the SNPs of risk to cancer had complementarity with their white sequence of 80%, 90% and 100%. Oligonucleotides "primers" for cancer risk SNPs had a range of Alignment Temperature (Tm) between 62 ° C and 65 ° C as previously described. Oligonucleotides "primers" for SNPs at risk of cancer They had a concentration range between 0.2 µM and 0.5 µM.

Figure 10 shows the identification of 20 cancer risk polymorphisms using synthetic controls by the GanPlex MR test (Panel A). The graph shows the MFI values recorded in each of the cancer risk polymorphisms when the Wilde type (CS WT), Mutant (MT) and Heterozygous (HET) synthetic controls were used. The bars represent the Average ± 1 D.E. of 2 replicas.

Figure 11 shows the identification of 22 cancer risk polymorphisms using synthetic controls by the GanPlex MR test (Panel B). The graph shows the MFI values recorded in each of the cancer risk polymorphisms when the Wilde type (CS WT), Mutant (MT) and Heterozygous (HET) synthetic controls were used. The bars represent the Average ± 1 D.E. of 2 replicas.

### EXAMPLE 7

### OLA ANALYSIS OF THE PCR PRODUCTS

After multiplex PCR, each sample was subjected to an OLA reaction, where an aliquot of the PCR product was used through the OLA reaction.

For the ligation reaction. From the PCR products obtained in the previously described examples were subjected to ligation reactions. The reaction mixture was carried out from aliquots that were at a concentration of 10 µM with a final concentration of each 20? or 22?; 3 µl of markers were added and the volume adjusted to 20 µL (probe OLA mixture). The probe OLA mixture was prepared at a final concentration of 5 µM in 20 µL. OLA-Mix 2X was prepared considering that each of the components must have the following final concentration: Buffer ligase 2X µM, Taq DNA ligase 1 U µM, OLA-TAG Mix 0.015 µM and OLA-PROBE at 0.5 µM, in 18 µL. The OLA Mix 2X must had a final concentration of 1 (?), and the ligation reaction was performed as described in Table 19.

Hybridization assay. After the ligation reaction a hybridization test is performed using the "TAG distribution" format. Beads were assigned to each marker and resuspended in a 1X Tm buffer. Microsphere mixtures were prepared for each panel (40 spheres). Seven reactions per panel were performed: C. -, C. WT, C. MT, C. HT, DNA saliva, DNA blood and background, according to table 20: microsphere mixtures for multiplex hybridization were prepared with the following final concentration of each of the following components: microsphere WT 1000 microspheres/µL, microsphere WT 1000 microspheres/µL and Buffer Tm 1X and mixed again by vortex for 30 seconds. Place 22.5 µL of the mixture of microspheres per well in a V bottom plate. 2.5 µL of bound product or water, were added, as appropriate. The plate was then covered with a film and the hybridization was performed in thermal cycler, as in Table 20

Microsphere marking and Reading in Magpix equipment. Streptavidin, R-Phycoerythrin conjugate (SAPE) was prepared at a final concentration of 2 µg / mL in 1X Tm buffer as indicated in Table 21. Once the hybridization was complete place the 96-well plate on the magnetic plate. The supernatant was removed and rinsed twice with 100µl of 1X Tm buffer. The plate was placed the 96-well plate on the black metal plate and 75 µL of SAPE was added at 2 µg/mL to each well. The plate was incubated for 15 minutes at 37 ° C in dark and the samples analyzed.

Analyze in the MagPix equipment, 50 µL of sample at 37 ° C

**Table 19. Thermocycling conditions for Multiplex Ligation reaction**

| Temperature (°C) | Time | Cycle |
|---|---|---|
| 96 °C | 2 min. | 1 |
| 94 °C | 15 sec. | 30 |
| 58 °C | 1 min | |
| 4 °C | ∞ | ---- |

**Table 20. Thermocycling conditions for multiplex hybridization**

| Temperature (°C) | Time | Cycle |
|---|---|---|
| 96 °C | 90 sec | 1 |
| 37 °C | 30 min | |
| 37 °C | ∞ | ---- |

**Table 21. Preparation of Buffer Tm, for the reading of 7 samples in duplicate.**

| Reagent | C. Initial (µg /mL) | C. Final (µg/mL) | V.1rxn (µL) | V.16 rxn (µL) |
|---|---|---|---|---|
| SAPE | 600 | 2 | 0.25 | 4 |
| Buffer Tm | 1X | 1X | 74.75 | 1,196 |
| | | Vol. Final | 110 | 1,200 |

### EXAMPLE 8

### CANCER SUSCEPTIBILITY EVALUATION

A subject's cancer risk or susceptibility to develop cancer is determined by the simultaneous identification and detection of both HPV infection and cancer susceptibility SNP genotyping.

For cancer susceptibility SNPs genotyping, wild type and mutant alleles are discriminated by evaluating fluorescence difference in an allelic discrimination assay. Colored beads are used to perform the analysis. The beads include two dyes where A) one excitation wavelength allows observation of two separate fluorescence emission wavelength, B) yielding around 100 unique bead sets, in this case we uses 50 different bead sets, each bead set can be coated with a nucleic acid CAPture molecule (antifigure 10-TAG) specific to a particular biological target in this case could be SNPs or different types of HPV. This target if it is CAPtured by the acid nucleic attached to the bead surface is marked with SAPE (Phycoerythrin) if is not in the sample we won't see SAPE in that bead set. The difference of each target is made by bead sets. The identification of each target is made by Phycoerythrin (same in every target)

We defined some ranges of fluorescence to identify is the target is in the sample, the parameter is 5 times over background to consider a positive (same in HPV and SNPs). On the other hand, about SNPs, we will always find a target that means that we will always have a positive, it could be WT, MT or HT, but to discriminate and determine if they are WT, MT or HT we use the allelic call, that is calculated by fluorescence proportions. It means that over the 100% of the fluorescence if the proportion of the total fluorescence is around 70% for WT target and 25% of the MT target it is established like a WT sample or participant, same thing for the MT. But if the proportion is 50/50 both targets are present and It is considered as a HT. The proportions could be 75/25, 80/20, 90/10 even 70/30 it depends of each target for Homocygotes. For HT could be 50/50, or even 64/40 in some cases.

As illustrated in Figure 12, using a synthetic wild type control (WT), a synthetic mutant control (MT) and a synthetic heterozygous control (WT/MT), by plotting the percentage of fluorescence associated with the wild type allele (WT) on the X axis, the fluorescence associated with a mutant allele (MT) on the Y axis, the allelic discrimination assay allows to determine fluorescence areas: a mutant area, a WT area and a heterozygous area that discriminate samples based on the cancer susceptibility SNPs' genotype. A sample is considered WT when the fluorescence of the dye associated with the WT allele is between 25 and 100% and the fluorescence of the dye associated with the mutant allele is less than 25%. A sample is considered mutant when the fluorescence of the dye associated with the WT allele is less than 25% and the fluorescence of the dye associated with the mutant allele is between 25 and 100%. A sample is considered heterozygous when the fluorescence of the dye associated with the WT allele is between 25 and 100% and the fluorescence of the dye associated with the mutant allele is between 25 and 100%. If both the fluorescence of the dye associated with the WT allele and the fluorescence of the dye associated with the mutant allele are less than 25% the sample is considered unlabeled and cannot be exploited further.

By using viral HPV DNA, and plotting the percentage of fluorescence associated with the said HPV DNA, the assays allows to determine a fluorescence range that discriminate samples based on the presence or absence of viral DNA. The fluorescence range is different in each HPV analysis, background fluorescence is around 200-300 MFI. The range of fluorescence to discriminate between positives to HPV panel it is around 1500-7000 MFI.

By defining MT, WT and heterozygous fluorescence areas, and the HPV DNA presence in a sample, the allelic discrimination assay combine with the HPV infection determination assay allow to determine if a sample presents a cancer susceptibility polymorphism and/or a HPV infection. The combination of both assays is thus used to determine if a patient presents a risk or a susceptibility of developing the corresponding cancer.

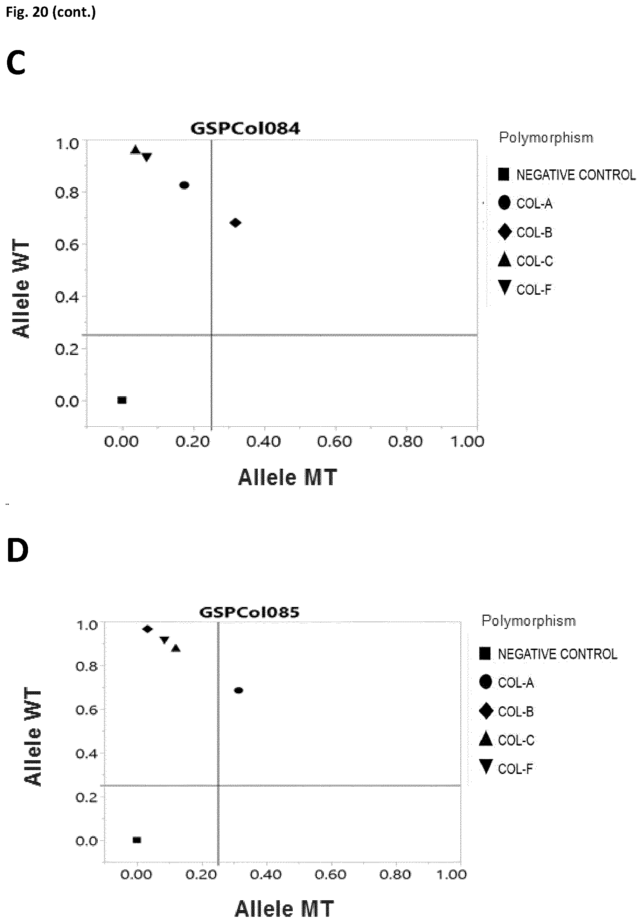

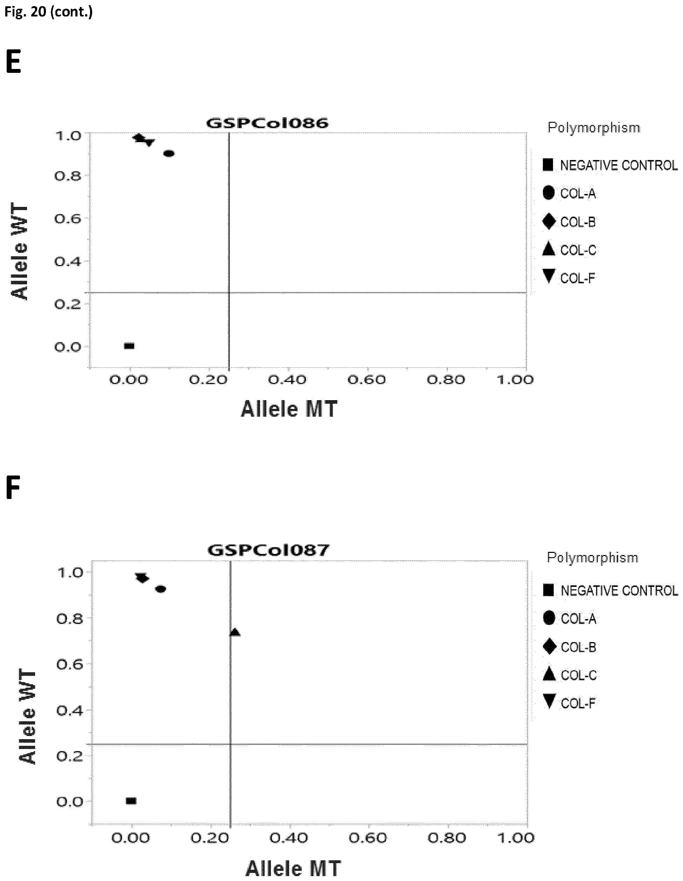

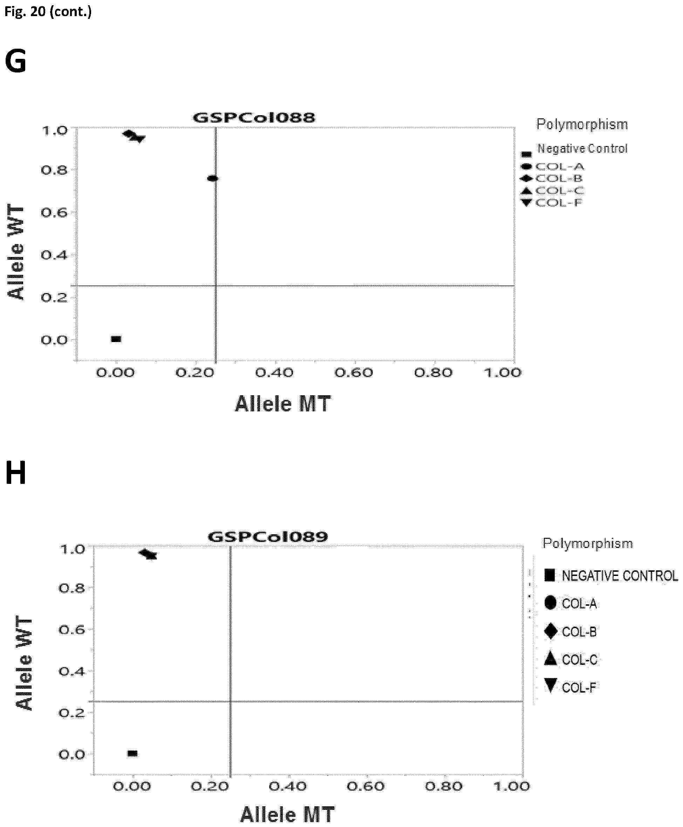

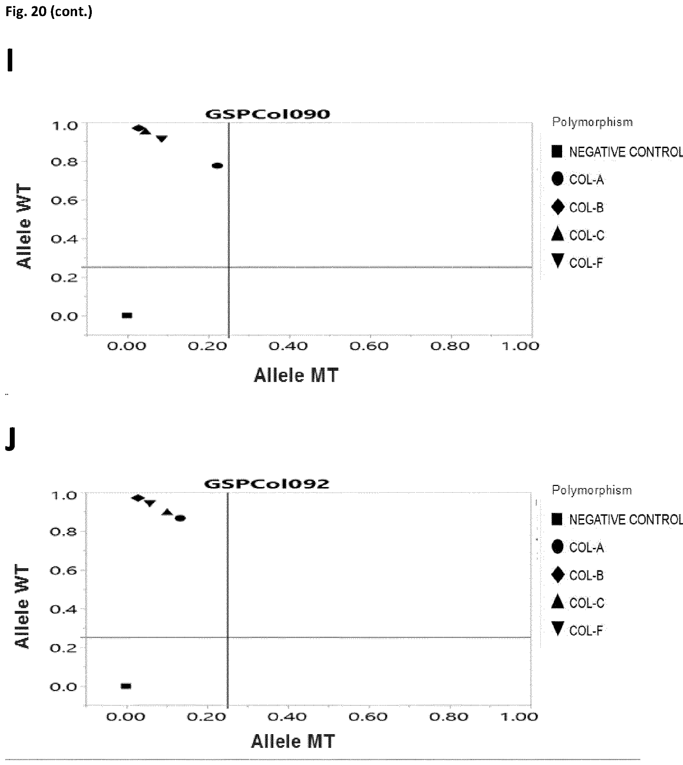

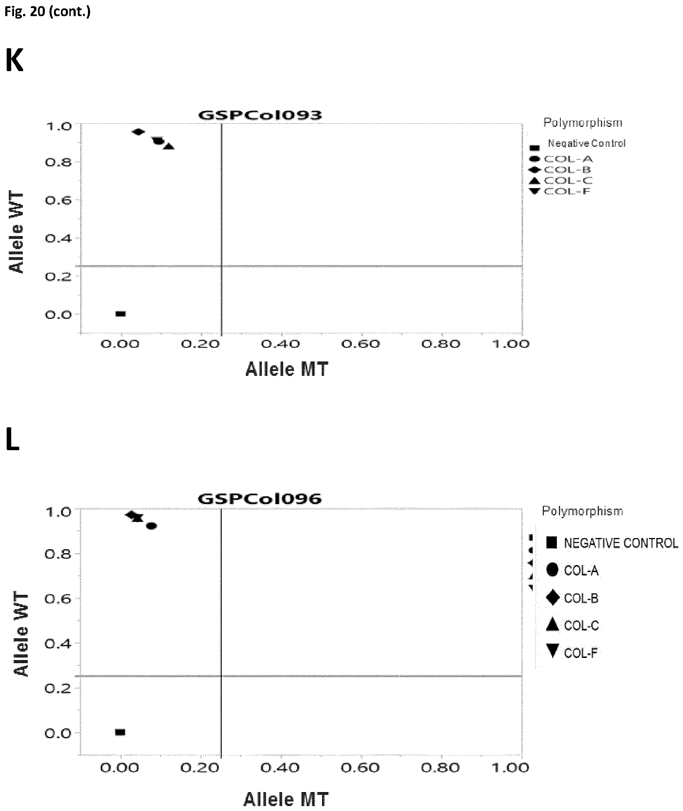

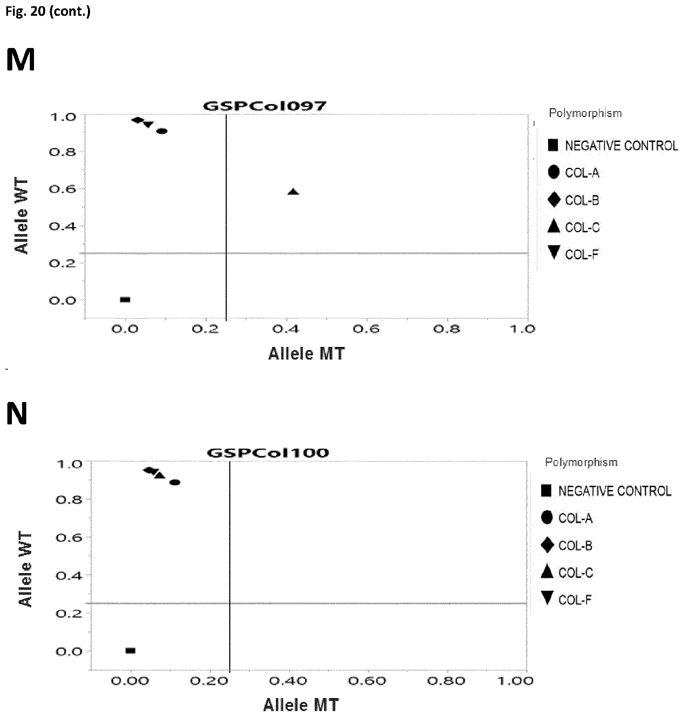

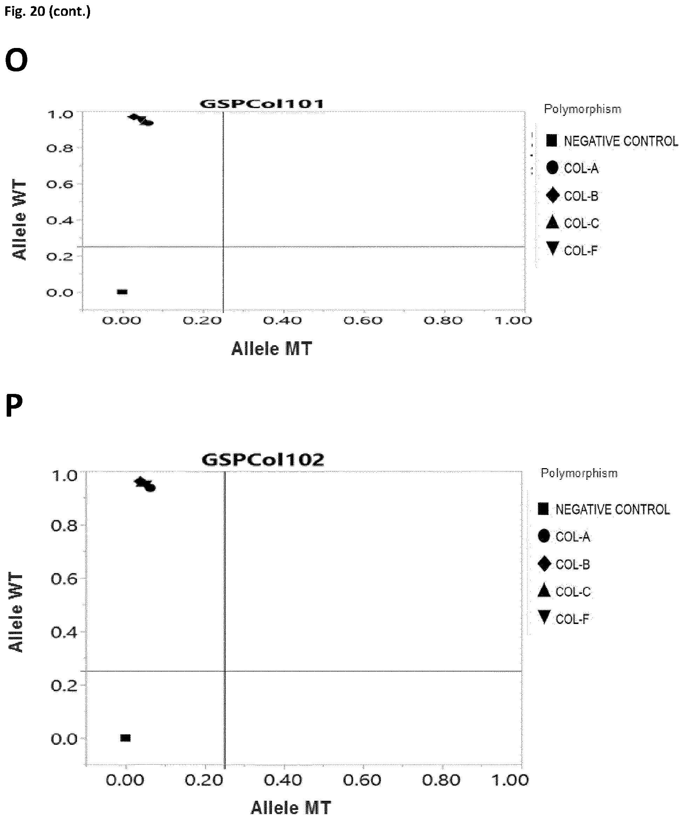

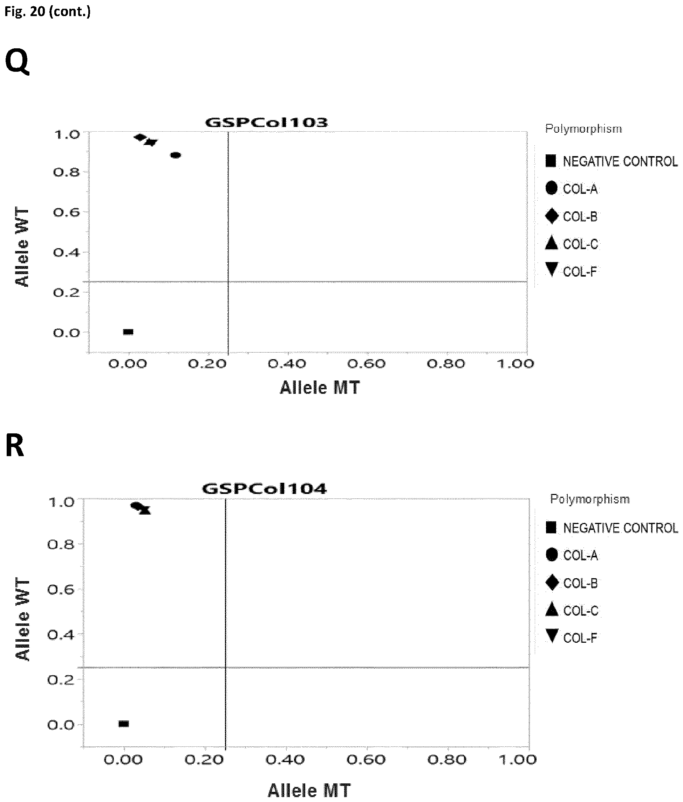

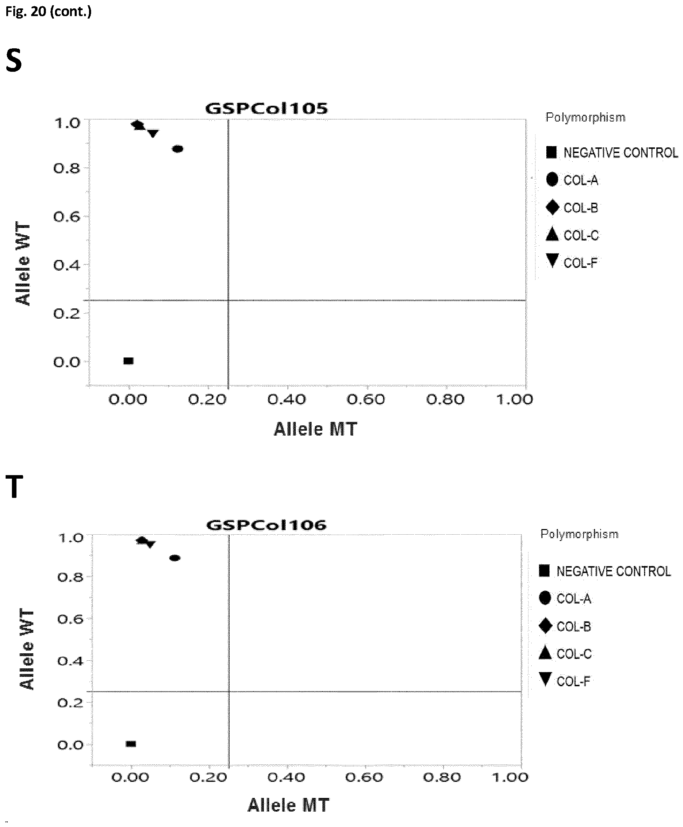

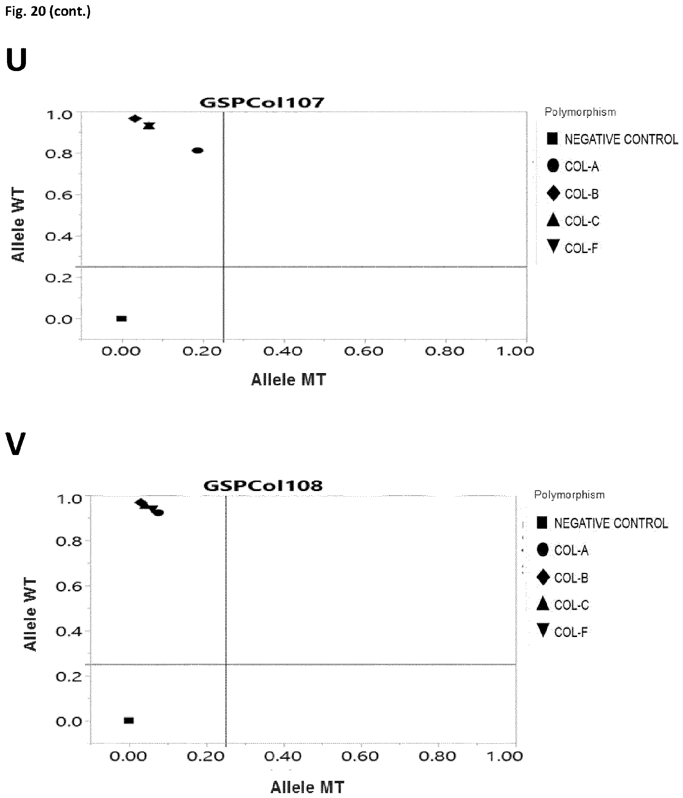

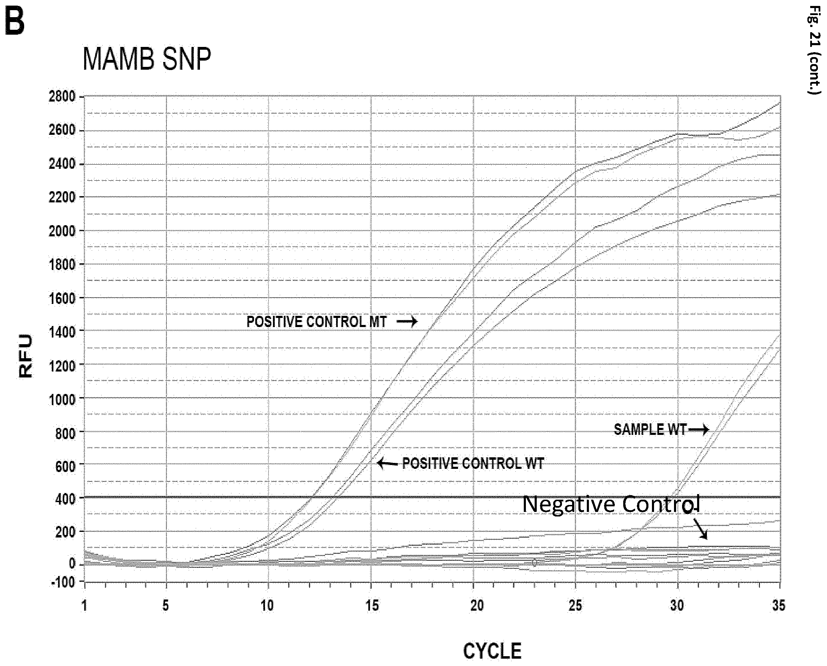

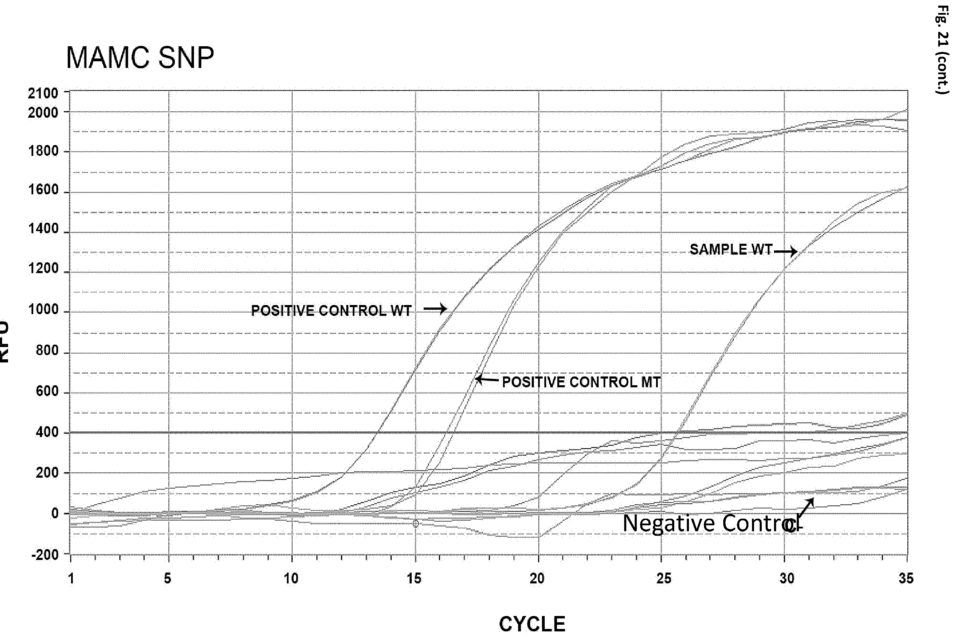

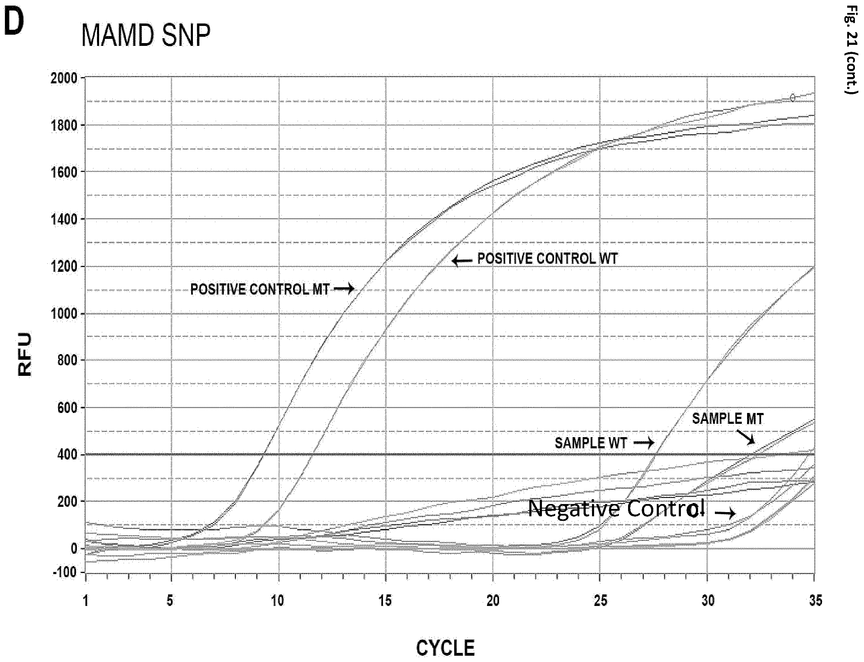

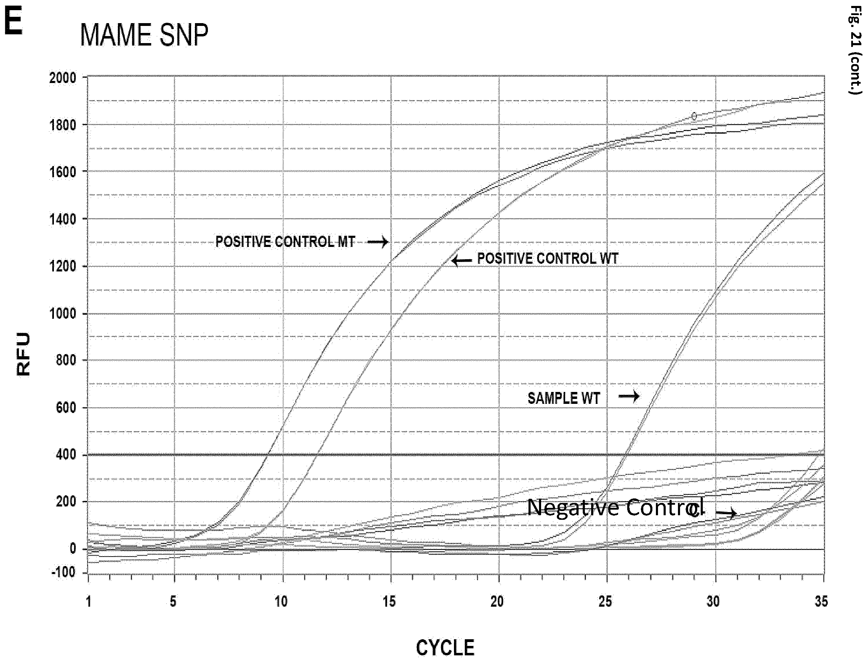

Specific examples are shown in Figure 20 and 21. Samples obtained from subjects having colon or breast cancer. Cell free DNA was obtained from the samples as described above and the DNA was subjected to amplification for colon and breast cancer susceptibility marker genes and detection of the marker genes with specific probes as described previously. The data was analyzed and the results are shown in Figure 20 and 21.

Although the invention has been described with reference to the above examples, it will be understood that modifications and variations are encompassed within the spirit and scope of the invention. Accordingly, the invention is limited only by the following claims.

## Claims

1. A method for assessing cancer susceptibility in a subject comprising simultaneously identifying one or more cancer susceptibility marker genes and one or more human papillomavirus (HPV) genotype strains, thereby assessing cancer susceptibility in the subject.

2. The method of claim 1, wherein identifying the one or more cancer susceptibility marker genes comprises:
a) amplifying a cell free DNA sample from the subject using primers to the one or more cancer susceptibility marker genes; and
b) detecting the amplified DNA.

3. The method of claim 2, further comprising amplifying the cell free DNA sample using primers to the one or more HPV strain genotypes.

4. The method of claim 1, wherein the cancer is selected from the group consisting of cervical, prostate, colon, pancreatic, breast, lung, leukemia and gastric.

5. The method of claim 1, wherein the one or more cancer susceptibility marker genes is selected from the group consisting of CACUA. CACUB, CACUC, CACUD, CACUE, CACUF, CACUG, CACUH, CACUI, CACUJ, CAPA, CAPB, CAPC, CAPD, CAPE, CAPF, CAPG, CAPH, CAPI, CAPJ, COLA, COLB, COLC, COLD, COLE, COLF, COLG, GASA, GASB, GASC, GASD, GASE, GASG, LEUA, LEUB, LEUC, LEUD, LEUE, LEUF, MAMA, MAMB, MAMC, MAMD, MAME, MAMF, MAMG, PROA, PROB, PROC, PROD, PROE, PROF, PANA, PANB, PANC, PAND, PULA, PULB, PULC, PULD, PULE, PULF, PULG, GENA, GENB, GEND, GENE, GENG, GENH and/or GENI.

6. The method of claim 1, wherein the one or more cancer susceptibility marker genes is a single nucleotide polymorphism (SNP) located within the gene encoding for IL10, TGF-β1, CYP1A1, ST3GAL4, IL4, AGTR1, ERBB2, HOXB13, Chr8, MSMB, RNASEL, CYP19, ABO, ABL1, ARID5B, Chr13, CCKBR, JAK2, NQO1, EGFR, EPHX1, CHRNA3, IRF4, PIK3CA, CXCL8, BRCA2, BRCA1, ATM, TCF2, TGFB1, KiSS1, APC, PGR, MTHFR, MUTYH, CDH1, IL1B, IL6, TLR4, TNF-α, MDM2, MLH1, BRAF, TP53, BCAR1, STK11, KRAS, FGFR2, SRD5A2, EIF3H and/or MSH6.

7. The method of claim 1, wherein the one or more cancer susceptibility marker genes is selected from the group consisting of SEQ ID NOs:1-67.

8. The method of claim 1, where in the one or more HPV strain genotype is selected from the group consisting of HPV-16, HPV-18, HPV-31, HPV-33, HPV-35, HPV-39, HPV-45, HPV-51, HPV-52, HPV-56, HPV-58, HPV-59, HPV-66, HPV-68 and/or HPV-73.

9. The method of claim 7, wherein the one or more HPV strain genotype is a high risk HPV genotype strain.

10. The method of claim 9, wherein the high risk HPV strain is HPV-16, HPV-18, HPV-31, HPV-45 and/or HPV-58.

11. The method of claim 2, wherein the primers to the one or more cancer susceptibility marker genes are designed to amplify CACUA. CACUB, CACUC, CACUD, CACUE, CACUF, CACUG, CACUH, CACUI, CACUJ, CAPA, CAPB, CAPC, CAPD, CAPE, CAPF, CAPG, CAPH, CAPI, CAPJ, COLA, COLB, COLC, COLD, COLE, COLF, COLG, GASA, GASB, GASC, GASD, GASE, GASG, LEUA, LEUB, LEUC, LEUD, LEUE, LEUF, MAMA, MAMB, MAMC, MAMD, MAME, MAMF, MAMG, PROA, PROB, PROC, PROD, PROE, PROF, PANA, PANB, PANC, PAND, PULA, PULB, PULC, PULD, PULE, PULF, PULG, GENA, GENB, GEND, GENE, GENG, GENH, GENI and/or SEQ ID NOs:1-67.

12. The method of claim 3, wherein the primers to the one or more HPV strain genotypes are designed to amplify HPVA, HPVB, HPVC, HPCD,HPVE, HPVF, HPVG, HPVI, HPVJ, HPVK, HPVL, HPVM, HPVN, HPVO and/or SEQ ID NOs:68-82.

13. The method of claim 2, wherein the detecting of the amplified DNA is performed by oligo ligation assay (OLA), PCR, qPCR, DNA sequencing, fluorescence, gel electrophoresis, magnetic beads, allele specific primer extension (ASPE) and/or direct hybridization.

14. The method of claim 13, wherein the OLA uses probes to the one or more cancer susceptibility marker genes.

15. The method of claim 14, further comprising using probes to the one or more HPV strain genotypes.

16. The method of claim 14 or 15, wherein the probes are designed to bind to SEQ ID NOs:1-82.

17. The method of claim 2, wherein the sample is a blood or urine sample.

18. A method comprising:
a) amplifying a cell free DNA sample from a subject using primers to one or more cancer susceptibility marker genes and to one or more human papillomavirus (HPV) strain genotypes; and
b) detecting the amplified DNA.

19. The method of claim 18, wherein the one or more cancer susceptibility marker genes is a marker for cervical, prostate, colon, pancreatic, gastric, lung leukemia and/or breast.

20. The method of claim 18, wherein the one or more cancer susceptibility marker genes is selected from the group consisting of CACUA. CACUB, CACUC, CACUD, CACUE, CACUF, CACUG, CACUH, CACUI, CACUJ, CAPA, CAPB, CAPC, CAPD, CAPE, CAPF, CAPG, CAPH, CAPI, CAPJ, COLA, COLB, COLC, COLD, COLE, COLF, COLG, GASA, GASB, GASC, GASD, GASE, GASG, LEUA, LEUB, LEUC, LEUD, LEUE, LEUF, MAMA, MAMB, MAMC, MAMD, MAME, MAMF, MAMG, PROA, PROB, PROC, PROD, PROE, PROF, PANA, PANB, PANC, PAND, PULA, PULB, PULC, PULD, PULE, PULF, PULG, GENA, GENB, GEND, GENE, GENG, GENH and/or GENI; a single nucleotide polymorphism (SNP) located within the gene encoding for IL10, TGF-β1, CYP1A1, ST3GAL4, IL4, AGTR1, ERBB2, HOXB13, Chr8, MSMB, RNASEL, CYP19, ABO, ABL1, ARID5B, Chr13, CCKBR, JAK2, NQO1, EGFR, EPHX1, CHRNA3, IRF4, PIK3CA, CXCL8, BRCA2, BRCA1, ATM, TCF2, TGFB1, KiSS1, APC, PGR, MTHFR, MUTYH, CDH1, IL1B, IL6, TLR4, TNF-α, MDM2, MLH1, BRAF, TP53, BCAR1, STK11, KRAS, FGFR2, SRD5A2, EIF3H and/or MSH6; and/or SEQ ID NOs: 1-67.

21. The method of claim 15, wherein the one or more HPV strain genotypes is selected from the group consisting of HPV-16, HPV-18, HPV-31, HPV-33, HPV-35, HPV-39, HPV-45, HPV-51, HPV-52, HPV-56, HPV-58, HPV-59, HPV-66, HPV-68 and/or HPV-73.

22. The method claim 18, wherein the detecting of the amplified DNA is performed by oligo ligation assay (OLA), PCR, DNA sequencing, fluorescence, gel electrophoresis, magnetic beads, allele specific primer extension (ASPE) and/or direct hybridization..

23. The method of claim 22, wherein the PCR is qPCR.

24. The method of claim 18, wherein the sample is a blood or urine sample.

25. A kit comprising:
a) primers to one or more cancer susceptibility marker genes;
b) primers to one or more human papillomavirus (HPV) strain genotypes; and
c) instructions for use.

26. The kit of claim 25, wherein the primers to one or more cancer susceptibility marker genes is selected from the group consisting CACUA. CACUB, CACUC, CACUD, CACUE, CACUF, CACUG, CACUH, CACUI, CACUJ, CAPA, CAPB, CAPC, CAPD, CAPE, CAPF, CAPG, CAPH, CAPI, CAPJ, COLA, COLB, COLC, COLD, COLE, COLF, COLG, GASA, GASB, GASC, GASD, GASE, GASG, LEUA, LEUB, LEUC, LEUD, LEUE, LEUF, MAMA, MAMB, MAMC, MAMD, MAME, MAMF, MAMG, PROA, PROB, PROC, PROD, PROE, PROF, PANA, PANB, PANC, PAND, PULA, PULB, PULC, PULD, PULE, PULF, PULG, GENA, GENB, GEND, GENE, GENG, GENH and/or GENI, and/or SEQ ID NOs: 1-67.

27. The kit of claim 25, wherein the primers to one or more HPV strain genotypes is selected from the group consisting of HPV-16, HPV-18, HPV-31, HPV-33, HPV-35, HPV-39, HPV-45, HPV-51, HPV-52, HPV-56, HPV-58, HPV-59, HPV-66, HPV-68 and/or HPV-73.

28. The kit of claim 25, further comprising probes to the one or more one or more cancer susceptibility marker genes.

29. The kit of claim 25, further comprising using probes to the one or more HPV strain genotypes.
